(19) Europäisches Patentamt
European Patent Office
Office européen des brevets

(11) EP 4 410 292 A1

(12) # EUROPEAN PATENT APPLICATION
published in accordance with Art. 153(4) EPC

(43) Date of publication:
**07.08.2024 Bulletin 2024/32**

(21) Application number: **22876159.9**

(22) Date of filing: **27.09.2022**

(51) International Patent Classification (IPC):
***A61K 31/5355*** *(2006.01)* ***A61K 31/53*** *(2006.01)*
***A61K 31/5377*** *(2006.01)* ***A61P 31/12*** *(2006.01)*
***A61P 31/14*** *(2006.01)* ***A61P 43/00*** *(2006.01)*

(52) Cooperative Patent Classification (CPC):
**A61K 31/53; A61K 31/5355; A61K 31/5377; A61P 31/12; A61P 31/14; A61P 43/00**

(86) International application number:
**PCT/JP2022/035803**

(87) International publication number:
**WO 2023/054292 (06.04.2023 Gazette 2023/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.09.2021 JP 2021157929**
**20.10.2021 JP 2021171725**
**05.01.2022 JP 2022000723**
**02.02.2022 JP 2022015035**
**22.08.2022 JP 2022131590**

(71) Applicants:
- **Shionogi & Co., Ltd**
  **Osaka-shi, Osaka 541-0045 (JP)**
- **National University Corporation Hokkaido University**
  **Sapporo-shi, Hokkaido 060-0808 (JP)**

(72) Inventors:
- **TACHIBANA Yuki**
  **Osaka-shi, Osaka 541-0045 (JP)**
- **UEHARA Shota**
  **Osaka-shi, Osaka 541-0045 (JP)**
- **UNOH Yuto**
  **Osaka-shi, Osaka 541-0045 (JP)**
- **NAKAHARA Kenji**
  **Osaka-shi, Osaka 541-0045 (JP)**
- **TAODA Yoshiyuki**
  **Osaka-shi, Osaka 541-0045 (JP)**
- **KASAMATSU Koji**
  **Osaka-shi, Osaka 541-0045 (JP)**
- **YAMATSU Yukiko**
  **Osaka-shi, Osaka 541-0045 (JP)**
- **ANDO Shigeru**
  **Osaka-shi, Osaka 541-0045 (JP)**
- **FUKAO Keita**
  **Osaka-shi, Osaka 541-0045 (JP)**
- **NOBORI Haruaki**
  **Osaka-shi, Osaka 541-0045 (JP)**
- **KURODA Takayuki**
  **Osaka-shi, Osaka 541-0045 (JP)**
- **TOBA Shinsuke**
  **Osaka-shi, Osaka 541-0045 (JP)**
- **UEMURA Kentaro**
  **Osaka-shi, Osaka 541-0045 (JP)**
- **MARUYAMA Yuki**
  **Osaka-shi, Osaka 541-0045 (JP)**
- **SASAKI Michihito**
  **Sapporo-shi, Hokkaido 060-0808 (JP)**
- **SAWA Hirofumi**
  **Sapporo-shi, Hokkaido 060-0808 (JP)**

(74) Representative: **Vossius & Partner Patentanwälte Rechtsanwälte mbB**
**Siebertstrasse 3**
**81675 München (DE)**

Remarks:
The complete document including Reference Table(s) and the Sequence Listing(s) can be downloaded from the EPO website

(54) **PHARMACEUTICAL COMPOSITION CONTAINING TRIAZINE DERIVATIVE**

(57) The present invention provides a pharmaceutical composition comprising a compound exhibiting coronavirus proliferation inhibitory activity.

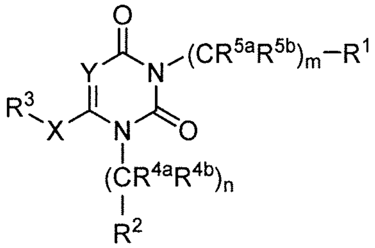

(I)

A pharmaceutical composition comprising a compound represented by the formula: wherein Y is N; $R^1$ is substituted or unsubstituted aromatic heterocyclyl; $R^2$ is substituted or unsubstituted 6-membered aromatic carbocyclyl; $R^3$ is substituted or unsubstituted aromatic heterocyclyl; -X- is -NH-; m is 0 or 1; $R^{5a}$ is a hydrogen atom; $R^{5b}$ is a hydrogen atom; n is 1; $R^{4a}$ is a hydrogen atom; and $R^{4b}$ is a hydrogen atom, or a pharmaceutically acceptable salt thereof.

## Description

[TECHNICAL FIELD]

The present invention relates to a pharmaceutical composition comprising a compound exhibiting coronavirus 3CL protease inhibitory activity.

[BACKGROUND ART]

Coronaviruses belonging to the subfamily Orthocoronavirinae in the family Coronaviridae, order Nidovirales have a genome size of approximately 30 kilobases and are the largest single-stranded plus-stranded RNA viruses in known RNA viruses. Coronaviruses are classified into four genera of Alphacoronavirus, Betacoronavirus, Gammacoronavirus, and Deltacoronavirus, and seven kinds in total of two kinds of the genus Alphacoronavirus (HCoV-229E and HCoV-NL63) and five kinds of the genus Betacoronavirus (HCoV-HKU1, HCoV-OC43, SARS-CoV, MERS-CoV, and SARS-CoV-2) are known as coronaviruses that infect humans. Among them, four kinds (HCoV-229E, HCoV-NL63, HCoV-HKU1, and HCoV-OC43) are pathogens of cold, and the remaining three kinds are severe acute respiratory syndrome (SARS) coronavirus (SARS-CoV), Middle East respiratory syndrome (MERS) coronavirus (MERS-CoV), and novel coronavirus (SARS-CoV-2) that cause severe pneumonia.

Coronavirus disease 2019 (COVID-19) outbreak in Wuhan, China in December 2019 has internationally widespread rapidly, and WHO announced pandemic on March 11, 2020. The number of infected persons confirmed on September 21, 2022 reaches 610 million or more, and the number of deaths reaches 6.5 million or more (Non-patent Document 1). Contact infection and aerosol infection have been reported as the main infection route of SARS-CoV-2, and it has been confirmed that SARS-CoV-2 keeps drifting in air with aerosols for about 3 hours and maintains infectivity (Non-patent Document 2). The incubation period is about 2 to 14 days, and cold-like symptoms such as fever (87.9%), dry coughing (67.7%), malaise (38.1%), and phlegm (33.4%) are typical (Non-patent Document 3). In severe cases, respiratory failures caused by acute respiratory distress syndrome, acute pulmonary disorder, interstitial pneumonia, etc. occur. Furthermore, multiple organ failures such as renal failure and hepatic failure have also been reported.

In Japan, as a result of drug repositioning of existing drugs, remdesivir, which is an antiviral drug, dexamethasone, which is an anti-inflammatory drug, and baricitinib, which is an antirheumatic drug, have been approved as therapeutic agents against COVID-19, and in January 2022, tocilizumab, which is an anti-IL-6 receptor antibody, have been received additional approval. Furthermore, in July 2021, ronapreve (casirivimab/imdevimab), which is an antibody cocktail therapy, was approved as special case approval, in September 2021, sotrovimab was approved as special case approval, and in December 2021, molnupiravir was approved as special case approval. Sufficient evidences have not been obtained for efficacy and safety of these medicaments. Therefore, creating a therapeutic agent, particularly, an oral medication for COVID-19 is urgent.

Upon infection of cells, coronaviruses synthesize various proteins required for self-replication. There are two polyproteins in the proteins, replication complexes producing viral genomes, and two proteases are included. Proteases cleave polyproteins synthesized from viruses and act indispensably to cause each protein to function. Of two proteases, a protease mostly taking charge of cleaving polyproteins is a 3CL protease (main protease) (Non-patent Document 4).

Regarding COVID-19 therapeutic agents targeting 3CL proteases, it was published in ClinicalTrials.gov in June 2021 that Phase 1b trials for Lufotrelvir (PF-07304814), which is a prodrug of PF-00835231, have completed by Pfizer Inc (NCT04535167). Furthermore, Pfizer Inc. announced in March 2021 to start Phase 1 test of the therapeutic agent PF-07321332 for coronavirus disease 2019. The structural formulae of PF-00835231, Lufotrelvir and PF-07321332 are as shown below, and these agents are different from the compound according to the present invention in chemical structure (Non-patent Documents 5, 12 and 13 and Patent Documents 5 and 6).

PF-00835231:

[Chemical Formula 1]

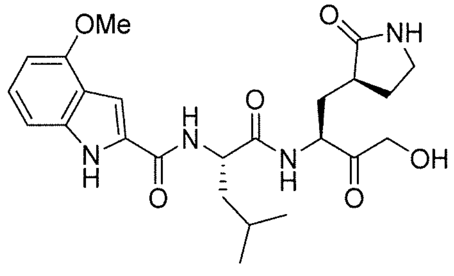

Lufotrelvir(PF-07304814):

[Chemical Formula 2]

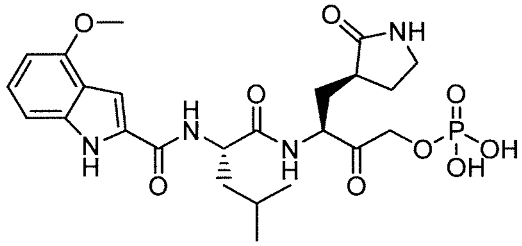

PF-07321332:

[Chemical Formula 3]

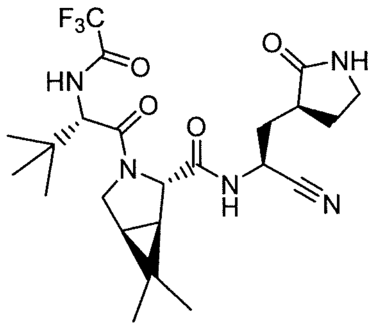

Further, it has been posted on ClinicalTrials.gov in July 2021 that Phase 2/3 test of combination use of PF-07321332 and Ritonavir for COVID-19 patients having high risk factors was started (NCT04960202). Moreover, in November 2021, it was reported on the Pfizer website, PAXLOVID (TM) (PF-07321332; ritonavir) reduced the risk of hospitalization or death by 89% in high-risk adult patients compared to placebo (Non-Patent Document 14). Furthermore, in December 2021, PAXLOVID (TM) was approved for emergency use in the United States, and on February 10, 2022, the Paxlovid (registered trademark) PACK was approved as special case approval in Japan.

Although compounds having 3CL protease inhibitory activity are disclosed in Non-patent Documents 5 to 8, the pharmaceutical composition comprising the compound according to the present invention has neither been described nor suggested in any literatures.

Although triazine derivatives having $P2X_3$ and/or $P2X_{2/3}$ receptor antagonistic activity are disclosed in Patent Documents 1 to 4, the pharmaceutical composition comprising the compound having 3CL protease inhibitory activity and antiviral effect has neither been described nor suggested in any literatures.

Although triazine derivatives having antitumor effects are disclosed in Non-patent Documents 9 to 11, coronavirus 3CL protease inhibitory activity and antiviral effect have not been described in any literatures, and the pharmaceutical composition comprising the compound according to the present invention has neither been described nor suggested.

[PRIOR ART REFERENCES]

[Patent Document]

[Patent Document 1] International Publication WO 2012/020749 A
[Patent Document 2] International Publication WO 2013/089212 A
[Patent Document 3] International Publication WO 2010/092966 A
[Patent Document 4] International Publication WO 2014/200078 A
[Patent Document 5] International Publication WO 2021/205298 A
[Patent Document 6] International Publication WO 2021/250648 A

[Non-patent Document]

[Non-patent Document 1] "COVID-19 Dashboard by the Center for Systems Science and Engineering at Johns Hopkins University", [online], Johns Hopkins University, [searched on September 21, 2022], Internet <URL:https://coronavirus.jhu.edu/map.html>
[Non-patent Document 2] The NEW ENGLAND JOURNAL of MEDICINE (2020), Vol. 382, pp. 1564 to 1567
[Non-patent Document 3] "Report of the WHO-China Joint Mission on Coronavirus Disease 2019 (COVID-19)", [online], February 28, 2020, WHO, [searched on September 21, 2022], Internet <URL: https://www.who.int/docs/default-source/coronaviruse/who-china-joint-mission-on-covid-19-final-report.pdf>
[Non-patent Document 4] Science (2003), Vol. 300, pp. 1763 to 1767
[Non-patent Document 5] "A comparative analysis of SARS-CoV-2 antivirals characterizes 3CLpro inhibitor PF-00835231 as a potential new treatment for COVID-19", Journal of Virology, [online], February 23, 2021, [searched on September 21, 2022], Internet <URL: https://jvi.asm.org/content/early/2021/02/19/JVI.01819-20><doi: 10.1128/JVI.01819-20>
[Non-patent Document 6] Cell Research (2020), Vol. 30, pp. 678 to 692
[Non-patent Document 7] Science (2020), Vol. 368, pp. 409 to 412
[Non-patent Document 8] ACS Central Science (2021), Vol. 7, No. 3, pp. 467 to 475
[Non-patent Document 9] Cancer Treatment Reviews (1984), Vol. 11, Supplement 1, pp. 99 to 110
[Non-patent Document 10] Contributions to Oncology (1984), Vol. 18, pp. 221 to 234
[Non-patent Document 11] Arzneimittel-Forschung (1984), Issue 11, Vol. 6, pp. 663 to 668
[Non-patent Document 12] 261st Am Chem Soc (ACS) Natl Meet - 2021-04-05/2021-04-16 . Virtual, N/A · Abst 243
[Non-patent Document 13] Science (2021), Vol. 374, pp. 1586 to 1593
[Non-patent Document 14] "Pfizer's Novel COVID-19 Oral Antiviral Treatment Candidate Reduced Risk Of Hospitalization Or Death By 89% In Interim Analysis Of Phase 2/3 EPIC-HR Study", [online], November 5, 2021, Pfizer Press Release, [retrieved on September 21, 2022], Internet <URL:https://www.pfizer.com/news/press-release/press-release-detail/pfizers-novel-covid-19-oral-antiviral-treatment-candidate>

[SUMMARY OF THE INVENTION]

[PROBLEMS TO BE SOLVED BY THE INVENTION]

An object of the present invention is to provide a pharmaceutical composition comprising a compound having coronavirus 3CL protease inhibitory activity. Preferably, the present invention provides a medicament comprising a compound having an antiviral activity, particularly, a coronavirus proliferation inhibitory activity.

[MEANS FOR SOLVING THE PROBLEM]

The present invention relates to the following.

(1) A pharmaceutical composition comprising a compound represented by Formula (I):

[Chemical Formula 4]

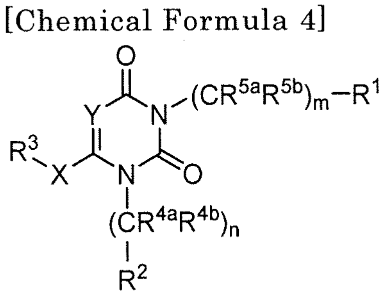

(I)

wherein Y is N;
$R^1$ is substituted or unsubstituted aromatic heterocyclyl;
$R^2$ is substituted or unsubstituted 6-membered aromatic carbocyclyl;
$R^3$ is substituted or unsubstituted aromatic heterocyclyl;

-X- is -NH-;
m is 0 or 1;
R5a is a hydrogen atom;
$R^{5b}$ is a hydrogen atom;
n is 1;
$R^{4a}$ is a hydrogen atom; and
$R^{4b}$ is a hydrogen atom, or a pharmaceutically acceptable salt thereof.

(2) The pharmaceutical composition described in the above item (1), wherein $R^1$ is substituted or unsubstituted 5- to 6-membered aromatic heterocyclyl.
(3) The pharmaceutical composition described in the above item (1) or (2), wherein $R^2$ is 6-membered aromatic carbocyclyl substituted with one, two, or three substituents selected from a substituent group G;
the substituent group G described here is a group consisting of halogen, cyano, and alkyl.
(4) The pharmaceutical composition described in any one of the above items (1) to (3), wherein $R^3$ is substituted or unsubstituted 9- to 10-membered aromatic heterocyclyl.
(5) A pharmaceutical composition comprising the compound described in the above item (1) or a pharmaceutically acceptable salt thereof, wherein the compound represented by Formula (I) is selected from the group consisting of Compound 1-003, Compound 1-005, Compound I-017, and Compound 1-023.
(6) The pharmaceutical composition described in any one of the above items (1) to (5) which is a 3CL protease inhibitor.
(7) The pharmaceutical composition described in any one of the above items (1) to (6) which is used for inhibiting virus proliferation of SARS-CoV-2.
(8) The pharmaceutical composition described in any one of the above items (1) to (7) which is a therapeutic agent and/or prophylactic agent for coronavirus disease 2019 (COVID-19).
(9) The pharmaceutical composition described in any one of the above items (1) to (8) which is used for suppression of exacerbation of infective disease due to SARS-CoV-2.
(10) The pharmaceutical composition described in any one of the above items (1) to (9) which is used such that administration is started within 72 hours from onset of a symptom of infective disease due to SARS-CoV-2 or from SARS-CoV-2 positive diagnosis.
(11) The pharmaceutical composition described in any one of the above items (1) to (9) which is used such that administration is started within 24 hours from onset of a symptom of infective disease due to SARS-CoV-2 or from SARS-CoV-2 positive diagnosis.
(12) The pharmaceutical composition described in any one of the above items (1) to (9) which is used such that administration is started within 120 hours from onset of a symptom of infective disease due to SARS-CoV-2 or from SARS-CoV-2 positive diagnosis.
(13) The pharmaceutical composition described in any one of the above items (1) to (12), wherein the symptom of infective disease due to SARS-CoV-2 has at least one or more of 12 symptoms of COVID-19, and the symptom is moderate to severe symptoms.
(14) The pharmaceutical composition described in any one of the above items (1) to (9) which is used for suppressing viral spread of SARS-CoV-2.
(15) A method for inhibiting virus proliferation of SARS-CoV-2, comprising administering a compound represented by Formula (I):

[Chemical Formula 5]

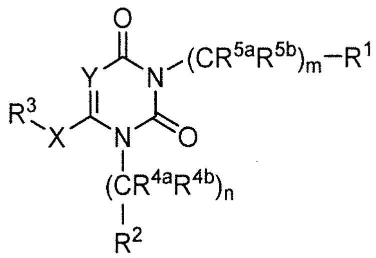

(I)

wherein Y is N;
$R^1$ is substituted or unsubstituted aromatic heterocyclyl;
$R^2$ is substituted or unsubstituted 6-membered aromatic carbocyclyl;
$R^3$ is substituted or unsubstituted aromatic heterocyclyl;

-X- is -NH-;
m is 0 or 1;
$R^{5a}$ is a hydrogen atom;
$R^{5b}$ is a hydrogen atom;
n is 1;
$R^{4a}$ is a hydrogen atom; and
$R^{4b}$ is a hydrogen atom, or a pharmaceutically acceptable salt thereof, to an individual in need of treatment and/or prevention of a novel coronavirus infection (COVID-19).

(16) The method for inhibiting virus proliferation of SARS-CoV-2 described in the above item (15), wherein $R^1$ is substituted or unsubstituted 5- to 6-membered aromatic heterocyclyl.
(17) The method for inhibiting virus proliferation of SARS-CoV-2 described in the above item (15) or (16), wherein $R^2$ is 6-membered aromatic carbocyclyl substituted with one, two, or three substituents selected from a substituent group G;
the substituent group G described here is a group consisting of halogen, cyano, and unsubstituted alkyl.
(18) The method for inhibiting virus proliferation of SARS-CoV-2 described in any one of the above items (15) to (17), wherein $R^3$ is substituted or unsubstituted 9-to 10-membered aromatic heterocyclyl.
(19) The method for inhibiting virus proliferation of SARS-CoV-2 described in the above item (15), wherein the compound represented by Formula (I) is selected from the group consisting of Compound I-003, Compound 1-005, Compound I-017, and Compound I-023.
(20) A method for treating and/or preventing a novel coronavirus infection (COVID-19), comprising administering a compound represented by Formula (I):

[Chemical Formula 6]

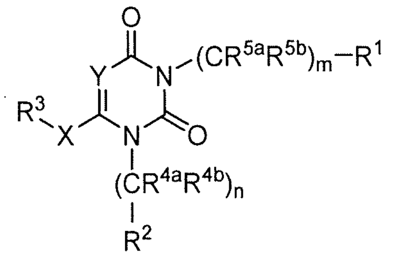

(I)

wherein Y is N;
$R^1$ is substituted or unsubstituted aromatic heterocyclyl;
$R^2$ is substituted or unsubstituted 6-membered aromatic carbocyclyl;
$R^3$ is substituted or unsubstituted aromatic heterocyclyl;
-X- is -NH-;
m is 0 or 1;
$R^{5a}$ is a hydrogen atom;
$R^{5b}$ is a hydrogen atom;
n is 1;
$R^{4a}$ is a hydrogen atom; and
$R^{4b}$ is a hydrogen atom, or a pharmaceutically acceptable salt thereof, to an individual in need of treatment and/or prevention of a novel coronavirus infection (COVID-19).

(21) The method for treating and/or preventing a novel coronavirus infection (COVID-19) described in the above item (20), wherein $R^1$ is substituted or unsubstituted 5- to 6-membered aromatic heterocyclyl.
(22) The method for treating and/or preventing a novel coronavirus infection (COVID-19) described in the above item (20) or (21), wherein $R^2$ is 6-membered aromatic carbocyclyl substituted with one, two, or three substituents selected from a substituent group G;
the substituent group G described here is a group consisting of halogen, cyano, and unsubstituted alkyl.
(23) The method for treating and/or preventing a novel coronavirus infection (COVID-19) described in any one of the above items (20) to (22), wherein $R^3$ is substituted or unsubstituted 9- to 10-membered aromatic heterocyclyl.
(24) The method for treating and/or preventing a novel coronavirus infection (COVID-19) described in the above item (20), wherein the compound represented by Formula (I) is selected from the group consisting of Compound I-003, Compound I-005, Compound I-017, and Compound 1-023.

(25) A method for suppressing exacerbation of infective disease due to SARS-CoV-2, comprising administering a compound represented by Formula (I):

[Chemical Formula 7]

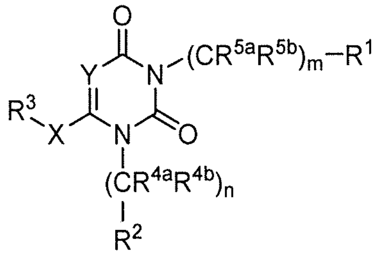

(I)

wherein Y is N;
$R^1$ is substituted or unsubstituted aromatic heterocyclyl;
$R^2$ is substituted or unsubstituted 6-membered aromatic carbocyclyl;
$R^3$ is substituted or unsubstituted aromatic heterocyclyl;
-X- is -NH-;
m is 0 or 1;
$R^{5a}$ is a hydrogen atom;
$R^{5b}$ is a hydrogen atom;
n is 1;
$R^{4a}$ is a hydrogen atom; and
$R^{4b}$ is a hydrogen atom, or a pharmaceutically acceptable salt thereof, to an individual in need of treatment and/or prevention of a novel coronavirus infection (COVID-19).

(26) The method for suppressing exacerbation of infective disease due to SARS-CoV-2 described in the above item (25), wherein $R^1$ is substituted or unsubstituted 5-to 6-membered aromatic heterocyclyl.
(27) The method for suppressing exacerbation of infective disease due to SARS-CoV-2 described in the above item (25) or (26), wherein $R^2$ is 6-membered aromatic carbocyclyl substituted with one, two, or three substituents selected from a substituent group G;
the substituent group G described here is a group consisting of halogen, cyano, and unsubstituted alkyl.
(28) The method for suppressing exacerbation of infective disease due to SARS-CoV-2 described in any one of the above items (25) to (27), wherein $R^3$ is substituted or unsubstituted 9- to 10-membered aromatic heterocyclyl.
(29) The method for suppressing exacerbation of infective disease due to SARS-CoV-2 described in the above item (25), wherein the compound represented by Formula (I) is selected from the group consisting of Compound 1-003, Compound I-005, Compound I-017, and Compound I-023.
(30) A method for treating and/or preventing a novel coronavirus infection (COVID-19), comprising administering a compound represented by Formula (I):

[Chemical Formula 8]

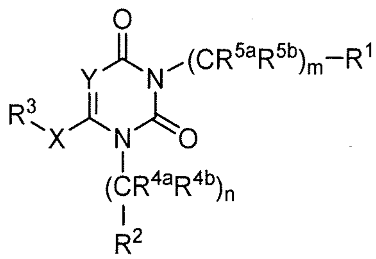

(I)

wherein Y is N;
$R^1$ is substituted or unsubstituted aromatic heterocyclyl;
$R^2$ is substituted or unsubstituted 6-membered aromatic carbocyclyl;
$R^3$ is substituted or unsubstituted aromatic heterocyclyl;
-X- is -NH-;
m is 0 or 1;

$R^{5a}$ is a hydrogen atom;
$R^{5b}$ is a hydrogen atom;
n is 1;
$R^{4a}$ is a hydrogen atom; and
$R^{4b}$ is a hydrogen atom, or a pharmaceutically acceptable salt thereof, which is used such that administration is started within 72 hours from onset of a symptom of infective disease due to SARS-CoV-2 or from SARS-CoV-2 positive diagnosis, to an individual in need of treatment and/or prevention of a novel coronavirus infection (COVID-19).

(31) The method for treating a novel coronavirus infection (COVID-19) described in the above item (30), wherein $R^1$ is substituted or unsubstituted 5- to 6-membered aromatic heterocyclyl.
(32) The method for treating a novel coronavirus infection (COVID-19) described in the above item (30) or (31), wherein $R^2$ is 6-membered aromatic carbocyclyl substituted with one, two, or three substituents selected from a substituent group G;
the substituent group G described here is a group consisting of halogen, cyano, and unsubstituted alkyl.
(33) The method for treating a novel coronavirus infection (COVID-19) described in any one of the above items (30) to (32), wherein $R^3$ is substituted or unsubstituted 9- to 10-membered aromatic heterocyclyl.
(34) The method for treating a novel coronavirus infection (COVID-19) described in the above item (30), wherein the compound represented by Formula (I) is selected from the group consisting of Compound I-003, Compound I-005, Compound I-017, and Compound 1-023.
(35) A method for treating and/or preventing a novel coronavirus infection (COVID-19), comprising administering a compound represented by Formula (I):

[Chemical Formula 9]

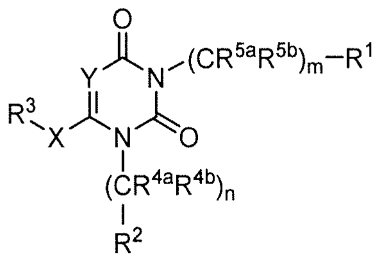

(I)

wherein Y is N;
$R^1$ is substituted or unsubstituted aromatic heterocyclyl;
$R^2$ is substituted or unsubstituted 6-membered aromatic carbocyclyl;
$R^3$ is substituted or unsubstituted aromatic heterocyclyl;
-X- is -NH-;
m is 0 or 1;
$R^{5a}$ is a hydrogen atom;
$R^{5b}$ is a hydrogen atom;
n is 1;
$R^{4a}$ is a hydrogen atom; and
$R^{4b}$ is a hydrogen atom, or a pharmaceutically acceptable salt thereof, which is used such that administration is started within 24 hours from onset of a symptom of infective disease due to SARS-CoV-2 or from SARS-CoV-2 positive diagnosis, to an individual in need of treatment and/or prevention of a novel coronavirus infection (COVID-19).

(36) The method for treating a novel coronavirus infection (COVID-19) described in the above item (35), wherein $R^1$ is substituted or unsubstituted 5- to 6-membered aromatic heterocyclyl.
(37) The method for treating a novel coronavirus infection (COVID-19) described in the above item (35) or (36), wherein $R^2$ is 6-membered aromatic carbocyclyl substituted with one, two, or three substituents selected from a substituent group G;
the substituent group G described here is a group consisting of halogen, cyano, and unsubstituted alkyl.
(38) The method for treating a novel coronavirus infection (COVID-19) described in any one of the above items (35) to (37), wherein $R^3$ is substituted or unsubstituted 9- to 10-membered aromatic heterocyclyl.
(39) The method for treating a novel coronavirus infection (COVID-19) described in the above item (35), wherein

the compound represented by Formula (I) is selected from the group consisting of Compound I-003, Compound I-005, Compound I-017, and Compound I-023.

(40) A method for suppressing viral spread of SARS-CoV-2, comprising administering a compound represented by Formula (I):

[Chemical Formula 10]

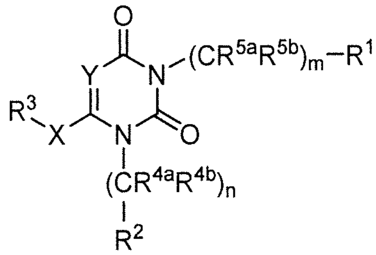

(I)

wherein Y is N;
$R^1$ is substituted or unsubstituted aromatic heterocyclyl;
$R^2$ is substituted or unsubstituted 6-membered aromatic carbocyclyl;
$R^3$ is substituted or unsubstituted aromatic heterocyclyl;
-X- is -NH-;
m is 0 or 1;
$R^{5a}$ is a hydrogen atom;
$R^{5b}$ is a hydrogen atom;
n is 1;
$R^{4a}$ is a hydrogen atom; and
$R^{4b}$ is a hydrogen atom, or a pharmaceutically acceptable salt thereof, to an individual in need of treatment and/or prevention of a novel coronavirus infection (COVID-19).

(41) The method for suppressing viral spread of SARS-CoV-2 described in the above item (40), wherein $R^1$ is substituted or unsubstituted 5- to 6-membered aromatic heterocyclyl.

(42) The method for suppressing viral spread of SARS-CoV-2 described in the above item (40) or (41), wherein $R^2$ is a 6-membered aromatic carbocyclyl substituted with one, two, or three substituents selected from a substituent group G;

the substituent group G described here is a group consisting of halogen, cyano, and unsubstituted alkyl.

(43) The method for suppressing viral spread of SARS-CoV-2 described in any one of the above items (40) to (42), wherein $R^3$ is substituted or unsubstituted 9- to 10-membered aromatic heterocyclyl.

(44) The method for suppressing viral spread of SARS-CoV-2 described in the above item (40), wherein the compound represented by Formula (I) is selected from the group consisting of Compound I-003, Compound I-005, Compound I-017, and Compound I-023.

(45) Use of a compound represented by Formula (I):

[Chemical Formula 11]

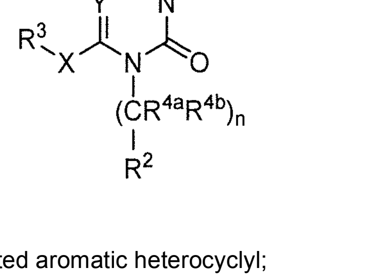

(I)

wherein Y is N;
$R^1$ is substituted or unsubstituted aromatic heterocyclyl;

$R^2$ is substituted or unsubstituted 6-membered aromatic carbocyclyl;
$R^3$ is substituted or unsubstituted aromatic heterocyclyl;
-X- is -NH-;
m is 0 or 1;
$R^{5a}$ is a hydrogen atom;
$R^{5b}$ is a hydrogen atom;
n is 1;
$R^{4a}$ is a hydrogen atom; and
$R^{4b}$ is a hydrogen atom, or a pharmaceutically acceptable salt thereof, for inhibiting virus proliferation of SARS-CoV-2.

(46) The use described in the above item (45), wherein $R^1$ is substituted or unsubstituted 5- to 6-membered aromatic heterocyclyl.
(47) The use described in the above item (45) or (46), wherein $R^2$ is 6-membered aromatic carbocyclyl substituted with one, two, or three substituents selected from a substituent group G;
the substituent group G described here is a group consisting of halogen, cyano, and unsubstituted alkyl.
(48) The use described in any one of the above items (45) to (47), wherein $R^3$ is substituted or unsubstituted 9- to 10-membered aromatic heterocyclyl.
(49) The use described in the above item (45), wherein the compound represented by Formula (I) is selected from the group consisting of Compound I-003, Compound I-005, Compound I-017, and Compound I-023.
(50) Use of a compound represented by Formula (I):

[Chemical Formula 12]

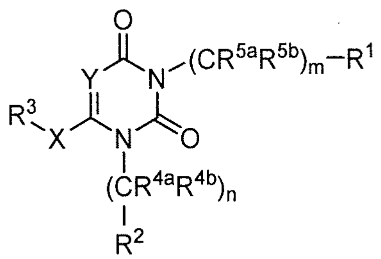

(I)

wherein Y is N;
$R^1$ is substituted or unsubstituted aromatic heterocyclyl;
$R^2$ is substituted or unsubstituted 6-membered aromatic carbocyclyl;
$R^3$ is substituted or unsubstituted aromatic heterocyclyl;
-X- is -NH-;
m is 0 or 1;
$R^{5a}$ is a hydrogen atom;
$R^{5b}$ is a hydrogen atom;
n is 1;
$R^{4a}$ is a hydrogen atom; and
$R^{4b}$ is a hydrogen atom, or a pharmaceutically acceptable salt thereof, for producing a medicament for treatment and/or prevention of novel coronavirus infection (COVID-19).

(51) The use described in the above item (50), wherein $R^1$ is substituted or unsubstituted 5- to 6-membered aromatic heterocyclyl.
(52) The use described in the above item (50) or (51), wherein $R^2$ is 6-membered aromatic carbocyclyl substituted with one, two, or three substituents selected from a substituent group G;
the substituent group G described here is a group consisting of halogen, cyano, and unsubstituted alkyl.
(53) The use described in any one of the above items (50) to (52), wherein $R^3$ is substituted or unsubstituted 9- to 10-membered aromatic heterocyclyl.
(54) The use described in the above item (50), wherein the compound represented by Formula (I) is selected from the group consisting of Compound I-003, Compound I-005, Compound I-017, and Compound I-023.
(55) Use of a compound represented by Formula (I):

[Chemical Formula 13]

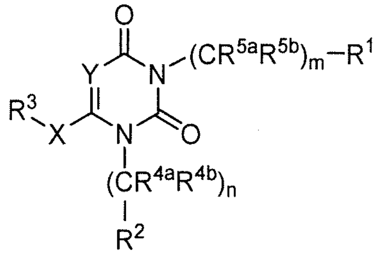

(I)

wherein Y is N;
$R^1$ is substituted or unsubstituted aromatic heterocyclyl;
$R^2$ is substituted or unsubstituted 6-membered aromatic carbocyclyl;
$R^3$ is substituted or unsubstituted aromatic heterocyclyl;
-X- is -NH-;
m is 0 or 1;
$R^{5a}$ is a hydrogen atom;
$R^{5b}$ is a hydrogen atom;
n is 1;
$R^{4a}$ is a hydrogen atom; and
$R^{4b}$ is a hydrogen atom, or a pharmaceutically acceptable salt thereof, for suppressing exacerbation of infective disease due to SARS-CoV-2.

(56) The use described in the above-described item (55), wherein $R^1$ is substituted or unsubstituted 5- to 6-membered aromatic heterocyclyl.
(57) The use described in the above item (55) or (56), wherein $R^2$ is 6-membered aromatic carbocyclyl substituted with one, two, or three substituents selected from a substituent group G;
the substituent group G described here is a group consisting of halogen, cyano, and unsubstituted alkyl.
(58) The use described in any one of the above items (55) to (57), wherein $R^3$ is substituted or unsubstituted 9- to 10-membered aromatic heterocyclyl.
(59) The use described in the above item (55), wherein the compound represented by Formula (I) is selected from the group consisting of Compound 1-003, Compound 1-005, Compound 1-017, and Compound 1-023.
(60) Use of a compound represented by Formula (I):

[Chemical Formula 14]

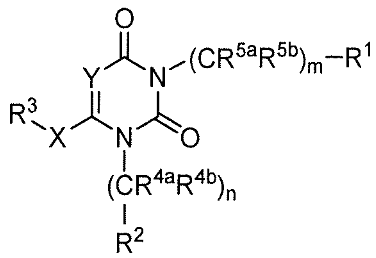

(I)

wherein Y is N;
$R^1$ is substituted or unsubstituted aromatic heterocyclyl;
$R^2$ is substituted or unsubstituted 6-membered aromatic carbocyclyl;
$R^3$ is substituted or unsubstituted aromatic heterocyclyl;
-X- is -NH-;
m is 0 or 1;
$R^{5a}$ is a hydrogen atom;
$R^{5b}$ is a hydrogen atom;
n is 1;
$R^{4a}$ is a hydrogen atom; and
$R^{4b}$ is a hydrogen atom, or a pharmaceutically acceptable salt thereof, which is used such that administration is started within 72 hours from onset of a symptom of infective disease due to SARS-CoV-2 or from SARS-CoV-2 positive diagnosis.

(61) The use described in the above item (60), wherein $R^1$ is substituted or unsubstituted 5- to 6-membered aromatic heterocyclyl.
(62) The use described in the above item (60) or (61), wherein $R^2$ is 6-membered aromatic carbocyclyl substituted with one, two, or three substituents selected from a substituent group G;
the substituent group G described here is a group consisting of halogen, cyano, and unsubstituted alkyl.
(63) The use described in any one of the above items (60) to (62), wherein $R^3$ is substituted or unsubstituted 9- to 10-membered aromatic heterocyclyl.
(64) The use described in the above item (60), wherein the compound represented by Formula (I) is selected from the group consisting of Compound 1-003, Compound I-005, Compound 1-017, and Compound I-023.
(65) Use of a compound represented by Formula (I):

[Chemical Formula 15]

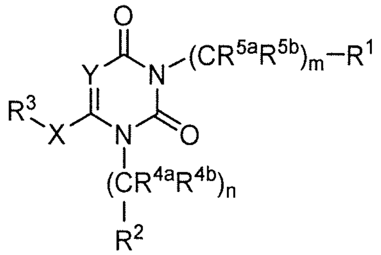

(I)

wherein Y is N;
$R^1$ is substituted or unsubstituted aromatic heterocyclyl;
$R^2$ is substituted or unsubstituted 6-membered aromatic carbocyclyl;
$R^3$ is substituted or unsubstituted aromatic heterocyclyl;
-X- is -NH-;
m is 0 or 1;
$R^{5a}$ is a hydrogen atom;
$R^{5b}$ is a hydrogen atom;
n is 1;
$R^{4a}$ is a hydrogen atom; and
$R^{4b}$ is a hydrogen atom, or a pharmaceutically acceptable salt thereof, which is used such that administration is started within 24 hours from onset of a symptom of infective disease due to SARS-CoV-2 or from SARS-CoV-2 positive diagnosis.

(66) The use described in the above-described item (65), in which $R^1$ is substituted or unsubstituted 5- to 6-membered aromatic heterocyclyl.
(67) The use described in the above-described item (65) or (66), wherein $R^2$ is 6-membered aromatic carbocyclyl substituted with one, two, or three substituents selected from a substituent group G;
the substituent group G described here is a group consisting of halogen, cyano, and unsubstituted alkyl.
(68) The use described in any one of the above items (65) to (67), wherein $R^3$ is substituted or unsubstituted 9- to 10-membered aromatic heterocyclyl.
(69) The use described in the above item (65), wherein the compound represented by Formula (I) is selected from the group consisting of Compound I-003, Compound I-005, Compound I-017, and Compound I-023.
(70) Use of a compound represented by Formula (I):

[Chemical Formula 16]

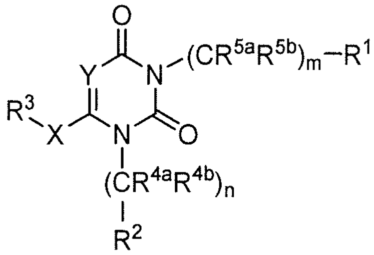

(I)

wherein Y is N;
$R^1$ is substituted or unsubstituted aromatic heterocyclyl;
$R^2$ is substituted or unsubstituted 6-membered aromatic carbocyclyl;
$R^3$ is substituted or unsubstituted aromatic heterocyclyl;
-X- is -NH-;
m is 0 or 1;
$R^{5a}$ is a hydrogen atom;
$R^{5b}$ is a hydrogen atom;
n is 1;
$R^{4a}$ is a hydrogen atom; and
$R^{4b}$ is a hydrogen atom, or a pharmaceutically acceptable salt thereof, for suppressing viral spread of SARS-CoV-2.

(71) The use described in the above-described item (70), wherein $R^1$ is substituted or unsubstituted 5- to 6-membered aromatic heterocyclyl.
(72) The use described in the above item (70) or (71), wherein $R^2$ is 6-membered aromatic carbocyclyl substituted with one, two, or three substituents selected from a substituent group G;
the substituent group G described here is a group consisting of halogen, cyano, and unsubstituted alkyl.
(73) The use described in any one of the above items (70) to (72), wherein $R^3$ is substituted or unsubstituted 9- to 10-membered aromatic heterocyclyl.
(74) The use described in the above item (70), wherein the compound represented by Formula (I) is selected from the group consisting of Compound 1-003, Compound I-005, Compound I-017, and Compound I-023.

[EFFECT OF THE INVENTION]

The compound according to present invention has inhibitory activity against the coronavirus 3CL protease, and the pharmaceutical composition comprising the compound according to the present invention is useful as a therapeutic agent and/or prophylactic agent for coronavirus disease.
Furthermore, a pharmaceutical composition comprising Compound (I-003) or Compound (I-005) of the compound according to the present invention is useful as an active pharmaceutical ingredient.
Further, a pharmaceutical composition comprising p-toluenesulfonate crystal of Compound (1-003) or fumaric acid cocrystal of Compound (1-005) is extremely useful as a therapeutic agent for coronavirus disease 2019 (COVID-19).

[BRIEF DESCRIPTION OF DRAWINGS]

Fig. 1 shows X-ray powder diffraction patterns of p-toluenesulfonate crystal Form I (Form I) of a compound represented by Formula (I-A). The horizontal axis represents 2θ (°) and the vertical axis represents intensity (Count).
Fig. 2 shows the structure of p-toluenesulfonate crystal Form I of the compound represented by Formula (I-A) in an asymmetric unit.
Fig. 3 shows X-ray powder diffraction patterns of fumaric acid cocrystal Form I (Form I) of a compound represented by Formula (I-B). The horizontal axis represents 2θ (°) and the vertical axis represents intensity (Count).
Fig. 4 shows the structure of fumaric acid cocrystal Form I (Form I) of the compound represented by Formula (I-B) in an asymmetric unit.
Fig. 5A shows lung virus titers 1 day after infection when a vehicle was administered twice a day or fumaric acid cocrystal Form I crystal of the compound represented by (I-B) was administered in a single dose immediately after

infection into hCoV-19/Japan/TY7-501/2021-infected mice ($1.00 \times 10^4$ $TCID_{50}$/mice were intranasally inoculated). The vertical axis represents lung virus titers and the horizontal axis represents each administered group.

Fig. 5B shows lung virus titers 1 day after infection when a vehicle and fumaric acid cocrystal Form I crystal of the compound represented by (I-B) were administered twice a day immediately after infection into hCoV-19/Japan/TY7-501/2021-infected mice ($1.00 \times 10^4$ $TCID_{50}$/mice were intranasally inoculated). The vertical axis represents lung virus titers and the horizontal axis represents each administered group.

Fig. 6A shows lung virus titers 1 to 3 days after infection when a vehicle and fumaric acid cocrystal Form I crystal of the compound represented by (I-B) were administered twice a day for two days starting 1 day after infection into hCoV-19/Japan/TY7-501/2021-infected mice ($1.00 \times 10^4$ $TCID_{50}$/mice were intranasally inoculated). The vertical axis represents lung virus titers and the horizontal axis represents days from infection.

Fig. 6B shows lung virus titers 1 to 5 days after infection when a vehicle and fumaric acid cocrystal Form I crystal of the compound represented by (I-B) were administered twice a day for two days starting 3 days after infection into hCoV-19/Japan/TY7-501/2021-infected mice ($1.00 \times 10^4$ $TCID_{50}$/mice were intranasally inoculated). The vertical axis represents lung virus titers and the horizontal axis represents days from infection.

Fig. 7 shows lung virus titers 1 to 3 days after infection when a vehicle and fumaric acid cocrystal Form I crystal of the compound represented by (I-B) were administered three times a day for two days starting 1 day after infection into hCoV-19/Japan/TY7- 501/202 1 -infected mice ($1.00 \times 10^4$ $TCID_{50}$/mice were intranasally inoculated). The vertical axis represents lung virus titers and the horizontal axis represents days from infection.

Fig. 8A shows body weight until 7 days after infection when a vehicle and fumaric acid cocrystal Form I crystal of the compound represented by (I-B) were administered twice a day for five days starting 1 day after infection into hCoV-19/Japan/TY7-501/202 1 -infected mice ($1.00 \times 10^5$ $TCID_{50}$/mice were intranasally inoculated, aged 35 to 45-week-old retired mice). The vertical axis represents body weight change based on the body weight on the day of infection as 100% and the horizontal axis represents days from infection.

Fig. 8B shows a survival rate until 7 days after infection when a vehicle and fumaric acid cocrystal Form I crystal of the compound represented by (I-B) were administered twice a day for five days starting 1 day after infection into hCoV-19/Japan/TY7-501/2021-infected mice ($1.00 \times 10^5$ $TCID_{50}$/mice were intranasally inoculated, aged 35 to 45-week-old retired mice). The vertical axis represents the survival rate (%) and the horizontal axis represents days from infection.

Fig. 8C shows body weight change until 7 days after infection when a vehicle and fumaric acid cocrystal Form I crystal of the compound represented by (I-B) were administered twice a day for five days starting 1 day afterinfection into hCoV19/Japan/TY/WK-521/2020 strain mouse-adapted strain MA-P10-infected mice ($1.00 \times 10^3$ or $1.00 \times 10^4$ $TCID_{50}$/mice were intranasally inoculated, 15-week-old mice). The vertical axis represents body weight change based on the body weight on the day of infection as 100% and the horizontal axis represents days from infection.

Fig. 8D shows a survival rate until 7 days after infection when a vehicle and fumaric acid cocrystal Form I crystal of the compound represented by (I-B) were administered twice a day for five days starting 1 day after infection into hCoV19/Japan/TY/WK-521/2020 strain mouse-adapted strain MA-P10-infected mice ($1.00 \times 10^3$ or $1.00 \times 10^4$ $TCID_{50}$/mice were intranasally inoculated, 15-week-old mice). The vertical axis represents the survival rate (%) and the horizontal axis represents days from infection.

Fig. 8E shows body weight change until 7 days after infection when a vehicle and fumaric acid cocrystal Form I crystal of the compound represented by (I-B) were administered twice a day for five days starting 1 day after infection into hCoV19/Japan/TY/WK-521/2020 strain mouse-adapted strain MA-P10-infected mice ($1.00 \times 10^3$ $TCID_{50}$/mice were intranasally inoculated, aged 35 to 45-week-old retired mice). The vertical axis represents body weight change based on the body weight on the day of infection as 100% and the horizontal axis represents days from infection.

Fig. 8F shows a survival rate until 7 days after infection when a vehicle and fumaric acid cocrystal Form I crystal of the compound represented by (I-B) were administered twice a day for five days starting 1 day afterf infection into hCoV19/Japan/TY/WK-521/2020 strain mouse-adapted strain MA-P10-infected mice ($1.00 \times 10^3$ $TCID_{50}$/mice were intranasally inoculated, aged 35 to 45-week-old retired mice). The vertical axis represents the survival rate (%) and the horizontal axis represents days from infection.

Fig. 9 shows lung virus titers of administered-hamsters 6 days after infection when hCoV19/Japan/TY11-927/2021-infected hamsters ($5.00 \times 10^3$ PFU/hamsters were intranasally inoculated) and the administered-hamsters that were not inoculated with virus were co-housed. The administered-hamsters were administered with fumaric acid cocrystal Form I crystal of the compound represented by (I-B) twice a day immediately after infection. The vertical axis represents lung virus titers and the horizontal axis represents each administered group.

Fig. 10 shows lung virus titers of infected (Infected) and uninfected (Contact) hamsters 6 days after infection when a compound was administered to hCoV19/Japan/TY11-927/2021-infected hamsters ($5.00 \times 10^3$ PFU/hamsters were intranasally inoculated) and uninfected hamsters not inoculated with virus were co-housed immediately after infection. The administered-hamsters were administered with fumaric acid cocrystal Form I crystal of the compound represented by (I-B) twice a day immediately after infection. The vertical axis represents lung virus titers and the

horizontal axis represents each administered group.

Fig. 11A shows lung virus titers of infected (Infected) and uninfected (Contact) hamsters 5 days after infection when a compound was administered to hCoV19/Japan/TY11-927/2021-infected hamsters ($1.00 \times 10^2$ $TCID_{50}$/hamsters were intranasally inoculated) and uninfected hamsters not inoculated with virus were co-housed from 2 days after infection. The administered-hamsters were administered with fumaric acid cocrystal Form I crystal of the compound represented by (I-B) twice a day starting 1 day after infection. The vertical axis represents lung virus titers and the horizontal axis represents each administered group.

Fig. 11B shows lung virus titers of infected (Infected) and uninfected (Contact) hamsters 5 days after infection when a compound was administered to hCoV19/Japan/TY11-927/2021-infected hamsters ($1.00 \times 10^2$ $TCID_{50}$/hamsters were intranasally inoculated) and uninfected hamsters not inoculated with virus were co-housed from 2 days after infection. The administered hamsters were administered with fumaric acid cocrystal Form I crystal of the compound represented by (I-B) twice a day starting 1 day after infection. The vertical axis represents nasal turbinate virus titers and the horizontal axis represents each administered group.

Fig. 12A shows body weight change until 14 days after infection when a vehicle and the compound represented by (I-B) were subcutaneously administered 24 hours before infection into hCoV19/Japan/TY/WK-521/2020 strain mouse-adapted strain MA-P10-infected mice ($3.00 \times 10^2$ $TCID_{50}$/mice were intranasally inoculated, 37 to 57-week-old mice). The vertical axis represents body weight change based on the body weight on the day of infection as 100% and the horizontal axis represents days from infection.

Fig. 12B shows a survival rate until 14 days after infection when a vehicle and the compound represented by (I-B) were subcutaneously administered 24 hours before infection into hCoV19/Japan/TY/WK-521/2020 strain mouse-adapted strain MA-P10-infected mice ($3.00 \times 10^2$ $TCID_{50}$/mice were intranasally inoculated, 37 to 57-week-old mice). The vertical axis represents the survival rate (%) and the horizontal axis represents days from infection.

[MODE FOR CARRYING OUT THE INVENTION]

The meanings o, each tf the terms as used herein are described below. Unless otherwise specifiederm has the same meaning when used alone or in combination with other terms.

The term "consisting of" means to have only the described elements.

The term "comprising" means not to limit to the described elements and not to exclude undescribed elements.

Hereinafter, the present invention will be described with showing embodiments. It should be understood that, throughout the present specification, the expression of a singular form includes the concept of its plural form unless specified otherwise. Therefore, it should be understood that the article of the singular form (for example, in English, "a", "an", "the", and the like) includes the concept of its plural form unless specified otherwise.

Furthermore, it should be understood that the terms used herein are used in a meaning normally used in the art unless specified otherwise. Thus, unless defined otherwise, all technical and scientific terms used herein have the same meaning as those generally understood by those skilled in the art in the field to which the present invention pertains. If there is a contradiction, the present specification (including definitions) precedes.

"Halogen" includes a fluorine atom, a chlorine atom, a bromine atom, and an iodine atom. In particular, halogen is preferably a fluorine atom and a chlorine atom.

"Alkyl" includes a linear or branched hydrocarbon group having 1 to 15 carbon atoms, preferably 1 to 10 carbon atoms, more preferably 1 to 6 carbon atoms, and further preferably 1 to 4 carbon atoms. Examples thereof include methyl, ethyl, n-propyl, isopropyl, n-butyl, isobutyl, sec-butyl, tert-butyl, n-pentyl, isopentyl, neopentyl, n-hexyl, isohexyl, n-heptyl, isoheptyl, n-octyl, isooctyl, n-nonyl, and n-decyl.

Examples of a preferred embodiment of "alkyl" include methyl, ethyl, n-propyl, isopropyl, n-butyl, isobutyl, sec-butyl, tert-butyl, and n-pentyl. Examples of a further preferred embodiment thereof include methyl, ethyl, n-propyl, isopropyl, and tert-butyl.

"Alkenyl" includes a linear or branched hydrocarbon group having one or more double bond(s) at any position(s) which has 2 to 15 carbon atoms, preferably 2 to 10 carbon atoms, more preferably 2 to 6 carbon atoms, and further preferably 2 to 4 carbon atoms. Examples thereof include vinyl, allyl, propenyl, isopropenyl, butenyl, isobutenyl, prenyl, butadienyl, pentenyl, isopentenyl, pentadienyl, hexenyl, isohexenyl, hexadienyl, heptenyl, octenyl, nonenyl, decenyl, undecenyl, dodecenyl, tridecenyl, tetradecenyl, and pentadecenyl.

Examples of a preferred embodiment of "alkenyl" include vinyl, allyl, propenyl, isopropenyl, and butenyl. Examples of a further preferred embodiment include ethenyl and n-propenyl.

"Alkynyl" includes a linear or branched hydrocarbon group having one or more triple bond(s) at any position(s) which has 2 to 10 carbon atoms, preferably 2 to 8 carbon atoms, more preferably 2 to 6 carbon atoms, and further preferably 2 to 4 carbon atoms. Further, "alkynyl" may have double bond(s) at any position(s). For example, "alkynyl" includes ethynyl, propynyl, butynyl, pentynyl, hexynyl, heptynyl, octynyl, nonynyl, decynyl, and the like.

Examples of a preferred embodiment of "alkynyl" include ethynyl, propynyl, butynyl, and pentynyl. Examples

of a further preferred embodiment thereof include ethynyl and propynyl.

"Aromatic carbocyclyl" means a cyclic aromatic hydrocarbon group which is monocyclic or polycyclic having two or more rings. Examples thereof include phenyl, naphthyl, anthryl, and phenanthryl.

Examples of a preferred embodiment of the "aromatic carbocyclyl" include phenyl.

"6-membered aromatic carbocyclyl" means a cyclic aromatic hydrocarbon group which is monocyclic. Examples thereof include phenyl.

"Non-aromatic carbocyclyl" means a cyclic saturated hydrocarbon group or a cyclic unsaturated non-aromatic hydrocarbon group which is monocyclic or polycyclic having two or more rings. The "non-aromatic carbocyclyl" which is polycyclic having two or more rings also includes a fused ring group wherein a non-aromatic carbocyclyl, which is monocyclic or polycyclic having two or more rings, is fused with a ring of the above-described "aromatic carbocyclyl".

Further, the "non-aromatic carbocyclyl" also includes a group having a bridge or a group to form a spiro ring as follows.

[Chemical Formula 17]

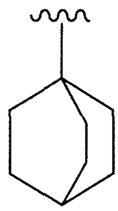
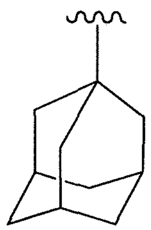
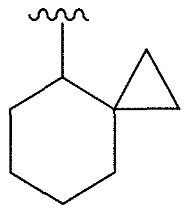

The non-aromatic carbocyclyl which is monocyclic is carbocyclyl having preferably 3 to 16 carbon atoms, more preferably 3 to 12 carbon atoms, and further preferably 4 to 8 carbon atoms. Examples thereof include cyclopropyl, cyclobutyl, cyclopentyl, cyclohexyl, cycloheptyl, cyclooctyl, cyclononyl, cyclodecyl, cyclopropenyl, cyclobutenyl, cyclopentenyl, cyclohexenyl, cycloheptenyl, and cyclohexadienyl.

The non-aromatic carbocyclyl which is polycyclic having two or more rings is carbocyclyl having preferably 8 to 20 carbon atoms and more preferably 8 to 16 carbon atoms. Examples thereof include indanyl, indenyl, acenaphthyl, tetrahydronaphthyl, and fluorenyl.

"Aromatic heterocyclyl" means an aromatic cyclyl, which is monocyclic or polycyclic having two or more rings, having one or more, same or different heteroatom(s) selected optionally from O, S, and N.

The aromatic heterocyclyl which is polycyclic having two or more rings include a fused ring group wherein aromatic heterocyclyl, which is monocyclic or polycyclic having two or more rings, is fused with a ring of the above-described "aromatic carbocyclyl" and may have the binding group at any ring(s).

The aromatic heterocyclyl which is monocyclic is preferably a 5- to 8-membered ring and more preferably a 5- or 6-membered ring. Examples of the 5-membered aromatic heterocyclyl include pyrrolyl, imidazolyl, pyrazolyl, triazolyl, tetrazolyl, furyl, thienyl, isoxazolyl, oxazolyl, oxadiazolyl, isothiazolyl, thiazolyl, and thiadiazolyl. Examples of the 6-membered aromatic heterocyclyl include pyridyl, pyridazinyl, pyrimidinyl, pyrazinyl, and triazinyl.

The aromatic heterocyclyl which is bicyclic is preferably an 8- to 10-membered ring and more preferably a 9- or 10-membered ring. Examples thereof include indolyl, isoindolyl, indazolyl, indolizinyl, quinolinyl, isoquinolinyl, cinnolinyl, phthalazinyl, quinazolinyl, naphthyridinyl, quinoxalinyl, purinyl, pteridinyl, benzimidazolyl, benzisoxazolyl, benzoxazolyl, benzoxadiazolyl, benzisothiazolyl, benzothiazolyl, benzothiadiazolyl, benzofuryl, isobenzofuryl, benzothienyl, benzotriazolyl, imidazopyridyl, triazolopyridyl, imidazothiazolyl, pyrazinopyridazinyl, oxazolopyridyl, and thiazolopyridyl. Examples of the 9-membered aromatic heterocyclyl include indolyl, isoindolyl, indazolyl, indolizinyl, purinyl, benzimidazolyl, benzisoxazolyl, benzoxazolyl, benzoxadiazolyl, benzisothiazolyl, benzothiazolyl, benzothiadiazolyl, benzotriazolyl, benzofuranyl, imidazopyridyl, triazolopyridyl, oxazolopyridyl, and thiazolopyridyl. Examples of the 10-membered aromatic heterocyclyl include quinolinyl, isoquinolinyl, cinnolinyl, phthalazinyl, quinazolinyl, naphthyridinyl, quinoxalinyl, pteridinyl, and pyrazinopyridazinyl.

The aromatic heterocyclyl which is polycyclic having three or more rings is preferably a 13- to 15-membered ring. Examples thereof include carbazolyl, acridinyl, xanthenyl, phenothiazinyl, phenoxathiinyl, phenoxazinyl, and dibenzofuryl.

The "5- to 6-membered aromatic heterocyclyl" means a 5- or 6-membered aromatic heterocyclyl of the above "aromatic heterocyclyl".

The "9- to 10-membered aromatic heterocyclyl" means a 9- or 10-membered aromatic heterocyclyl of the above-described "aromatic heterocyclyl".

"Non-aromatic heterocyclyl" means a non-aromatic cyclyl, which is monocyclic or polycyclic having two or more rings, having one or more, same or different heteroatom(s) selected optionally from O, S, and N. The non-aromatic

heterocyclyl which is polycyclic having two or more rings also includes a fused ring group wherein a non-aromatic heterocyclyl, which is monocyclic or polycyclic having two or more rings, is fused with a ring of each of the above "aromatic carbocyclyl", "non-aromatic carbocyclyl", and/or "aromatic heterocyclyl" and further, a fused ring group wherein a non-aromatic carbocyclyl, which is monocyclic or polycyclic having two or more rings, is fused with a ring of the above-described "aromatic heterocyclyl", and may have the binding group at any ring(s).

Further, the "non-aromatic heterocyclyl" also includes a group having a bridge or a group to form a spiro ring as follows.

[Chemical Formula 18]

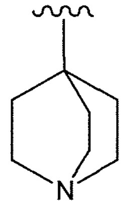

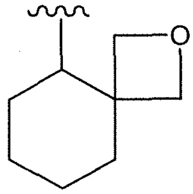

The non-aromatic heterocyclyl which is monocyclic is preferably a 3- to 8-membered ring and more preferably a 5- or 6-membered ring.

Examples of the 3-membered non-aromatic heterocyclyl include thiiranyl, oxiranyl, and aziridinyl. Examples of the 4-membered non-aromatic heterocyclyl include oxetanyl and azetidinyl. Examples of the 5-membered non-aromatic heterocyclyl include oxathiolanyl, thiazolidinyl, pyrrolidinyl, pyrrolinyl, imidazolidinyl, imidazolinyl, pyrazolidinyl, pyrazolinyl, tetrahydrofuryl, dihydrothiazolyl, tetrahydroisothiazolyl, dioxolanyl, dioxolyl, and thiolanyl. Examples of the 6-membered non-aromatic heterocyclyl include dioxanyl, thianyl, piperidyl, piperazinyl, morpholinyl, morpholino, thiomorpholinyl, thiomorpholino, dihydropyridyl, tetrahydropyridyl, tetrahydropyranyl, dihydrooxazinyl, tetrahydropyridazinyl, hexahydropyrimidinyl, dioxazinyl, thiinyl, and thiazinyl. Examples of the 7-membered non-aromatic heterocyclyl include hexahydroazepinyl, tetrahydrodiazepinyl, and oxepanyl.

The non-aromatic heterocyclyl which is polycyclic having two or more rings is preferably an 8- to 20-membered ring, more preferably an 8- to 13-membered ring, and further preferably 8- to 10-membered ring. Examples thereof include indolinyl, isoindolinyl, chromanyl, and isochromanyl.

The expression "may be substituted with a substituent group α" herein means "may be substituted with one or more groups selected from a substituent group α". The same is true in substituent groups 8, γ, and γ'.

Substituent group α: halogen, hydroxy, carboxy, alkyloxy, haloalkyloxy, alkenyloxy, alkynyloxy, sulfanyl, and cyano.

Substituent group β: halogen, hydroxy, carboxy, cyano, alkyl which may be substituted with the substituent group α, alkenyl which may be substituted with the substituent group α, alkynyl which may be substituted with the substituent group α, alkylcarbonyl which may be substituted with the substituent group α, alkenylcarbonyl which may be substituted with the substituent group α, alkynylcarbonyl which may be substituted with the substituent group α, alkylsulfanyl which may be substituted with the substituent group α, alkenylsulfanyl which may be substituted with the substituent group α, alkynylsulfanyl which may be substituted with the substituent group α, alkylsulfinyl which may be substituted with the substituent group α, alkenylsulfinyl which may be substituted with the substituent group α, alkynylsulfinyl which may be substituted with the substituent group α, alkylsulfonyl which may be substituted with the substituent group α, alkenylsulfonyl which may be substituted with the substituent group α, alkynylsulfonyl which may be substituted with the substituent group α,

an aromatic carbocyclyl which may be substituted with the substituent group γ, a non-aromatic carbocyclyl which may be substituted with the substituent group y', an aromatic heterocyclyl which may be substituted with the substituent group γ, a non-aromatic heterocyclyl which may be substituted with the substituent group γ', aromatic carbocyclylalkyl which may be substituted with the substituent group γ, non-aromatic carbocyclylalkyl which may be substituted with the substituent group y', aromatic heterocyclylalkyl which may be substituted with the substituent group γ, non-aromatic heterocyclylalkyl which may be substituted with the substituent group y', aromatic carbocyclylcarbonyl which may be substituted with the substituent group γ, non-aromatic carbocyclylcarbonyl which may be substituted with the substituent group γ', aromatic heterocyclylcarbonyl which may be substituted with the substituent group γ, non-aromatic heterocyclylcarbonyl which may be substituted with the substituent group γ', aromatic carbocyclyloxycarbonyl which may be substituted with the substituent group γ, non-aromatic carbocyclyloxycarbonyl which may be substituted with the substituent group γ', aromatic heterocyclyloxycarbonyl which may be substituted with the substituent group γ, non-aromatic heterocyclyloxycarbonyl which may be substituted with the substituent group γ', aromatic carbocyclylsulfanyl which may be substituted with the substituent group γ, non-aromatic carbocyclylsulfanyl which may be substituted with the substituent group γ', aromatic heterocyclylsulfanyl which may be substituted with the substituent group γ, non-aromatic heterocyclylsulfanyl which may be substituted with the substituent group γ', aromatic carbocyclylsulfinyl which may be substituted with the substituent group γ, non-aromatic carbocyclylsulfinyl which may be substituted with the substituent group γ', aromatic heterocyclylsulfinyl which may be substituted with the substituent group γ, non-aromatic heterocyclylsulfinyl which may be substituted with the substituent group γ', aromatic carbocyclylsulfonyl which may be substituted with the substituent group γ, non-aromatic carbocyclylsulfonyl which may be substituted with the substituent group γ', aromatic heterocyclylsulfonyl which may be substituted with the substituent group γ, and non-aromatic heterocyclylsulfonyl which may be substituted with the substituent group y'.

Substituent group γ: substituent group α, alkyl, haloalkyl, hydroxyalkyl, alkenyl, alkynyl, alkylcarbonyl, haloalkylcarbonyl, alkenylcarbonyl, and alkynylcarbonyl.

Substituent group y': substituent group y and oxo.

Examples of substituents on the ring of "aromatic carbocycle" and "aromatic heterocycle" of "substituted aromatic carbocyclyl" and "substituted aromatic heterocyclyl" include the following substituent group B. An atom at any position(s) on the ring may be bonded to one or more group(s) selected from the following substituent group B.

Substituent group B: halogen, hydroxy, carboxy, formyl, formyloxy, sulfanyl, sulfino, sulfo, thioformyl, thiocarboxy, dithiocarboxy, thiocarbamoyl, cyano, nitro, nitroso, azide, hydrazino, ureide, amidino, guanidino, pentafluorothio, trialkylsilyl,

alkyl which may be substituted with the substituent group α, alkenyl which may be substituted with the substituent group α, alkynyl which may be substituted with the substituent group α, alkyloxy which may be substituted with the substituent group α, alkenyloxy which may be substituted with the substituent group α, alkynyloxy which may be substituted with the substituent group α, alkylcarbonyloxy which may be substituted with the substituent group α, alkenylcarbonyloxy which may be substituted with the substituent group α, alkynylcarbonyloxy which may be substituted with the substituent group α, alkylcarbonyl which may be substituted with the substituent group α, alkenylcarbonyl which may be substituted with the substituent group α, alkynylcarbonyl which may be substituted with the substituent group α, alkyloxycarbonyl which may be substituted with the substituent group α, alkenyloxycarbonyl which may be substituted with the substituent group α, alkynyloxycarbonyl which may be substituted with the substituent group α, alkylsulfanyl which may be substituted with the substituent group α, alkenylsulfanyl which may be substituted with the substituent group α, alkynylsulfanyl which may be substituted with the substituent group α, alkylsulfinyl which may be substituted with the substituent group α, alkenylsulfinyl which may be substituted with the substituent group α, alkynylsulfinyl which may be substituted with the substituent group α, alkylsulfonyl which may be substituted with the substituent group α, alkenylsulfonyl which may be substituted with the substituent group α, alkynylsulfonyl which may be substituted with the substituent group α,

amino which may be substituted with the substituent group 8, imino which may be substituted with the substituent group β, carbamoyl which may be substituted with the substituent group 8, sulfamoyl which may be substituted with the substituent group β,

an aromatic carbocyclyl which may be substituted with the substituent group γ, a non-aromatic carbocyclyl which may be substituted with the substituent group γ', an aromatic heterocyclyl which may be substituted with the substituent group γ, a non-aromatic heterocyclyl which may be substituted with the substituent group γ', aromatic carbocyclyloxy which may be substituted with the substituent group γ, non-aromatic carbocyclyloxy which may be substituted with the substituent group γ', aromatic heterocyclyloxy which may be substituted with the substituent group γ, non-aromatic heterocyclyloxy which may be substituted with the substituent group γ', aromatic carbocyclylcarbonyloxy which may be substituted with the substituent group γ, non-aromatic carbocyclylcarbonyloxy which may be substituted with the substituent group γ', aromatic heterocyclylcarbonyloxy which may be substituted with the substituent group γ, non-aromatic heterocyclylcarbonyloxy which may be substituted with the substituent group γ', aromatic carbocyclylcarbonyl which may be substituted with the substituent group γ, non-aromatic carbocyclylcarbonyl which may be substituted with the substituent group γ', aromatic heterocyclylcarbonyl which may be substituted with the substituent group γ, non-aromatic heterocyclylcarbonyl which may be substituted with the substituent group y', aromatic carbocyclyloxycarbonyl which may be substituted with the substituent group γ, non-aromatic carbocyclyloxycarbonyl which may be substituted with the substituent group γ', aromatic heterocyclyloxycarbonyl which may be substituted with the substituent group γ, non-aromatic heterocyclyloxycarbonyl which may be substituted with the substituent group γ', aromatic carbocyclylalkyl which may be substituted with the substituent group γ, non-aromatic carbocyclylalkyl which may be substituted with the substituent group γ', aromatic heterocyclylalkyl which may be substituted with the substituent group γ, non-aromatic heterocyclylalkyl which may be substituted with the substituent group γ', aromatic carbocyclylalkyloxy which may be substituted with the substituent group γ, non-aromatic carbocyclylalkyloxy which may be substituted with the substituent group γ', aromatic heterocyclylalkyloxy which may be substituted with the substituent group γ, non-aromatic heterocyclylalkyloxy which may be substituted with the

substituent group γ', aromatic carbocyclylalkyloxycarbonyl which may be substituted with the substituent group γ, non-aromatic carbocyclylalkyloxycarbonyl which may be substituted with the substituent group γ', aromatic heterocyclylalkyloxycarbonyl which may be substituted with the substituent group γ, non-aromatic heterocyclylalkyloxycarbonyl which may be substituted with the substituent group γ', aromatic carbocyclylalkyloxyalkyl which may be substituted with the substituent group γ, non-aromatic carbocyclylalkyloxyalkyl which may be substituted with the substituent group γ', aromatic heterocyclylalkyloxyalkyl which may be substituted with the substituent group γ, non-aromatic heterocyclylalkyloxyalkyl which may be substituted with the substituent group γ', aromatic carbocyclylsulfanyl which may be substituted with the substituent group γ, non-aromatic carbocyclylsulfanyl which may be substituted with the substituent group γ', aromatic heterocyclylsulfanyl which may be substituted with the substituent group γ, non-aromatic heterocyclylsulfanyl which may be substituted with the substituent group γ', aromatic carbocyclylsulfinyl which may be substituted with the substituent group γ, non-aromatic carbocyclylsulfinyl which may be substituted with the substituent group γ', aromatic heterocyclylsulfinyl which may be substituted with the substituent group γ, non-aromatic heterocyclylsulfinyl which may be substituted with the substituent group γ', aromatic carbocyclylsulfonyl which may be substituted with the substituent group γ, non-aromatic carbocyclylsulfonyl which may be substituted with the substituent group γ', aromatic heterocyclylsulfonyl which may be substituted with the substituent group γ, and non-aromatic heterocyclylsulfonyl which may be substituted with the substituent group γ'.

Examples of substituents on the ring of "non-aromatic carbocycle" and "non-aromatic heterocycle" of "substituted non-aromatic carbocyclyl" and "substituted non-aromatic heterocyclyl" include the following substituent group C. An atom at any position(s) on the ring may be bonded to one or more group(s) selected from the following substituent group C.

Substituent group C: substituent group B and oxo.

When the "non-aromatic carbocycle" and the "non-aromatic heterocycle" are substituted with "oxo", it means a ring in which two hydrogen atoms on the carbon atom are substituted as below.

[Chemical Formula 19]

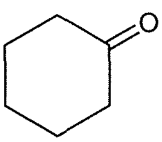
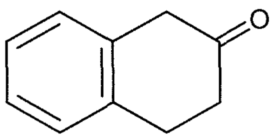
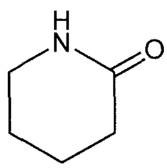
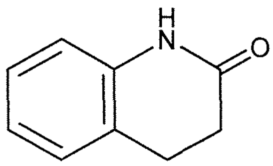

Examples of substituents of the "substituted or unsubstituted aromatic heterocyclyl" or the "substituted or unsubstituted 5- to 6-membered aromatic heterocyclyl" in $R^1$ include

halogen; and
substituted or unsubstituted alkyl. It may be substituted with one or more group(s) selected from the above substituents.

Examples of substituents of the "substituted or unsubstituted aromatic heterocyclyl" or the "substituted or unsubstituted 5- to 6-membered aromatic heterocyclyl" in $R^1$ include

halogen;
substituted alkyl (as the substituent, hydroxy); and unsubstituted alkyl. It may be substituted with one or more group(s) selected from the above substituents.

Examples of substituents of the "substituted or unsubstituted 6-membered aromatic carbocyclyl" in $R^2$ include

halogen; cyano; and
substituted or unsubstituted alkyl. It may be substituted with one or more group(s) selected from the above substituents.

Examples of substituents of the "substituted or unsubstituted 6-membered aromatic carbocyclyl" in $R^2$ include

halogen; cyano;
substituted alkyl (as the substituent, halogen); and unsubstituted alkyl. It may be substituted with one or more group(s) selected from the above substituents.

Examples of substituents of the "substituted or unsubstituted aromatic heterocyclyl" or the "substituted or unsubstituted 9- to 10-membered aromatic heterocyclyl" in $R^3$ include

halogen;
substituted or unsubstituted alkyl; and
substituted or unsubstituted non-aromatic heterocyclyl. It may be substituted with one or more group(s) selected from the above substituents.

Examples of substituents of the "substituted or unsubstituted aromatic heterocyclyl" or the "substituted or unsubstituted 9- to 10-membered aromatic heterocyclyl" in $R^3$ include

halogen;
substituted alkyl (as the substituent, halogen, hydroxy, alkylcarbonylamino, or a non-aromatic heterocyclyl); unsubstituted alkyl;
substituted non-aromatic heterocyclyl (as the substituent, alkylcarbonyl); and unsubstituted non-aromatic heterocyclyl. It may be substituted with one or more group(s) selected from the above substituents.

Preferred embodiments of $R^1$, $R^2$, $R^3$, and m in the compound represented by Formula (I) are described below:

[Chemical Formula 20]

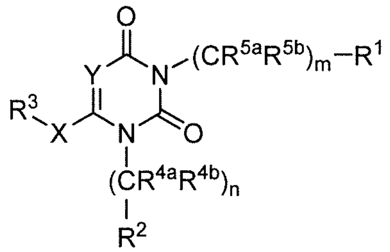

(I)

Examples of the compound represented by Formula (I) include embodiments of all combinations of specific examples described below. Note that, Y, -X-, $R^{5a}$, $R^{5b}$, n, $R^{4a}$, and $R^{4b}$ are as described in the above-described item (1).

As $R^1$, substituted or unsubstituted aromatic heterocyclyl is exemplified (hereinafter, referred to as A-1).

As $R^1$, substituted or unsubstituted 5- to 6-membered aromatic heterocyclyl is exemplified (hereinafter, referred to as A-2).

As $R^1$, aromatic heterocyclyl which is substituted with halogen, substituted alkyl (substituent: hydroxy) or unsubstituted alkyl, or an unsubstituted aromatic heterocyclyl is exemplified (hereinafter, referred to as A-3).

As $R^1$, 5- to 6-membered aromatic heterocyclyl which is substituted with halogen, substituted alkyl (substituent: hydroxy) or unsubstituted alkyl, or unsubstituted 5- to 6-membered aromatic heterocyclyl is exemplified (hereinafter, referred to as A-4).

As $R^1$, aromatic heterocyclyl which is substituted with unsubstituted alkyl or halogen, or unsubstituted aromatic heterocyclyl is exemplified (hereinafter, referred to as A-5).

As $R^1$, 5- to 6-membered aromatic heterocyclyl which is substituted with unsubstituted alkyl or halogen, or unsubstituted 5- to 6-membered aromatic heterocyclyl is exemplified (hereinafter, referred to as A-6).

As $R^1$, aromatic heterocyclyl which is substituted with unsubstituted alkyl or halogen is exemplified (hereinafter, referred to as A-7).

As $R^1$, 5- to 6-membered aromatic heterocyclyl which is substituted with unsubstituted alkyl or halogen is exemplified (hereinafter, referred to as A-8).

As $R^1$, aromatic heterocyclyl which is substituted with unsubstituted alkyl, or unsubstituted aromatic heterocyclyl is exemplified (hereinafter, referred to as A-9).

As $R^1$, 5- to 6-membered aromatic heterocyclyl which is substituted with unsubstituted alkyl, or unsubstituted 5- to 6-membered aromatic heterocyclyl is exemplified (hereinafter, referred to as A-10).

As $R^1$, aromatic heterocyclyl which is substituted with unsubstituted alkyl is exemplified (hereinafter, referred to as A-11).

As $R^1$, 5- to 6-membered aromatic heterocyclyl which is substituted with unsubstituted alkyl is exemplified (hereinafter, referred to as A-12).

As $R^2$, substituted or unsubstituted 6-membered aromatic carbocyclyl is exemplified (hereinafter, referred to as B-1).

As $R^2$, 6-membered aromatic carbocyclyl which is substituted with halogen, cyano, substituted alkyl (substituent: halogen) or unsubstituted alkyl is exemplified (hereinafter, referred to as B-2).

As $R^2$, 6-membered aromatic carbocyclyl which is substituted with halogen, cyano or unsubstituted alkyl is exemplified (hereinafter, referred to as B-3).

As $R^2$, 6-membered aromatic carbocyclyl which is substituted with two to four substituents selected from a substituent group G (substituent group G: halogen, cyano, and unsubstituted alkyl) is exemplified (hereinafter, referred to as B-4).

As $R^2$, 6-membered aromatic carbocyclyl which is substituted with two or three substituents selected from a substituent group G (substituent group G: halogen, cyano, and unsubstituted alkyl) is exemplified (hereinafter, referred to as B-5).

As $R^2$, 6-membered aromatic carbocyclyl which is substituted with three or four substituents selected from a substituent group G (substituent group G: halogen, cyano, and unsubstituted alkyl) is exemplified (hereinafter, referred to as B-6).

As $R^2$, 6-membered aromatic carbocyclyl which is substituted with three halogens is exemplified (hereinafter, referred to as B-7).

As $R^3$, substituted or unsubstituted aromatic heterocyclyl is exemplified (hereinafter, referred to as C-1).

As $R^3$, substituted or unsubstituted 9- to 10-membered aromatic heterocyclyl is exemplified (hereinafter, referred to as C-2).

As $R^3$, aromatic heterocyclyl which is substituted with halogen or substituted or unsubstituted alkyl is exemplified (hereinafter, referred to as C-3).

As $R^3$, 9- to 10-membered aromatic heterocyclyl which is substituted with halogen or substituted or unsubstituted alkyl is exemplified (hereinafter, referred to as C-4).

As $R^3$, aromatic heterocyclyl which is substituted with halogen or unsubstituted alkyl is exemplified (hereinafter, referred to as C-5).

As $R^3$, 9- to 10-membered aromatic heterocyclyl which is substituted with halogen or unsubstituted alkyl is exemplified (hereinafter, referred to as C-6).

As $R^3$, indazolyl which is substituted with halogen or unsubstituted alkyl is exemplified (hereinafter, referred to as C-7).

As $R^3$, indazolyl which is substituted with halogen and unsubstituted alkyl is exemplified (hereinafter, referred to as C-8).

m includes 0 or 1 (hereinafter, referred to as D-1).

m includes 0 (hereinafter, referred to as D-2).

m includes 1 (hereinafter, referred to as D-3).

Examples of the compound represented by Formula (I) include embodiments described below.

(a-1)

$R^1$ is (A-12);
$R^2$ is (B-7);
$R^3$ is (C-8); and
m is (D-2).

(a-2)

$R^1$ is (A-12);
$R^2$ is (B-7);
$R^3$ is (C-8); and
m is (D-3).

(a-3)

$R^1$ is (A-12);
$R^2$ is (B-7);
$R^3$ is (C-8); and

m is (D-1).

(a-4)

$R^1$ is (A-4);
$R^2$ is (B-4);
$R^3$ is (C-4); and
m is (D-1).

The compound represented by Formula (I) is preferably a compound represented by Formula (I-A) or a compound represented by Formula (I-B).

The chemical structural formulas represented by Formula (I), Formula (I-A), and Formula (I-B) are also commonly used in the respective formulas.

The compound represented by Formula (I) is not limited to particular isomers, but includes any possible isomers (for example, keto-enol isomer, imine-enamine isomer, diastereoisomer, optical isomer, rotamer, etc.), racemates, and a mixture thereof. For example, the compound represented by Formula (I) includes a tautomer as shown below.

[Chemical Formula 21]

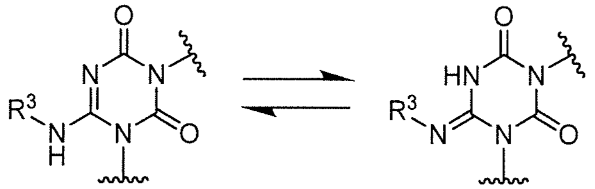

For example, the compound represented by Formula (I-A) and Compound (I-003) include tautomers as shown below and a mixture thereof.

[Chemical Formula 22]

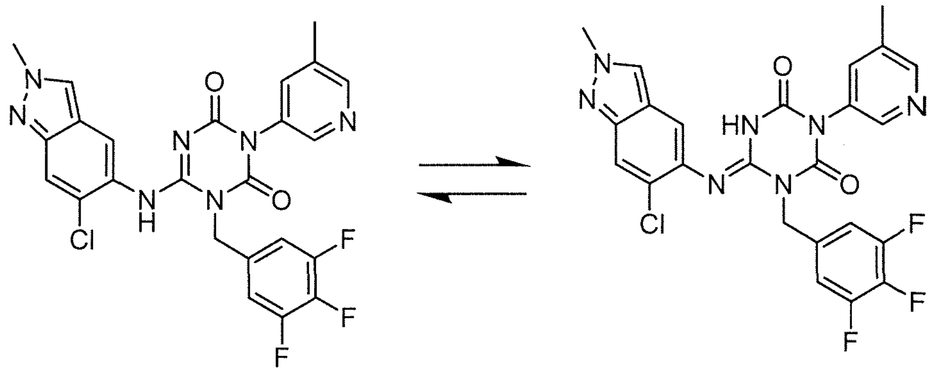

For example, the compound represented by Formula (I-B) and Compound (I-005) include tautomers as shown below and a mixture thereof.

[Chemical Formula 23]

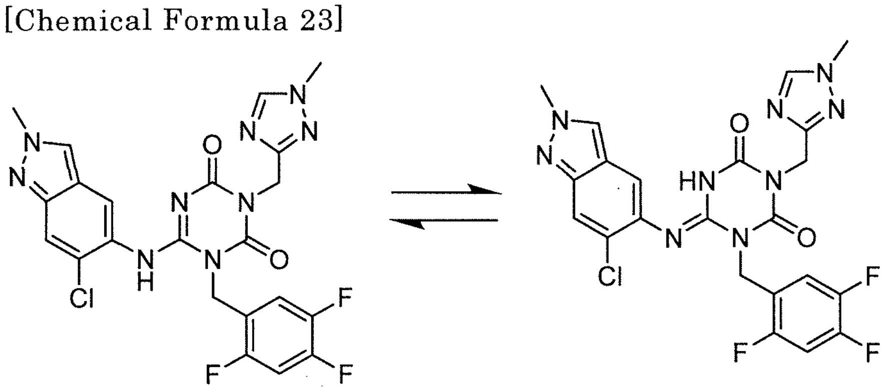

One or more hydrogen atom, carbon atom and/or another atom of the compound represented by Formula (I) may be replaced with an isotope of the hydrogen atom, carbon atom and/or another atom. Examples of such an isotope include hydrogen, carbon, nitrogen, oxygen, phosphorus, sulfur, fluorine, iodine, and chlorine such as $^{2}H$, $^{3}H$, $^{11}C$, $^{13}C$, $^{14}C$, $^{15}N$, $^{18}O$, $^{17}O$, $^{31}P$, $^{32}P$, $^{35}S$, $^{18}F$, $^{123}I$, and $^{36}Cl$. The compound represented by Formula (I) also includes compounds replaced with such an isotope. The compounds replaced with an isotope are also useful as a pharmaceutical product and include all of radiolabeled forms of the compound represented by Formula (I). Furthermore, a "method of radioactive labeling" for the production of the "radiolabeled form" is also included in the present invention, and this "radiolabeled form" is useful as a tool for research for metabolic pharmacokinetics, research and/or diagnosis in binding assay.

Furthermore, the crystal of the present invention may be a deuterated form. The crystal of the present invention may be labeled with an isotopic element (for example, $^{3}H$, $^{14}C$, $^{35}S$, $^{125}I$, etc.).

The radiolabeled form of the compound represented by Formula (I) can be prepared by the method well known in this technical field. For example, a tritium-labeled compound represented by Formula (I) can be prepared by introducing tritium into a specific compound represented by Formula (I) by catalytic dehalogenation reaction using tritium. This method includes reaction of a precursor which is a compound represented by Formula (I) appropriately halogenated with tritium gas in the presence of an appropriate catalyst, for example, Pd/C, and in the presence or absence of a base. For another appropriate method for preparing a tritium-labeled compound, "Isotopes in the Physical and Biomedical Sciences, Vol. 1, Labeled Compounds (Part A), Chapter 6 (1987)" can be referred to. $^{14}C$-labeled compound can be prepared using a raw material having $^{14}C$ carbon.

Examples of the pharmaceutically acceptable salt of the compound represented by Formula (I) include salts of the compound represented by Formula (I) with alkali metal (for example, lithium, sodium, potassium, etc.), alkaline earth metal (for example, calcium, barium, etc.), magnesium, transition metal (for example, zinc, iron, etc.), ammonia, organic base (for example, trimethylamine, triethylamine, dicyclohexylamine, ethanolamine, diethanolamine, triethanolamine, meglumine, ethylenediamine, pyrydine, picoline, quinoline, etc.) and amino acid or salts of the compound represented by Formula (I) with inorganic acid (for example, hydrochloric acid, sulfuric acid, nitric acid, carbonic acid, hydrobromic acid, phosphoric acid, hydroiodic acid, etc.), and organic acid (for example, formic acid, acetic acid, propionic acid, trifluoroacetic acid, citric acid, lactic acid, tartaric acid, oxalic acid, maleic acid, fumaric acid, succinic acid, mandelic acid, glutaric acid, malic acid, benzoic acid, phthalic acid, ascorbic acid, benzenesulfonic acid, p-toluenesulfonic acid, methanesulfonic acid, ethanesulfonic acid, trifluoroacetic acid, etc.). These salts can be formed by the method which is usually performed.

The pharmaceutically acceptable salt of the compound represented by Formula (I-A) may be composed of, for example, the compound represented by Formula (I-A) and a counter molecule or a counter ion and may include any number of counter molecules or counter ions. The pharmaceutically acceptable salt of the compound represented by Formula (I-A) indicates one which mediates ion bonding by proton movement between the compound and a counter molecule or a counter atm.

The pharmaceutically acceptable salt of the compound represented by Formula (I-A) is preferably a p-toluenesulfonate of the compound represented by Formula (I-A).

In the formulation of the present invention, a complex of the compound represented by Formula (I) or a pharmaceutically acceptable salt thereof can be used. The compound represented by Formula (I) or a pharmaceutically acceptable salt thereof may form a solvate (for example, hydrate, etc.), a cocrystal and/or a clathrate, and these are described as "complex" herein.

In the "solvate" used herein, any number of solvent molecules (for example, water molecule, etc.) may be coordinated, for example, to the compound represented by Formula (I). By leaving the compound represented by Formula (I) or a pharmaceutically acceptable salt thereof in the atmosphere, it may absorb moisture to adhere with absorbed water or form a hydrate thereof.

Examples of the solvent molecule include acetonitrile, chlorobenzene, chloroform, cyclohexane, 1,2-dichloroethene, dichloromethane, 1,2-dimethoxyethane, N,N-dimethylacetamide, N,N-dimethylformamide, 1,4-dioxane, 2-ethoxyethanol, ethylene glycol, formamide, hexane, methanol, 2-methoxyethanol, methylbutyl ketone, methylcyclohexane, N-methylpyrrolidone, nitromethane, pyridine, sulfolane, tetralin, toluene, 1,1,2-trichloroethene, xylene, acetic acid, anisole, 1-butanol, 2-butanol, n-butyl acetate, t-butyl methyl ether, cumene, dimethylsulfoxide, ethyl acetate, diethyl ether, ethyl formate, formic acid, heptane, isobutyl acetate, isopropyl acetate, methyl acetate, 3-methyl-1-butanol, methyl ethyl ketone, methyl isobutyl ketone, 2-methyl-1-propanol, pentane, 1-pentanol, 1-propanol, 2-propanol, propyl acetate, tetrahydrofuran, water (that is, hydrate), ethanol, acetone, 1,1-diethoxypropane, 1,1-dimethoxymethane, 2,2-dimethoxypropane, iso-octane, isopropyl ether, methyl isopropyl ketone, methyltetrahydrofuran, petroleum ether, trichloroacetic acid, and trifluoroacetic acid, preferably, acetic acid, anisole, 1-butanol, 2-butanol, n-butyl acetate, t-butyl methyl ether, cumene, dimethylsulfoxide, ethyl acetate, diethyl ether, ethyl formate, formic acid, heptane, isobutyl acetate, isopropyl acetate, methyl acetate, 3-methyl-1-butanol, methyl ethyl ketone, methyl isobutyl ketone, 2-methyl-1-propanol, pentane, 1-pentanol, 1-propanol, 2-propanol, propyl acetate, tetrahydrofuran, water (that is, hydrate), ethanol, acetone, 1,1-diethoxypropane, 1,1-dimethoxymethane, 2,2-dimethoxypropane, iso-octane, isopropyl ether, methyl isopropyl ketone, methyltetrahydrofuran, petroleum ether, trichloroacetic acid, and trifluoroacetic acid, and more preferably, water (that is, hydrate), ethanol, acetone, 1,1-diethoxypropane, 1,1-dimethoxymethane, 2,2-dimethoxypropane, iso-octane, isopropyl ether, methyl isopropyl ketone, methyltetrahydrofuran, petroleum ether, trichloroacetic acid, and trifluoroacetic acid.
The "cocrystal" used herein means that counter molecules are regularly arranged in the same crystal lattice and may include any number of counter molecules. Further, the cocrystal indicates one in which the intermolecular interaction between the compound and the counter molecule is mediated with noncovalent and non-ionic chemical interaction such as hydrogen bonding or van der Waals' force.
For example, the cocrystal of the compound represented by Formula (I-B) may be composed of the compound represented by Formula (I-B) and a counter molecule and may include any number of counter molecules. Preferably, the cocrystal may be composed of the compound represented by Formula (I-B) and fumaric acid and may include any number of fumaric acids. Further preferably, the cocrystal is a cocrystal composed of the compound represented by Formula (I-B) and fumaric acid at a molar ratio of 1 : 1.
The cocrystal is distinguished from a salt in that the compound is essentially uncharged or neutral.
The cocrystal is distinguished from a hydrate or a solvate in that the counter molecule is not water or a solvent.
The "crystal" used herein means a solid in which constituent atoms, ions, molecules, etc. are three-dimensionally arranged with regularity, and is distinguished from a non-crystalline solid not having such a regular inner structure. The crystal of the present invention may be a single crystal, a twin crystal, a polycrystal, and the like.
Further, in the "crystal", there may be a "crystalline polymorphism" which has the same composition but has different arrangement in the crystal, and crystals including these are referred to as the "crystalline form".
The crystalline form and the crystallinity can be measured by many techniques including, for example, X-ray powder diffraction measurement, Raman spectroscopy, an infrared absorption spectrum measurement method, moisture adsorptiondesorption measurement, differential scanning calorimetry, and dissolution properties.
Furthermore, a "crystalline polymorphism" may be formed by recrystallization of the compound represented by Formula (I), a pharmaceutically acceptable salt thereof, or the complex thereof.
In the formulation of the present invention, such various salts, complexes (hydrate, solvate, cocrystal, and clathrate), and the crystalline polymorphism can be used, and a mixture of two or more kinds thereof can also be used.

(X-ray powder diffraction (XRPD))

The X-ray powder diffraction (XRPD) is one of the most sensitive analytical methods for measuring the crystalline form and crystallinity of solid. When crystals are irradiated with X-rays, the X-rays are reflected by the crystal lattice planes and mutually interfere, and the ordered diffraction lines corresponding to the periodicity of the structure are observed. On the other hand, in the case of amorphous solids, usually, since they do not have the ordered iteration periodicity in the structure, diffraction phenomenon does not occur, and featureless broad XRPD patterns (also called halo patterns) are shown.
The crystalline form of the compounds represented by Formula (I-A) and Formula (I-B) can be identified by the X-ray powder diffraction pattern and characteristic diffraction peaks. The crystalline form of the compounds represented by Formula (I-A) and Formula (I-B) can be distinguished from the other crystalline form by the presence of characteristic diffraction peaks.
The characteristic diffraction peaks used herein are peaks selected from the observed diffraction pattern. The characteristic diffraction peaks are selected from preferably about ten, more preferably about five, and further preferably about three in the diffraction pattern.
In order to distinguish between multiple crystals, a peak which is shown for the crystal and not shown for the other crystal becomes a more preferable characteristic peak than the intensity of a peak when the crystal is specified.

The crystal can be characterized by one or two peak(s) if it is such characteristic peak(s). By comparing the chart obtained by measuring, if these characteristic peaks coincide, the X-ray powder diffraction pattern can be said to substantially match up.

Since an error in the range of ±0.2° may occur in diffraction angles (2θ) in X-ray powder diffraction, in general, the value of the diffraction angle of X-ray powder diffraction should be understood as the one including values in a range of around ±0.2°. Therefore, the present invention includes not only crystalline forms whose diffraction angles of the peaks in X ray powder diffraction perfectly match, but also crystalline forms whose diffraction angles of the peaks match within an error of around ±0.2°.

In general, it is known that the intensities of the peaks shown in the following tables and drawings may vary depending on a number of factors, for example, selected orientation effects of crystals in the X-ray beam, effect of coarse particle, purity of the material to be analyzed, or crystallinity of the sample. Furthermore, the peak positions may also shift for variations in sample height. Further, measurements using a different wavelength will result in different shifts according to the Bragg equation (nλ = 2dsin0). Such another XRPD patterns obtained by using a different wavelength are also within the scope of the present invention.

(Single crystal structural analysis)

By one of methods of identifying a crystal, crystallographic parameters in the crystal, atomic coordinates (values indicating spatial positional relationship of individual atoms), and the three-dimensional structural model can be obtained. Refer to "Manual of X-ray structural analysis" written by Sakurai Toshio, published by Shokabo Co., Ltd. (1983), X-Ray Structure Determination: A Practical Guide, written by Stout & Jensen, Macmillan Co., New York (1968), and the like. The single crystal structural analysis is useful to identify the crystalline structures of the complex, salt, optical isomer, tautomer, and geometric isomer as described in the present invention.

The compound according to the present invention has coronavirus 3CL protease inhibitory activity, and thus is useful as a therapeutic and/or prophylactic agent for diseases that involve the coronavirus 3CL protease. A "therapeutic agent and/or prophylactic agent" in the present invention also includes a symptom-improving agent. As the diseases that involve the coronavirus 3CL protease, virus diseases are exemplified, and preferably, coronavirus diseases are exemplified.

As an embodiment, examples of the coronavirus include coronaviruses that infect humans. Examples of the coronaviruses that infect humans include HCoV-229E, HCoV-NL63, HCoV-HKU1, HCoV-OC43, SARS-CoV, MERS-CoV, and/or SARS-CoV-2.

As an embodiment, as the coronavirus, Alphacoronavirus and/or Betacoronavirus, more preferably Betacoronavirus, and further preferably Sarbecovirus are exemplified.

As an embodiment, examples of the Alphacoronavirus include HCoV-229E and HCoV-NL63. Particularly preferably, HCoV-229E is exemplified.

As an embodiment, examples of the Betacoronavirus include HCoV-HKU1, HCoV-OC43, SARS-CoV, MERS-CoV, and/or SARS-CoV-2. Preferably, HCoV-OC43 or SARS-CoV-2 is exemplified, and particularly preferably, SARS-CoV-2 is exemplified.

As an embodiment, examples of the Betacoronavirus include β-coronavirus lineage A, β-coronavirus lineage B, and β-coronavirus lineage C. More preferably, β-coronavirus lineage A and β-coronavirus lineage B are exemplified, and particularly preferably, β-coronavirus lineage B is exemplified.

As the β-coronavirus lineage A, for example, HCoV-HKU1 and HCoV-OC43 are exemplified, and preferably, HCoV-OC43 is exemplified. As the β-coronavirus lineage B, for example, SARS-CoV and SARS-CoV-2 are exemplified, and preferably, SARS-CoV-2 is exemplified. As the β-coronavirus lineage C, preferably, MERS-CoV is exemplified.

As an embodiment, as the coronavirus, HCoV-229E, HCoV-OC43, and/or SARS-CoV-2 are exemplified, and particularly preferably, SARS-CoV-2 is exemplified.

It is generally known that viruses are mutated while repeating proliferation and infection. The coronavirus includes not only mutant strains known in the art but also mutant strains that will appear in the future as long as the compound according to the present invention is a strain capable of exhibiting a coronavirus 3CL protease inhibitory activity. Example of known mutant strains of SARS-CoV-2 include the mutant strains used in Examples herein.

Examples of the coronavirus disease include infective diseases due to HCoV-229E, HCoV-NL63, HCoV-OC43, HCoV-HKU1, SARS-CoV, MERS-CoV, and/or SARS-CoV-2. Preferably, infective diseases due to HCoV-229E, HCoV-OC43, and/or SARS-CoV-2 are exemplified, and particularly preferably, infective diseases due to SARS-CoV-2 are exemplified.

As the coronavirus disease, particularly preferably, coronavirus disease 2019 (COVID-19) is exemplified.

The classification of the degree of severity of coronavirus disease 2019 infected persons is exemplified below, for example. (Reference: Coronavirus disease 2019 (COVID-19) Guidance of medical examination 5.2 Edition (Ministry of Health, Labour and Welfare))

(Mild severity)

The oxygen saturation is 96% or more. In the clinical state, there is no respiratory symptom or there is only coughing without difficulty of breathing, and in any cases, a finding of pneumonia is not recognized.

(Moderate severity I)

The oxygen saturation is less than 96% to more than 93%. There is difficulty of breathing, and a finding of pneumonia is recognized.

(Moderate severity II)

The oxygen saturation is less than 93%. There is respiratory failure, and oxygen administration is required.

(Severe severity)

A patient is in ICU or requires an inhalator.

The above classification is based on definition of the degree of severity in Japan, and for example, the classification of the degree of severity in China or U.S. NIH can be employed.

Furthermore, an asymptomatic SARS-CoV-2-infected person means a person having an asymptomatic pathogen. For example, persons who do not have fourteen symptoms of COVID-19 symptoms recognized are exemplified.

(Fourteen symptoms of COVID-19: a feeling of fatigue, pains in muscles or body, headache, chillness, fever, cacogeusia, dysosmia, runny nose or stuffy nose, sore throat, coughing, shortness of breath, nausea, emesis, and diarrhea)

Herein, twelve symptoms of COVID-19 include a feeling of fatigue, pains in muscles or body, headache, chillness, fever, runny nose or stuffy nose, sore throat, coughing, shortness of breath, nausea, emesis, and diarrhea.

Herein, the exacerbation suppression means that the symptom of an asymptomatic SARS-CoV-2-infected person is suppressed from being raised to the degree of severity classified into mild severity, moderate severity I, moderate severity II, or severe severity.

Herein, the exacerbation suppression means that the symptom of an asymptomatic SARS-CoV-2-infected person or a mild-severity SARS-CoV-2-infected person is suppressed from being raised to the degree of severity classified into moderate severity I, moderate severity II, or severe severity.

Herein, the exacerbation suppression means that the symptom of an asymptomatic SARS-CoV-2-infected person, a mild-severity SARS-CoV-2-infected person, or a moderate-severity I SARS-CoV-2-infected person is suppressed from being raised to the degree of severity classified into moderate severity II or severe severity.

Herein, the exacerbation suppression means that the symptom of an asymptomatic SARS-CoV-2-infected person, a mild-severity SARS-CoV-2-infected person, a moderate-severity I SARS-CoV-2-infected person, or a moderate-severity II SARS-CoV-2-infected person is suppressed from being raised to the degree of severity classified into severe severity.

Herein, the exacerbation suppression means that the hospitalization or death risk of a SARS-CoV-2-infected person is decreased through the virus proliferation suppression effect of the present agent.

Herein, the exacerbation suppression means that the inflammation in the lung of a SARS-CoV-2-infected person is alleviated through the virus proliferation suppression effect of the present agent.

Herein, the exacerbation suppression means that pneumonia caused by virus infection of SARS-CoV-2 is suppressed through the virus proliferation suppression effect of the present agent.

Herein, the exacerbation suppression means that overactive immune response of a host caused by virus infection of SARS-CoV-2 is suppressed through the virus proliferation suppression effect of the present agent.

As an embodiment, the pharmaceutical composition of the present invention is administered to an infected person having at least one of exacerbation risk factors shown below among SARS-CoV-2-infected persons.

· 50 years old or older
· Obesity (BMI 30 kg/$m^2$ or more)
· Cardiovascular disease (including cardiovascular disease including hypertension and congenital cardiac disease)
· Chronic pulmonary disease (including asthma and interstitial pulmonary disease)
· Type 1 or type 2 diabetes
· Chronic renal impairment (including dialyzed patients)
· Chronic hepatic disease
· Immunosuppressed state (e.g.: malignancy treatment, bone marrow or organ transplantation, immune deficiency,

uncontrolled HIV, AIDS, sickle-cell anemia, thalassemia, and long-term administration of an immunosuppressant)

· Chronic obstructive pulmonary disease (COPD)
· Hyperlipidemia
· Smoking
· Immune deficiency after solid organ transplantation
· Pregnancy
· Patient having a neurodevelopmental disease or complicated clinical conditions (e.g.: cerebral palsy and congenital disease)
· Patient having a high degree of medical dependence (e.g.: tracheostomy, gastric fistula, and positive pressure ventilation)

As an embodiment, the pharmaceutical composition of the present invention is administered to an infected person having at least one of exacerbation risk factors and used for suppressing the exacerbation of COVID-19 symptoms.
As an embodiment, the pharmaceutical composition of the present invention is used for a patient having pneumonia caused by SARS-CoV-2.
As an embodiment, the pharmaceutical composition of the present invention is administered to an infected person having at least one of exacerbation risk factors shown below among SARS-CoV-2-infected persons.

· Patient with installation of extracorporeal membrane oxygenation (ECMO)
· Patient with installation of inhalator
· Patient in ICU
· Patient having an oxygen saturation ($SpO_2$) of less than 93% (room air) or requiring oxygen inhalation

As an embodiment, the pharmaceutical composition of the present invention is administered to an infected person having at least one of exacerbation risk factors shown below among SARS-CoV-2-infected persons.

· Oxygen saturation (SpO2) of less than 94% (room air, sea level)
· PaO2/FiO2 of less than 300 mmHg
· Respiration rate of 30 or more/min
· Pulmonary infiltration of 50% or more

(Process method of compound represented by Formula (I))

The compound represented by Formula (I) can be produced, for example, by a general synthesis method described below. Extraction, purification, and the like may be carried out by conventional methods practiced in organic chemistry experiments. The compound can be synthesized with reference to methods known in the art. Extraction, purification, and the like may be carried out by conventional methods practiced in organic chemistry experiments.
The compound represented by Formula (I) can be produced with reference to methods known in the art. The compound can be produced, for example, with reference to WO 2010092966, WO 2012020749, WO 2013089212, WO 2014200078, WO 2012020742, WO 2013118855, and the like.

(Method A)

[Chemical Formula 24]

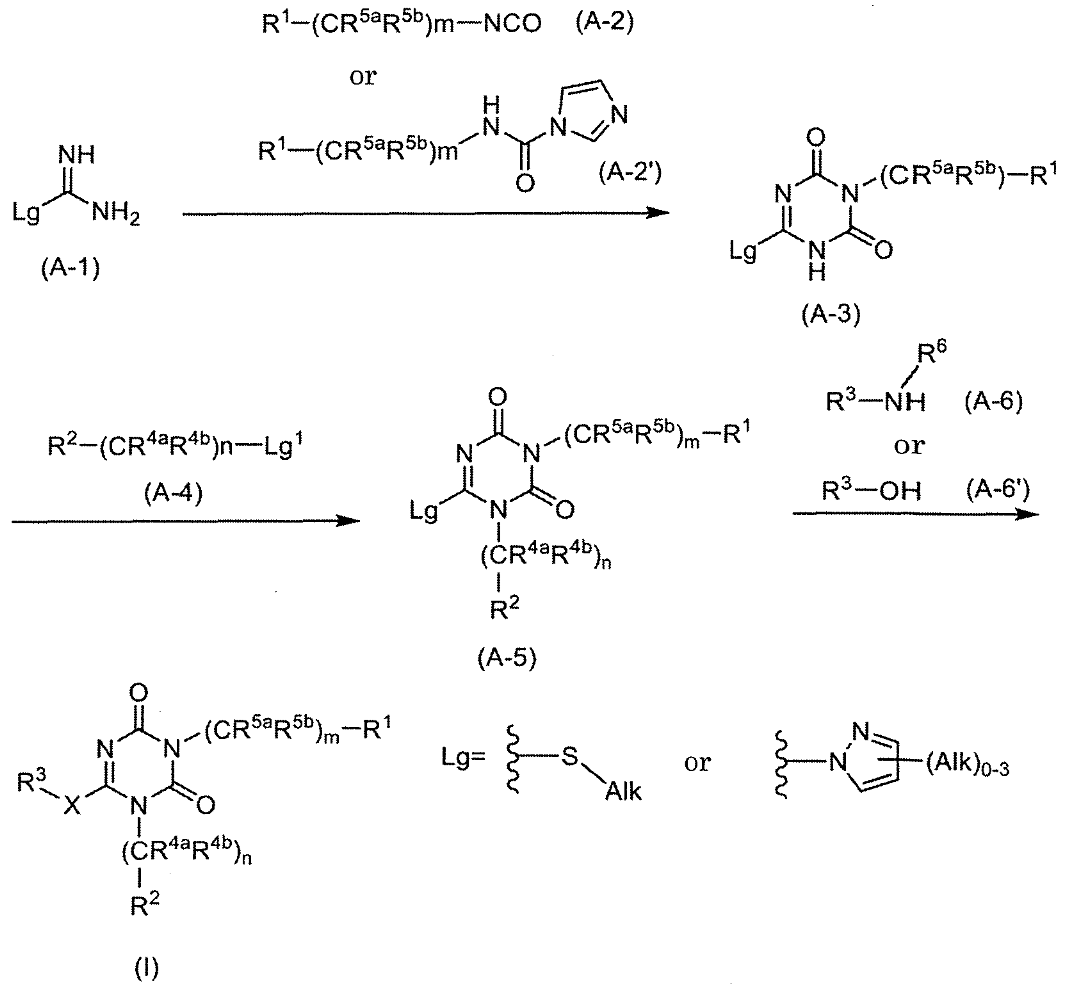

(A-2) (A-2') (A-1) (A-3) (A-6) (A-6') (A-4) (A-5) (I)

wherein Alk is C1-C3 alkyl, $Lg^1$ is a leaving group, $R^6$ is a hydrogen atom, and other symbols are as defined above.

(First step)

Compound (A-1) or its hydrochloride or bromate, etc. is reacted with isocyanate (A-2) or 1-carbamoylimidazole (A-2') in a solvent such as N,N-dimethylformamide, N,N-dimethylacetamide, N,N'-dimethylimidazolidinone, dimethylsulfoxide, or THF in the presence of a base such as DBU, triethylamine, N,N-diisopropylethylamine, or pyridine (preferably, DBU) at -20°C to 50°C, preferably -10°C to under ice-cooling. Subsequently, Compound (A-3) can be produced by reacting the reaction mixture with a carbonylating agent such as 1,1'-carbonyldiimidazole, phosgene, or triphosgene and a base such as DBU, triethylamine, N,N-diisopropylethylamine, or pyridine (preferably, DBU) at -20°C to 50°C, preferably -10°C to under ice-cooling.

(Second step)

Compound (A-5) can be produced by reacting Compound (A-3) with Compound (A-4) in a solvent such as acetonitrile, acetone, DMF, or DMSO in the presence of a base such as potassium carbonate, sodium carbonate, N,N-diisopropylethylamine, at 50°C to under refluxing with heating, preferably under refluxing with heating.

Examples of the leaving group include halogen and $-OSO_2(C_tF_{2t+1})$ (wherein t is an integer of 1 to 4). The halogen is preferably chlorine, iodine, and bromine, and the $OSO_2(C_tF_{2t+1})$ group is preferably a -OTf group (trifluoromethanesulfonic acid ester).

(Third step)

A compound represented by Compound (I) can be produced by reacting Compound (A-5) with Compound (A-6) or Compound (A-6') in a solvent such as NMP, DMF, DMA, DMSO, tert-butanol, or 2-methyl-2-butanol, in the presence or absence of an acid such as acetic acid at 60°C to 150°C, preferably 80°C to 120°C.
By using the optically active isocyanate (A-2), a compound represented by Compound (I) which is optically active can be produced.

(Method B)

[Chemical Formula 25]

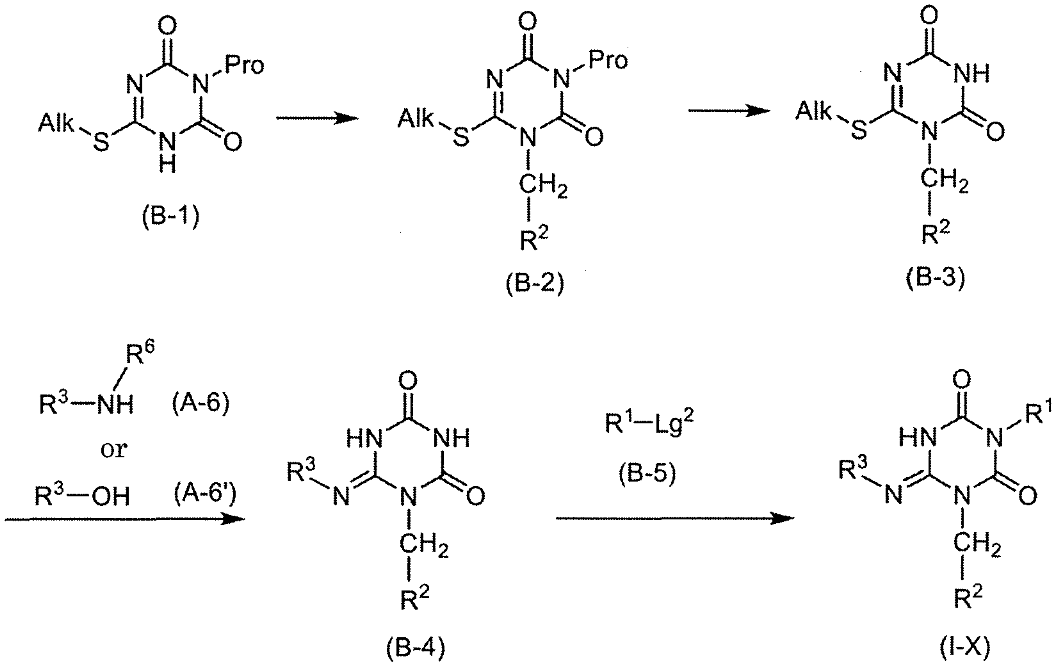


(B-1) (B-2) (B-3) (B-4) (I-X)

wherein Alk is C1-C3 alkyl, Pro is C1-C4 alkyl or tert-butoxycarbonyl, $Lg^2$ is a leaving group, $R^6$ is a hydrogen atom, and other symbols are as defined above.

(First step)

Compound (B-2) can be produced from Compound (B-1) in the same manner as in the second step of Method A described above.

(Second step)

Compound (B-3) can be produced by treating Compound (B-2) at -20°C to room temperature, preferably at room temperature, with a strong acid such as TFA in the presence or absence of an organic solvent.

(Third step)

Compound (B-4) can be produced from Compound (B-3) in the same manner as in the third step of Method A described above.

(Fourth step)

Compound (I-X) can be produced by Goldberg amination reaction using Compound (B-4) and Compound (B-5).
As a leaving group, the leaving group described in Step 1 of Method A is exemplified.

As a catalyst, for example, commercially available copper catalysts such as copper iodide, copper cyanide, and copper bromide can be used.

As a ligand, 1,2-dimethylethylenediamine, trans-N,N'-dimethylcyclohexane-1,2-diamine, and the like can be used.

As a base, potassium carbonate, potassium phosphate, and the like can be used.

As a solvent, NMP, dioxane, DMSO, and the like can be used.

Regarding the reaction temperature, the reaction may be performed in the range of room temperature to the reflux temperature of the solvent, and preferably may be performed under refluxing with heating.

(Method C)

[Chemical Formula 26]

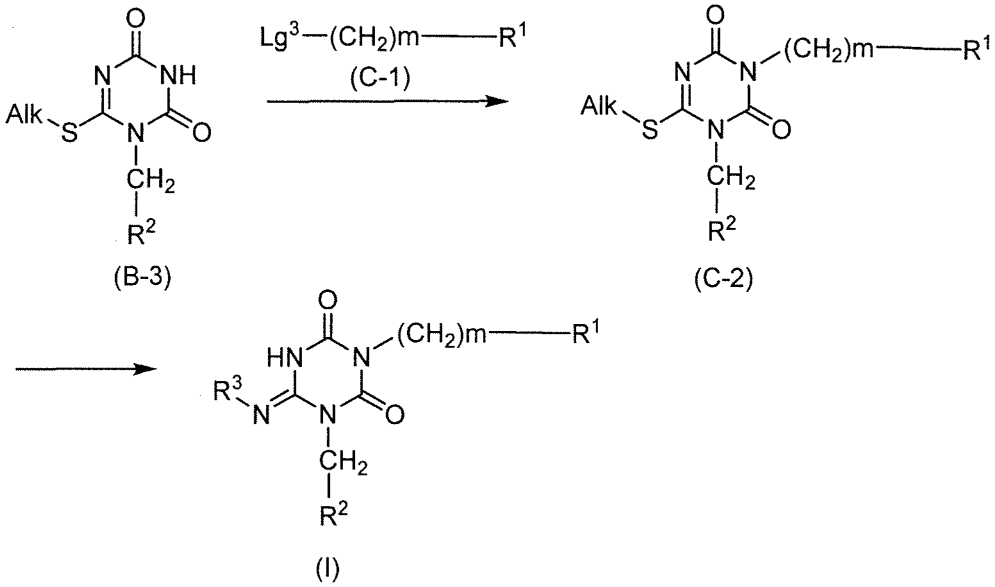


(B-3) (C-2) (I)

wherein Alk is C1-C3 alkyl, $Lg^3$ is a leaving group, and other symbols are as defined above.

(First step)

Compound (C-2) can be produced in the same manner as in the second step of Method A described above.

As a leaving group, the leaving group described in Step 1 of Method A is exemplified.

(Second step)

The compound represented by Compound (I) can be produced in the same manner as in the third step of Method A described above.

The compound according to the present invention has coronavirus 3CL protease inhibitory activity, and thus is useful as a therapeutic and/or prophylactic agent for virus diseases.

Further, the compound according to the present invention has usefulness as a medicament and has preferably any or a plurality of the following superior properties.

a) The compound has weak inhibitory activity against CYP enzymes (for example, CYP1A2, CYP2C9, CYP2C19, CYP2D6, CYP3A4, etc.).
b) The compound shows excellent pharmacokinetics such as high bioavailability or moderate clearance.
c) The compound has high metabolic stability.
d) The compound does not show irreversible inhibitory activity against CYP enzymes (for example, CYP3A4) in the range of the concentration of the measurement conditions described herein.
e) The compound does not show mutagenesis.
f) The compound has a low risk of cardiovascular systems.

g) The compound shows high solubility.
h) The compound shows a high protein unbinding ratio (fu value).
i) The compound has high coronavirus 3CL protease selectivity.
j) The compound has high coronavirus replication inhibitory activity. For example, the compound has high coronavirus replication inhibitory activity with addition of human serum (HS) or human serum albumin (HSA).

Examples of a coronavirus replication inhibitor include embodiments, for example, having $EC_{50}$ of 10 μM or less, preferably 1 μM or less, and more preferably 100 nM or less, for example, in a CPE effect (SARS-CoV-2) described below.
Furthermore, the salt -crystal -complex (cocrystal) of the compound represented by Formula (I) has usefulness as a medicament and has preferably any or a plurality of the following superior properties.

A) It shows excellent pharmacokinetics such as high bioavailability, moderate clearance, high AUC, or high maximum drug concentration.
B) It shows high solubility, high chemical stability, and low moisture absorbency.

The pharmaceutical composition of the present invention can also be administered orally or parenterally. Examples of methods for parenteral administration include dermal, subcutaneous, intravenous, intraarterial, intramuscular, intraperitoneal, transmucosal, inhalation, transnasal, ophthalmic, inner ear or vaginal administration, and the like.
In the case of oral administration, any forms, which are usually used, such as oral solid formulations (for example, tablets, powders, granules, capsules, pills, films, etc.), oral liquid formulations (for example, suspension, emulsion, elixir, syrup, limonade, spirit, aromatic water, extract, decoction, tincture, etc.) and the like may be prepared according to the usual method and administered. The tablets may be sugar-coated tablets, film-coated tablets, enteric-coating tablets, sustained-release tablets, troche tablets, sublingual tablets, buccal tablets, chewable tablets, or orally disintegrated tablets, powders and granules may be dry syrups, and capsules may be soft capsules, micro capsules, or sustained-release capsules.
In the case of parenteral administration, any forms, which are usually used, such as injections, infusion, external formulation (for example, eye drops, nasal drops, ear drops, aerosol, inhalation, lotion, injection agents, coating agents, mouthwash, enemas, ointments, plasters, jellies, creams, patches, cataplasms, external powders, suppositories, etc.) and the like can be preferably administrated. The injections may be emulsions whose type is O/W, W/O, O/W/O, W/O/W, or the like.
The pharmaceutical composition can be manufactured by mixing an effective amount of the compound according to the present invention with various pharmaceutical additives suitable for the formulation, such as excipients, binders, disintegrants, and lubricants, as necessary. Further, the pharmaceutical composition can also be used for pediatric patients, geriatric patients, serious cases, or operations by appropriately changing the effective amount of the compound according to the present invention, formulation and/or various pharmaceutical additives. For example, pediatric pharmaceutical compositions may be administered to patients who are neonates (younger than 4 weeks old after the birth), infants (4 weeks old to younger than 1 year old after the birth), children (1 year old or older and younger than 7 years old), infant children (7 years old or older and younger than 15 years old), or 15 to 18 years old. For example, the geriatric pharmaceutical compositions may be administered to patients who are 65 years old or older.
Although it is desirable to set the dose of the pharmaceutical composition of the present invention (for example, the pharmaceutical composition containing p-toluenesulfonate crystal Form I of the compound represented by Formula (I-A), or the pharmaceutical composition containing fumaric acid cocrystal Form I of the compound represented by Formula (I-B)) in consideration of the age and body weight of the patient, disease type and degree or administration route, and the like, the dose in the case of orally administration is within the range of usually 0.05 to 200 mg/kg/day and preferably 0.1 to 100 mg/kg/day. Although varying depending on the administration route, the dose in the case of parenteral administration is within the range of usually 0.005 to 200 mg/kg/day and preferably 0.01 to 100 mg/kg/day. It may be administered once to several times a day.
The compound according to the present invention may be used, for example, combining other medicaments for treating coronavirus disease 2019 (COVID-19) (as the therapeutic agents, including approved medicaments, and medicaments which are under development or will be developed in the future) (hereinafter, referred to as a concomitant medicament) for the purpose of enforcement of the activity of the compound or reduction of the dose of the compound or the like. In this case, timing of administration of the compound according to the present invention and the concomitant medicament is not limited and these may be administered to the subject simultaneously or at regular intervals. Further, the compound according to the present invention and the concomitant medicament may be administered as two or more kinds of different compositions containing each active ingredient or as a single formulation containing both active ingredient.
The dose of the concomitant medicament can be appropriately selected on the basis of the dose used on

clinical. Furthermore, the mixing ratio of the compound according to the present invention and a concomitant medicament can be appropriately selected in consideration of the subject of administration, administration route, target diseases, symptoms, combinations, and the like. For example, when the subject of administration is human, the concomitant medicament may be used in the range of 0.01 to 100 parts by weight with respect to 1 part by weight of the compound according to the present invention.

[EXAMPLES]

The present invention will be described in more detail below by way of Examples and Reference Examples, as well as Test Examples; however, the present invention is not limited thereto.
Furthermore, the meaning of each abbreviation used herein is as follows.

Boc: tert-butoxycarbonyl
CDI: carbonyldiimidazole
DBU: 1,8-diazabicyclo[5.4.0]-7-undecene
DIEA: N,N-diisopropylethylamine
DMA: N,N-dimethylacetamide
DMF: N,N-dimethylformamide
DMSO: dimethyl sulfoxide
DTT: dithiothreitol
EDC: 1-ethyl-3-(3-dimethylaminopropyl)carbodiimide
EDT: 1,2-ethanedithiol
EDTA: ethylenediaminetetraacetic acid
FBS: fetal bovine serum
HOBT: 1-hydroxybenzotriazole
LHMDS: lithium bis(trimethylsilyl)amide
MEM: Eagle's minimum essential medium
NMP: N-methylpyrrolidone
$Pd(OAc)_2$: palladium acetate
TFA: trifluoroacetic acid
TMSCl: chlorotrimethylsilane
Xantphos: 4,5'-bis(diphenylphosphino)-9,9'-dimethylxanthene
mM: mmol/L
μM: μmol/L
nM: nmol/L

(Identification of compounds)

NMR analysis obtained in each Example was performed at 400 MHz, and measurement was made using DMSO-$d_6$, $CDCl_3$ and MeOH-$d_4$. Furthermore, in NMR data shown in Examples and Reference Examples, not all measured peaks may be described.
"RT" herein means retention time in LC/MS: liquid chromatography/mass spectrometry and measured under the following conditions.

(Measurement conditions 1)

Column: ACQUITY UPLC (registered trademark) BEH C18 (1.7 μm i.d.2.1 × 50 mm) (Waters)
Flow rate: 0.8 mL/min
UV detection wavelength: 254 nm
Mobile phases: [A] is a 0.1% formic acid-containing aqueous solution, and [B] is a 0.1% formic acid-containing acetonitrile solution
Gradient: Linear gradient of 5% to 100% solvent [B] for 3.5 minutes was performed, and then 100% solvent [B] was maintained for 0.5 minutes.

(Measurement conditions 2)

Column: Shim-pack XR-ODS (2.2 μm, i.d.3.0 × 50 mm) (Shimadzu)
Flow rate: 1.6 mL/min
UV detection wavelength: 254 nm
Mobile phases: [A] is a 0.1% formic acid-containing aqueous solution, and [B] is a 0.1% formic acid-containing acetonitrile solution
Gradient: Linear gradient of 10% to 100% solvent [B] for 3 minutes was performed, and 100% solvent [B] was maintained for 0.5 minutes.

Note that, herein, the description MS (m/z) indicates the value observed in the mass spectrometry.

(X-ray powder diffraction pattern measurement)

X-ray powder diffraction pattern measurement of crystals obtained in each Example was performed according to a powder X-ray diffraction measurement method described in General Tests in Japanese Pharmacopoeia. Measurement conditions are as follows.

(Device)

SmartLab manufactured by Rigaku Corporation

(Operation method)

Measuring method: reflection method
Wavelength used: CuKα ray
Tube current: 200 mA
Tube voltage: 45 kV
Sampling plate: aluminum
X-ray incident angle: 2.5°
Sampling width: 0.02°
Detector: HyPix-3000 (two-dimensional detection mode)

(Measurement of single crystal structural analysis and analysis method)

The measurement conditions of the single crystal structural analysis and the analysis method are as follows.

(Device)

XtaLAB P200 MM007 manufactured by Rigaku Corporation

(Measurement conditions)

Measurement temperature: 25°C
Wavelength used: CuKα ray (λ = 1.5418 Å)
Software: CrysAlisPro 1.171.39.46e (Rigaku Oxford Diffraction, 2018)

(Data processing)

Software: CrysAlisPro 1.171.39.46e (Rigaku Oxford Diffraction, 2018)
The data were corrected for the Lorentz, polarization and absorption effects.

(Crystal structural analysis)

The phase determination was performed by using the direct method program ShelXT (Sheldrick, G.M., 2015), and the structural refinement by full-matrix leastsquare method was then performed by using ShelXL (Sheldrick, G.M., 2015). All temperature factors of non-hydrogen atoms were refined with anisotropic parameters. Hydrogen atoms were placed by calculation using default parameters of ShelXL and regarded as riding atom. All hydrogen atoms were refined with isotropic parameters.

Fig. 2 and Fig. 4 were made using PLATON (Spek, 1991)/ORTEP (Johnson, 1976).

[Example 1]

Synthesis of Compound (I-003)

[Chemical Formula 27]

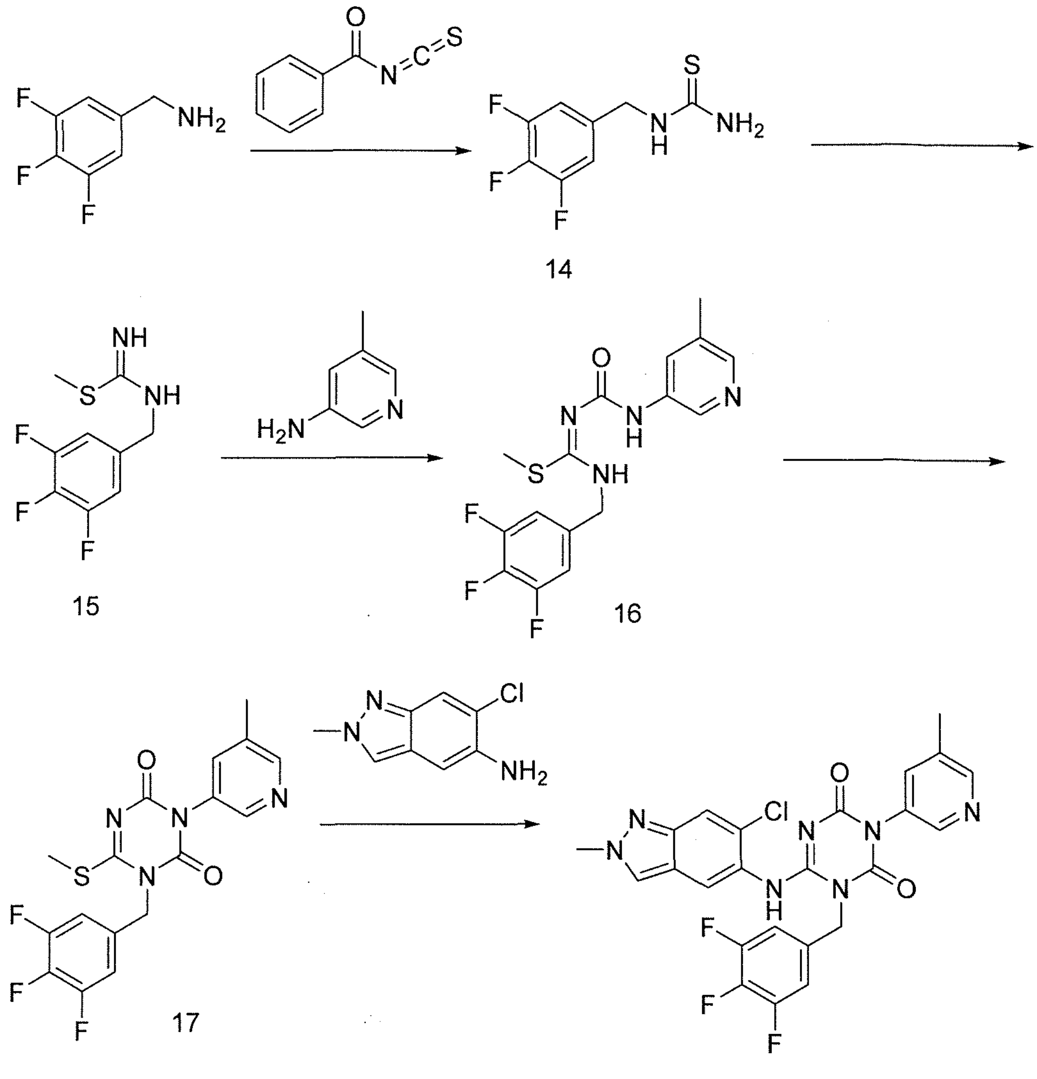

14

15

16

17

I-003

Step 1 Synthesis of Compound 14

3,4,5-trifluorobenzylamine (3.34 g, 20.7 mmol) was dissolved in dichloromethane (33.4 mL) and cooled in a water bath. Benzoyl isothiocyanate (2.93 mL, 21.8 mmol) was added to the reaction solution and stirred at room temperature for 30 minutes.

The solvent was distilled, the residue was diluted with methanol, and a 1 mol/L aqueous solution of sodium oxide (7.45 mL, 7.45 mmol) was added. The reaction solution was stirred at room temperature for 30 minutes and a 2 mol/L aqueous solution of hydrochloric acid was added. The aqueous layer was extracted with ethyl acetate, and the organic layer was washed with a saturated sodium hydrogen carbonate aqueous solution and brine. The organic layer was dried with sodium sulfate, and the solvent was distilled under reduced pressure, thereby obtaining a crude product (8.3 g) of Compound 14. This crude product was used for the next step without further purification, assuming that the yield was 100%.

LC/MS (ESI): m/z = 221, RT = 1.45 min, LC/MS measurement conditions 2

Step 2 Synthesis of Compound 15

The crude product (8.3 g) of Compound 14, DMF (85 mL), and methyl iodide (4.84 mL, 77 mmol) were mixed, and the reaction solution was stirred at 50°C for 40 minutes. Water was added to the reaction solution, extracted with ethyl acetate, and washed with water. A 2 mol/L aqueous solution of sodium hydroxide was added to the aqueous layer, and the aqueous layer was extracted with ethyl acetate. The organic layer was washed with water and brine and dried with sodium sulfate. The solvent was distilled under reduced pressure, thereby obtaining a crude product (3.86 g, 16.5 mmol, yield 80%) of Compound 15.

LC/MS (ESI): m/z = 235, RT = 0.84 min, LC/MS measurement conditions 2

Step 3 Synthesis of Compound 16

Triphosgene (0.507 g, 1.71 mmol) and THF (6 mL) were mixed, and the reaction solution was cooled in an ice bath. 3-Amino-5-methylpyridine (0.462 g, 4.27 mmol) and triethylamine (1.48 mL, 10.7 mmol) were mixed in THF (6 mL), and the solution thus obtained was added dropwise to the reaction solution. The reaction solution was stirred at room temperature for 40 minutes and then cooled in an ice bath. Compound 15 (1 g, 4.27 mmol) was added to the reaction solution and stirred at room temperature for 55 minutes. Water was added, the aqueous layer was extracted with ethyl acetate, and the organic layer was washed with water. The organic layer was dried with magnesium sulfate, and the solvent was distilled under reduced pressure, thereby obtaining a crude product (1.57 g, 4.26 mmol, yield: quantitative) of Compound 16.

LC/MS (ESI): m/z = 369, RT = 1.52 min, LC/MS measurement conditions 1

Step 4 Synthesis of Compound 17

CDI(2.78 g, 17.2 mmol), Compound 16 (1.58 g, 4.29 mmol), and DMF (12.6 mL) were mixed. Diisopropylethylamine (3.00 mL, 17.2 mmol) was added to the reaction solution and the resultant solution was irradiated with microwave for 30 minutes while stirred at 110°C. The reaction solution was poured into ice, and the precipitates thus generated were filtered and washed with water. The residue thus obtained was dried under reduced pressure, thereby obtaining a crude product (649 mg, 1.51 mmol, yield 35%) of Compound 17.

LC/MS (ESI): m/z = 395, RT = 1.74 min, LC/MS measurement conditions 1

Step 5 Synthesis of Compound (I-003)

6-Chloro-2-methyl-2H-indazole-5-amine (55.3 mg, 0.304 mmol), Compound 17 (100 mg, 0.254 mmol), and THF (1 mL) were mixed. The reaction solution was cooled in an ice bath, and LHMDS (0.761 mL, 0.761 mmol) was added thereto. The reaction solution was stirred in the ice bath for 40 minutes, and a saturated ammonium chloride aqueous solution was added thereto. The organic layer was extracted with ethyl acetate and concentrated under reduced pressure. The residue was purified with silica gel column chromatography (chloroform/methanol), thereby obtaining Compound (I-003) (80 mg, 0.145 mmol, yield 57.4%).

$^1$H-NMR (Methanol-d4) δ: 8.43 (d, J = 1.0 Hz, 1H), 8.36 (d, J = 2.0 Hz, 1H), 8.18 (s, 1H), 7.74 (s, 1H), 7.72 (br s, 1H), 7.48-7.35 (m, 3H), 5.32 (s, 2H), 4.20 (s, 3H), 2.42 (s, 3H).

LC/MS (ESI): m/z = 528, RT = 1.93 min, LC/MS measurement conditions 1

[Example 2]

Synthesis of Compound (I-005)

[Chemical Formula 28]

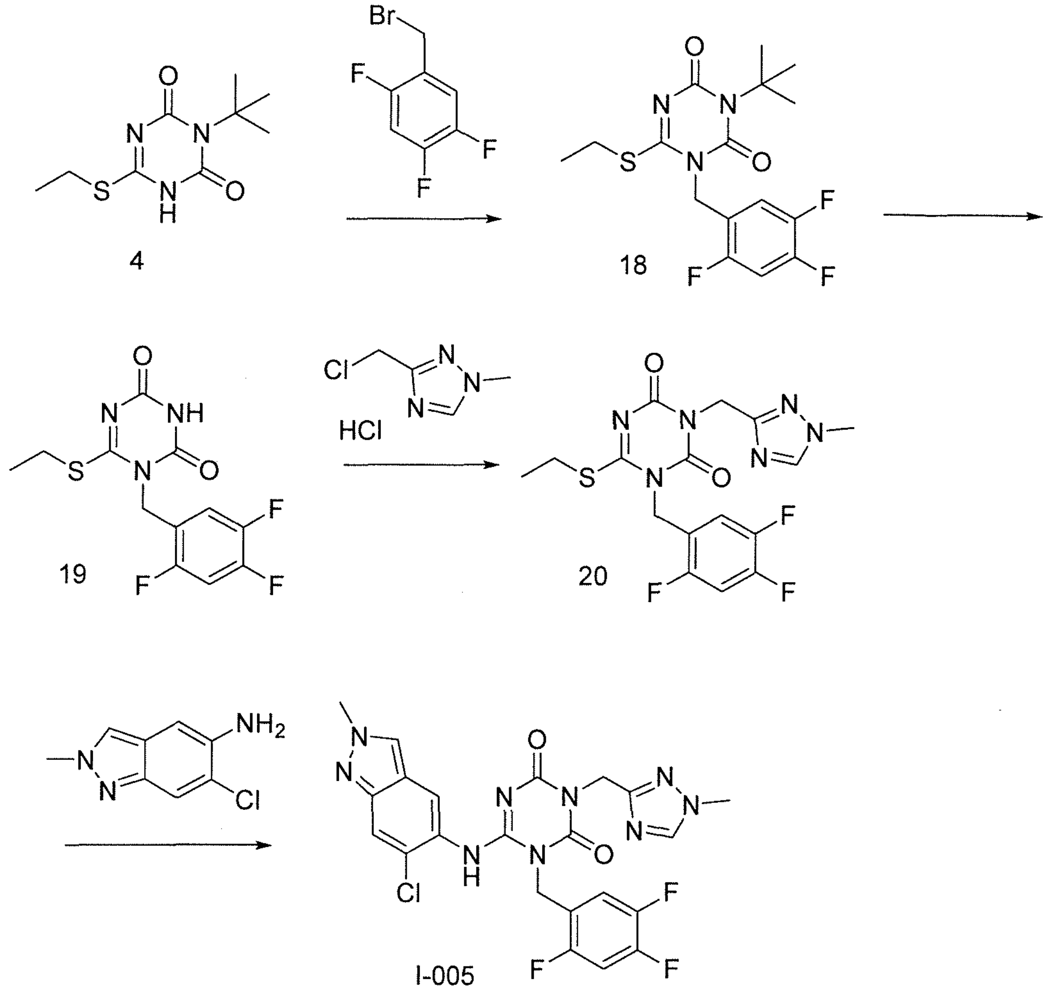


4 18 19 20 I-005

Step 1 Synthesis of Compound 18

Compound 4 (926 mg, 4.04 mmol) (the synthesis method is referred to WO 2012020749, WO 2013089212, and WO 2014200078), acetonitrile (7.41 mL), potassium carbonate (726 mg, 5.25 mmol), and 2,4,5-trifluorobenzyl bromide (1000 mg, 4.44 mmol) were mixed. The reaction solution was stirred at 80°C for 40 minutes, left to be cooled, and then diluted with ethyl acetate. The insoluble matters were filtered, and the filtrate was concentrated, thereby obtaining a crude product (1.51 g, 4.04 mmol, yield: quantitative) of Compound 18.
LC/MS (ESI): m/z = 374, RT = 2.54 min, LC/MS measurement conditions 1

Step 2 Synthesis of Compound 19

Compound 18 (1.51 g, 4.04 mmol) and TFA (3.02 mL) were mixed. The reaction solution was stirred at room temperature for 4 hours and left to stand still overnight. TFA was distilled under reduced pressure, and the residue was azeotropically distilled by adding toluene thereto. The residue was suspended in isopropyl ether and then collected by filtration, thereby obtaining Compound 19 (1.22 g, 3.84 mmol, yield 95%).
LC/MS (ESI): m/z = 318, RT = 1.68 min, LC/MS measurement conditions 1

Step 3 Synthesis of Compound 20

Compound 19 (200 mg, 0.63 mmol), DMF (1.8 mL), potassium carbonate (261 mg, 1.89 mmol), and 3-(chloromethyl)-1-methyl-1H-1,2,4-triazole hydrochloride (159 mg, 0.946 mmol) were mixed. The reaction solution was stirred at 60°C for 2 hours, and a saturated ammonium chloride aqueous solution was added thereto. The aqueous layer was extracted with ethyl acetate, and the organic layer was washed with brine. The organic layer was dried with magnesium sulfate, filtered, and concentrated. The residue was suspended in a mixed solvent of isopropyl ether, hexane, ethyl

acetate, and chloroform and collected by filtration. The residue, DMF (1.8 mL), potassium carbonate (261 mg, 1.89 mmol), and 3-(chloromethyl)-1-methyl-1H-1,2,4-triazole hydrochloride (159 mg, 0.946 mmol) were mixed. The reaction solution was stirred at 60°C for 6 hours, and a saturated ammonium chloride aqueous solution was added thereto. The aqueous layer was extracted with ethyl acetate, and the organic layer was washed with brine. The organic layer was dried with magnesium sulfate, filtered, and concentrated. The residue was suspended in a mixed solvent of isopropyl ether, hexane, ethyl acetate, and chloroform and collected by filtration, thereby obtaining Compound 20 (116 mg, 0.281 mmol, yield 45%)

LC/MS (ESI): m/z = 413, RT = 1.84 min, LC/MS measurement conditions: 1

Step 4 Synthesis of Compound (I-005)

Compound 20 (115 mg, 0.279 mmol), THF (2.30 mL), and 6-chloro-2-methyl-2H-indazole-5-amine (60.8 mg, 0.335 mmol) were mixed. LHMDS (558 μL, 0.558 mmol) was added dropwise at 0°C to the reaction solution. The reaction solution was stirred at 0°C for 2.5 hours and stirred at room temperature for 40 minutes, and a saturated ammonium chloride aqueous solution was added thereto. The aqueous layer was extracted with chloroform, and the organic layer was concentrated. The residue was purified with silica gel column chromatography (chloroform/methanol), thereby obtaining Compound (I-005) (61.8 mg, 0.116 mmol, yield 42%).

$^1$H-NMR ($CDCl_3$) δ: 7.96 (s, 1H), 7.82 (d, J = 2.5Hz, 2H), 7.48 (br s, 1H), 7.45-7.37 (m, 1H), 7.08 (s, 1H), 6.97-6.88 (m, 1H), 5.35 (s, 2H), 5.17 (s, 2H), 4.21 (s, 3H), 3.89 (s, 3H).

LC/MS (ESI): m/z = 532, RT = 1.70 min, LC/MS measurement conditions 1

Compounds which can be used in the present invention were synthesized according to the above-described general synthesis method and the methods described in Examples 1 and 2. The structure and physical properties (LC/MS data) of each compound are shown in the following tables, including the compounds produced in Examples 1 and 2. The compound described by the amino structure in the table may have an imino structure, and the compound described by the imino structure may have an amino structure. That is, even with the same compound, there are cases where the compound has an imino structure or an amino structure depending on crystallization conditions and the like. Even in the case of forming its salt or complex, there are cases where the compound has an imino structure or an amino structure depending on the types of the counter molecule of its salt or complex. Even with the same counter molecule, there are cases where the compound has an imino structure or an amino structure depending on crystallization conditions and the like. Furthermore, it may also be a mixture of a compound having an imino structure, its salt or complex, and a compound having an amino structure, its salt or complex.

[Table 1]

| Compound number | Structural formula | LC/MS Measurement condition | Retention time (min) | m/z |
|---|---|---|---|---|
| I-001 | 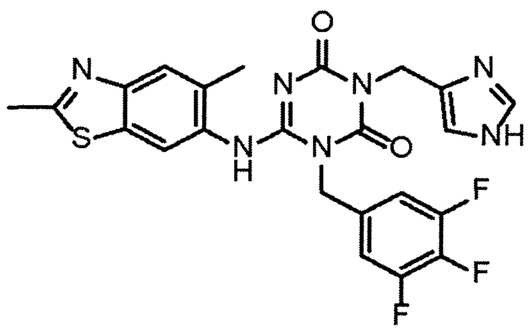 | 1 | 1.74 | 514.05 |

(continued)

| Compound number | Structural formula | LC/MS Measurement condition | Retention time (min) | m/z |
|---|---|---|---|---|
| I-002 | 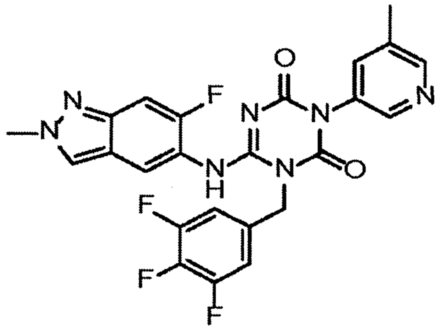 | 1 | 1.80 | 512 |
| I-003 | 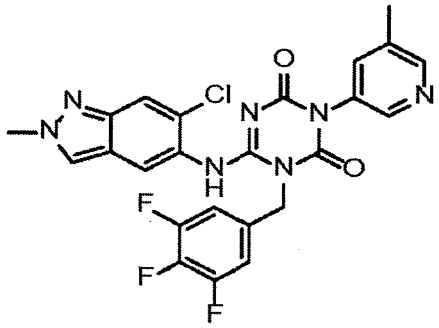 | 1 | 1.93 | 528 |
| I-004 | 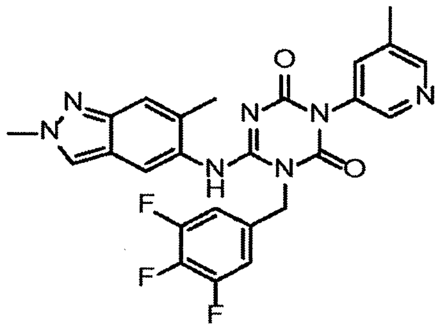 | 1 | 1.68 | 508 |

[Table 2]

| Compound number | Structural formula | LC/MS Measurement condition | Retention time (min) | m/z |
|---|---|---|---|---|
| I-005 | 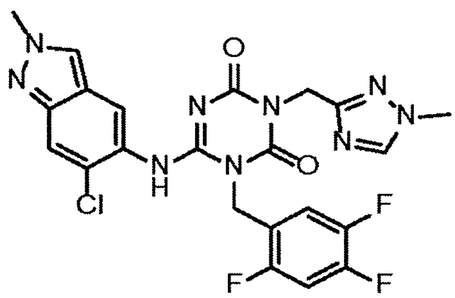 | 1 | 1.70 | 532 |

(continued)

| Compound number | Structural formula | LC/MS Measurement condition | Retention time (min) | m/z |
|---|---|---|---|---|
| I-006 | 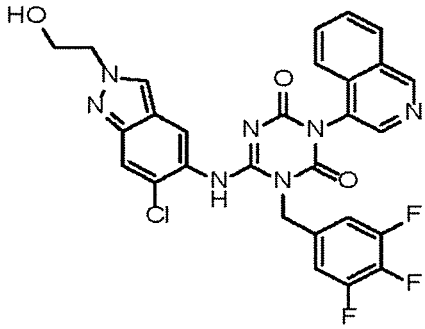 | 2 | 1.82 | 593.95 |
| I-007 | 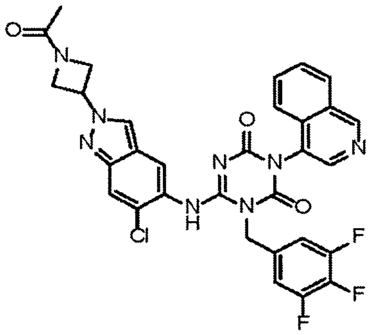 | 1 | 1.93 | 647.15 |
| I-008 | 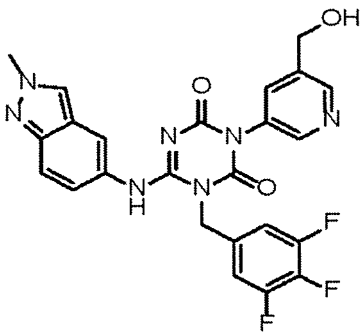 | 1 | 1.54 | 510.2 |

[Table 3]

| Compound number | Structural formula | LC/MS Measurement condition | Retention time (min) | m/z |
|---|---|---|---|---|
| I-009 | 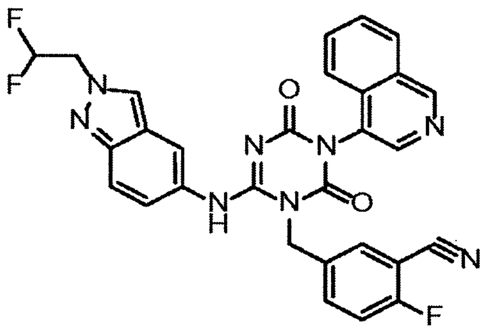 | 1 | 1.83 | 569.2 |

(continued)

| Compound number | Structural formula | LC/MS Measurement condition | Retention time (min) | m/z |
|---|---|---|---|---|
| I-010 | 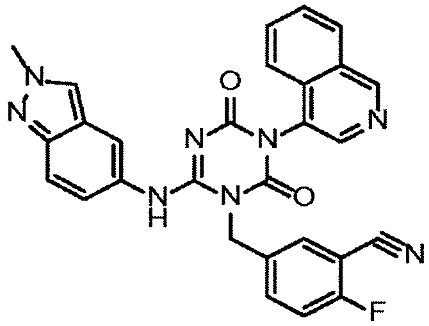 | 1 | 1.68 | 519.2 |
| I-011 | 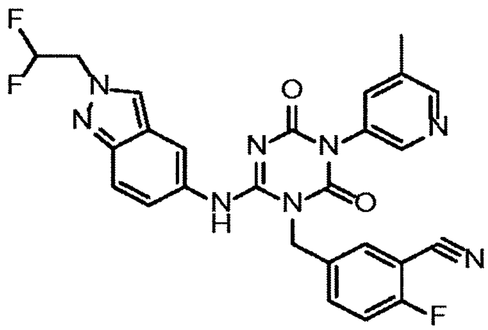 | 1 | 1.68 | 533 |
| I-012 | 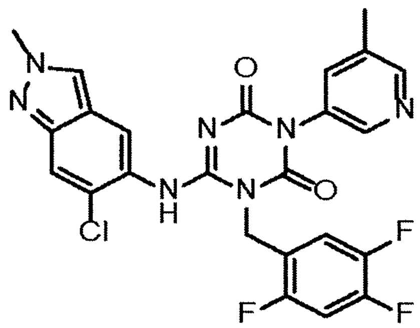 | 1 | 1.85 | 528 |

Table 4]

| Compound number | Structural formula | LC/MS Measurement condition | Retention time (min) | m/z |
|---|---|---|---|---|
| I-013 | 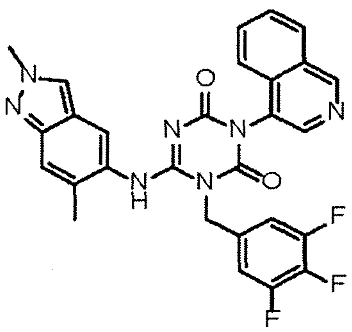 | 1 | 1.81 | 544 |

(continued)

| Compound number | Structural formula | LC/MS Measurement condition | Retention time (min) | m/z |
|---|---|---|---|---|
| I-014 | 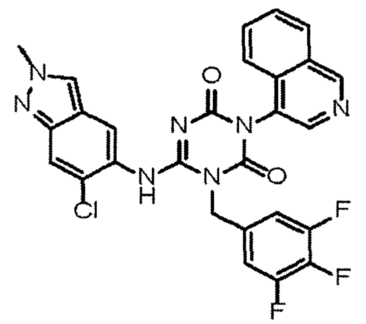 | 2 | 1.96 | 564 |
| I-015 | 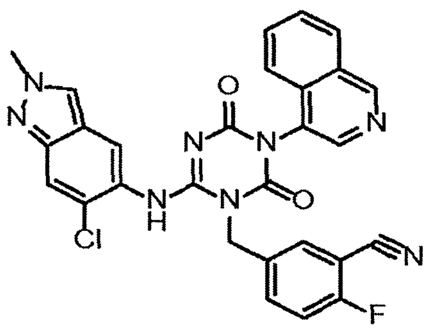 | 2 | 1.76 | 553 |
| I-016 | 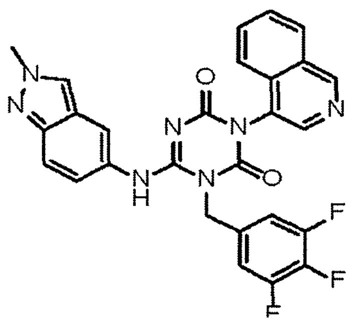 | 2 | 1.70 | 530 |

[Table 5]

| Compound number | Structural formula | LC/MS Measurement condition | Retention time (min) | m/z |
|---|---|---|---|---|
| I-017 | 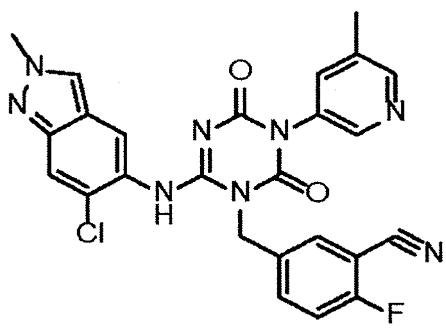 | 2 | 1.64 | 517 |

(continued)

| Compound number | Structural formula | LC/MS Measurement condition | Retention time (min) | m/z |
|---|---|---|---|---|
| I-018 | 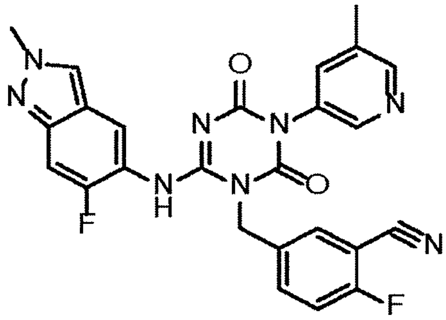 | 2 | 1.54 | 501 |
| I-019 | 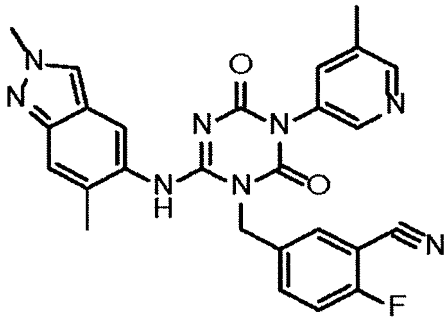 | 2 | 1.49 | 497 |
| I-020 | 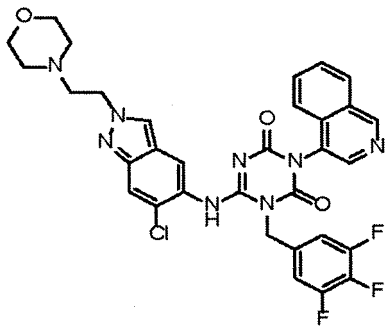 | 1 | 1.68 | 663.25 |

[Table 6]

| Compound number | Structural formula | LC/MS Measurement condition | Retention time (min) | m!z |
|---|---|---|---|---|
| I-021 | 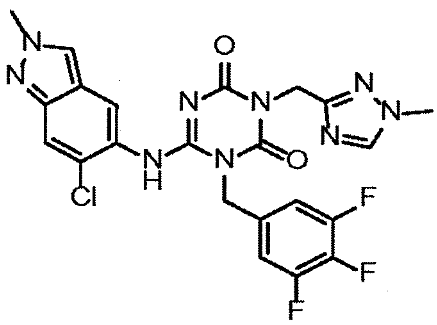 | 2 | 1.80 | 532 |

(continued)

| Compound number | Structural formula | LC/MS Measurement condition | Retention time (min) | m!z |
|---|---|---|---|---|
| I-022 | 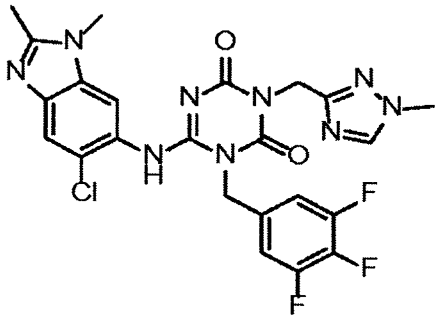 | 2 | 1.49 | 546 |
| I-023 | 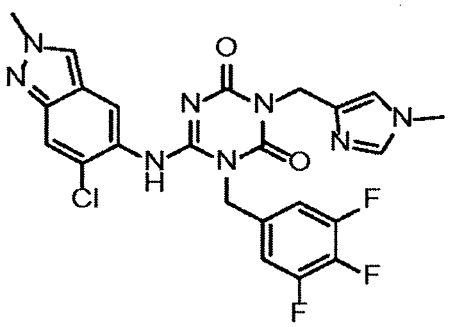 | 2 | 1.31 | 531 |
| I-024 | 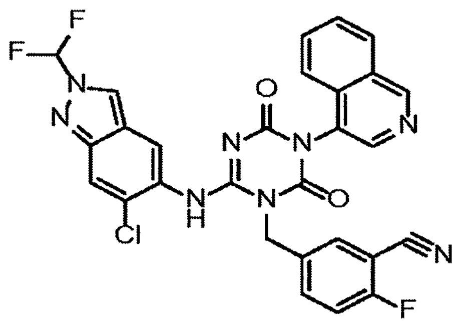 | 2 | 2.04 | 589 |

[Table 7]

| Compound number | Structural formula | LC/MS Measurement condition | Retention time (min) | m/z |
|---|---|---|---|---|
| I-025 | 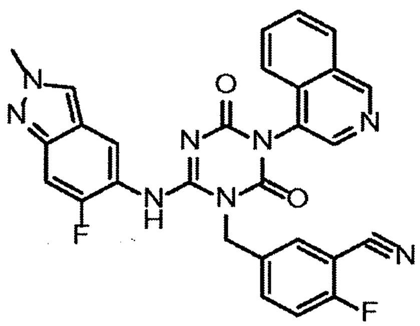 | 2 | 1.76 | 537 |

(continued)

| Compound number | Structural formula | LC/MS Measurement condition | Retention time (min) | m/z |
|---|---|---|---|---|
| I-026 | 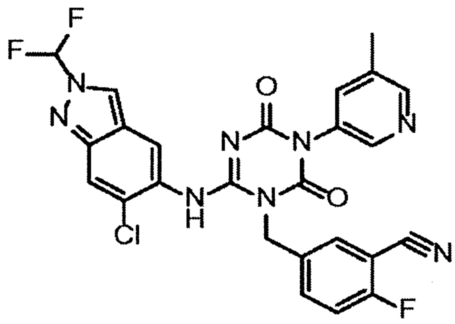 | 2 | 2.05 | 553 |
| I-027 | 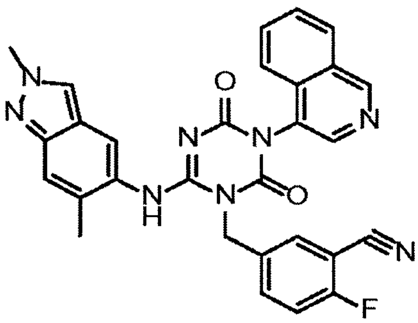 | 2 | 1.61 | 533 |
| I-028 | 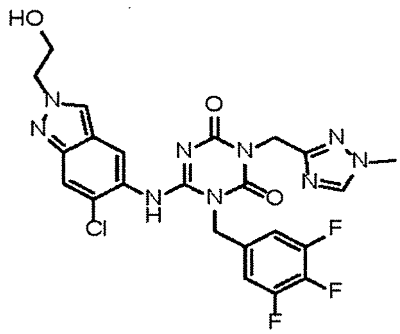 | 2 | 1.69 | 562 |

[Table 8]

| Compound number | Structural formula | LC/MS Measurement condition | Retention time (min) | m/z |
|---|---|---|---|---|
| I-029 | 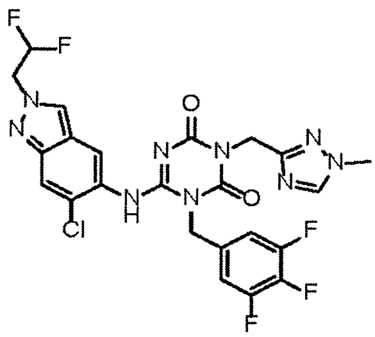 | 2 | 1.98 | 582 |

(continued)

| Compound number | Structural formula | LC/MS Measurement condition | Retention time (min) | m/z |
|---|---|---|---|---|
| I-030 | 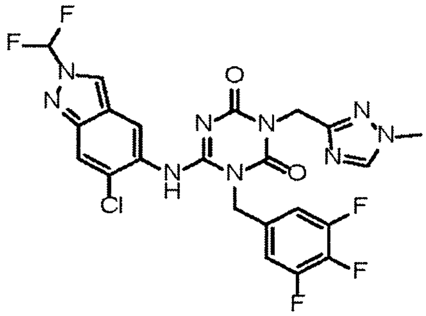 | 2 | 2.00 | 568 |
| I-031 | 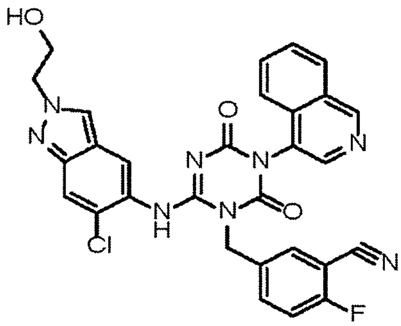 | 2 | 1.74 | 583 |
| I-032 | 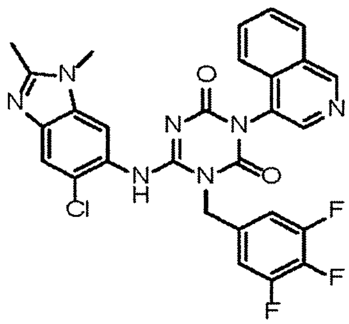 | 1 | 1.71 | 578.2 |

[Table 9]

| Compound number | Structural formula | LC/MS Measurement condition | Retention time (min) | m/z |
|---|---|---|---|---|
| I-033 | 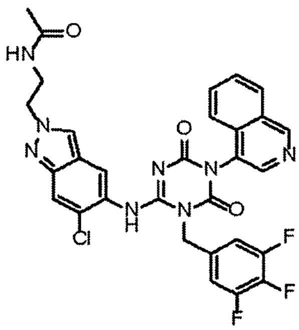 | 1 | 1.94 | 635 |

(continued)

| Compound number | Structural formula | LC/MS Measurement condition | Retention time (min) | m/z |
|---|---|---|---|---|
| I-034 | 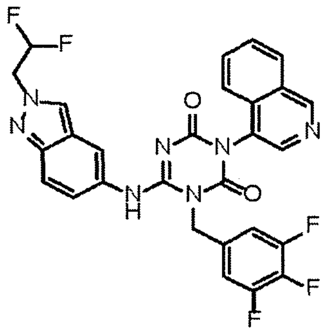 | 2 | 1.87 | 579.95 |
| I-035 | 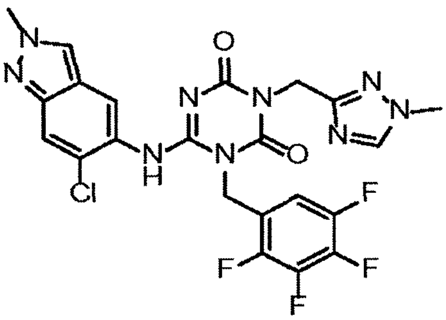 | 1 | 1.93 | 550 |

[Example 3]

To 1400 mg of Compound (I-003), 7 mL of ethyl acetate and a 557 μL (1.05 eq) aqueous solution of 5 mol/L p-toluenesulfonic acid were added. The resultant mixture was stirred at 60°C for 15 minutes and then stirred at 25°C for 2 hours. The solid was collected by filtration and dried, there by obtaining p-toluenesulfonate crystal Form I (1289.6 mg, 69%) of the compound represented by Formula (I-A).

The result of the single crystal structural analysis of p-toluenesulfonate crystal Form I of the compound represented by Formula (I-A) is shown below.

R1 (I > 2.00s(I)) was 0.0444, and it was confirmed that there is neither a lack of electronic density nor misplacing of atom from final difference Fourier.

Crystallographic data are shown in Table 10.

[Table 10]

| | |
|---|---|
| Space group | P-1 |
| a (Å) | 8.7844(2) |
| b (Å) | 10.2991(2) |
| c (Å) | 18.0182(3) |
| α (°) | 103.727(2) |
| β (°) | 97.411(2) |
| γ (°) | 100.358(2) |
| Volume ($Å^3$) | 1532.43(6) |
| Z | 2 |
| Density (Calculation value) ($g/cm^3$) | 1.517 |
| Measurement temperature (K) | 298 |

Herein, Volume means the unit lattice volume, and Z means the number of molecules in the unit lattice.
The structure of the p-toluenesulfonate crystal Form I of the compound represented by Formula (I-A) in the asymmetric unit is shown in Fig. 2.
One molecule of each of the compound represented by Formula (I-A) and p-toluenesulfonic acid was present in the asymmetric unit. It was confirmed that the salt is formed since hydrogen atoms are not bonded to oxygen atoms represented by O3, O4, and O5 of p-toluenesulfonic acid and hydrogen atoms are bonded to the nitrogen atom of N7 of the compound represented by Formula (I-A).
The bond length of N3-C9 was about 1.27 Å, and the bond length of N4-C9 was about 1.37 Å. From this bond length, the compound represented by Formula (I-A) of the p-toluenesulfonate crystal Form I was identified to have an imino structure:

[Chemical Formula 29]

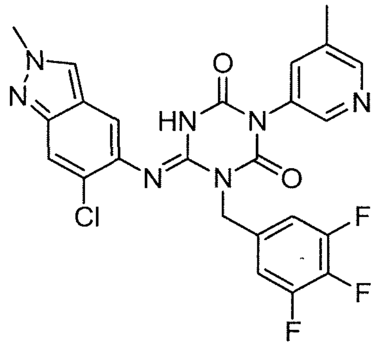

Furthermore, the result of the X-ray powder diffraction of p-toluenesulfonate crystal Form I of the compound represented by Formula (I-A) is shown.
In the X-ray powder diffraction pattern, the peaks were observed at the diffraction angle (2θ): 9.1 ± 0.2°, 11.5 ± 0.2°, 14.6 ± 0.2°, 15.2 ± 0.2°, 18.8 ± 0.2°, 20.2 ± 0.2°, 23.6 ± 0.2°, 24.2 ± 0.2°, 24.9 ± 0.2°, and 26.9 ± 0.2°.
In the above X-ray powder diffraction pattern, the peaks of the diffraction angle (20): 9.1 ± 0.2°, 15.2 ± 0.2°, 18.8 ± 0.2°, 23.6 ± 0.2°, and 24.9 ± 0.2° are particularly characteristic as the p-toluenesulfonate crystal Form I of the compound represented by Formula (I-A).

[Example 4]

To 1170 mg of Compound (I-005), 278 mg (1.1 eq) of fumaric acid and 5.85 mL of ethyl acetate were added, and the resultant mixture was stirred at room temperature for 45 minutes. The solid was collected by filtration and dried, there by obtaining fumaric acid cocrystal Form I crystal (1369.4 mg, 94.6%) of the compound represented by Formula (I-B).
The result of the single crystal structural analysis of fumaric acid cocrystal Form I of the compound represented by Formula (I-B) is shown below.
R1 (I > 2.00s(I)) was 0.0470, and it was confirmed that there is neither a lack of electronic density nor misplacing of atom from final difference Fourier.
Crystallographic data are shown in Table 11.

[Table 11]

| | |
|---|---|
| Space group | P-1 |
| a (Å) | 8.4374(2) |
| b (Å) | 11.6780(3) |
| c (Å) | 15.1612(4) |
| α (°) | 83.827(2) |
| β (°) | 78.868(2) |
| γ (°) | 77.147(2) |
| Volume ($Å^3$) | 1425.77(6) |

(continued)

| | |
|---|---|
| Space group | P-1 |
| Z | 2 |
| Density (Calculation value) (g/cm$^3$) | 1.509 |
| Measurement temperature (K) | 298 |

Herein, Volume means the unit lattice volume, and Z means the number of molecules in the unit lattice.

The structure of the fumaric acid cocrystal Form I of the compound represented by Formula (I-B) in the asymmetric unit is shown in Fig. 4.

One molecule of each of the compound represented by Formula (I-B) and fumaric acid was present in the asymmetric unit. The ionic chemical interaction was not confirmed, and the structure was confirmed to be a cocrystal at a molar ratio of 1 : 1.

The bond length of N10-C9 was about 1.26 Å, and the bond length of N16-C9 was about 1.37 Å. From this bond length, the compound represented by Formula (I-B) of the fumaric acid cocrystal Form I was identified to have an imino structure:

[Chemical Formula 30]

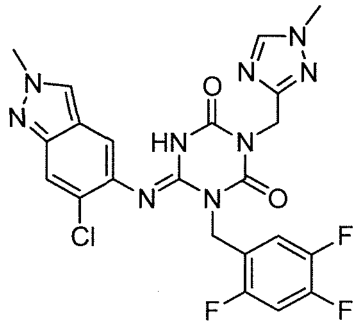

Furthermore, the result of the X-ray powder diffraction of fumaric acid cocrystal Form I crystal of the compound represented by Formula (I-B) is shown.

In the X-ray powder diffraction pattern, the peaks were observed at the diffraction angle (2θ): 7.8 ± 0.2°, 9.5 ± 0.2°, 10.1 ± 0.2°, 10.9 ± 0.2°, 13.8 ± 0.2°, 14.7 ± 0.2°, 18.6 ± 0.2°, 22.6 ± 0.2°, 23.5 ± 0.2°, and 24.6 ± 0.2°.

In the above X-ray powder diffraction pattern, the peaks of the diffraction angle (2θ): 9.5 ± 0.2°, 10.9 ± 0.2°, 18.6 ± 0.2°, 23.5 ± 0.2°, and 24.6 ± 0.2° are particularly characteristic as the fumaric acid cocrystal Form I crystal of the compound represented by Formula (I-B).

Hereinafter, biological test examples of the compound according to the present invention are described. The compounds of the present invention can be tested essentially as described in test examples below.

The compound represented by Formula (I) according to the present invention may have coronavirus 3CL protease inhibitory activity and may inhibit coronavirus 3CL protease.

Specifically, in the evaluation method described below, IC50 is preferably 50 μM or less, more preferably 1 μM or less, and even more preferably 100 nM or less.

Test Example 1: Inhibitory activity of cytopathic effect (CPE) in Vero E6 cells expressing human TMPRSS2 (Vero E6/TMPRSS2 cells)

<Operation procedure>

· Diluting and dispensing of test sample

The test sample is preliminarily diluted with DMSO to an appropriate concentration, and a 2- to 5-fold serial dilution series is prepared and then dispensed into a 384-well plate.

· Diluting and dispensing of cells and SARS-CoV-2

VeroE6/TMPRSS2 cells (JCRB1819, $5 \times 10^3$ cells/well) and SARS-CoV-2 (100-300 $TCID_{50}$/well) are mixed in a medium (MEM, 2% FBS, penicillin-streptomycin), dispensed into a well containing the test sample, and then cultured in a $CO_2$ incubator for 3 days.

· Dispensing and measuring of luminescence signal of CellTiter-Glo (registered trademark) 2.0

After returning the plate cultured for 3 days to room temperature, CellTiter-Glo (registered trademark) 2.0 is dispensed into each well and mixed with a plate mixer. After a certain time interval, the luminescence signal (Lum) is measured with a plate reader.

<Calculation of each measurement item>

· Calculation of 50% SARS-CoV-2 infected cell death inhibitory concentration ($EC_{50}$)

When x is taken as the logarithmic value of the compound concentration and y as %Efficacy, the inhibition curve is approximated by the following Logistic regression equation, and the value of x when substituting 50 (%) for y is calculated as $EC_{50}$.

$$y = \min + (\max - \min)/\{1 + (X50/x)\ \hat{}\,\mathrm{Hill}\}$$

$$\%\mathrm{Efficacy} = \{(\mathrm{Sample} - \mathrm{virus\ control})/(\mathrm{cell\ control} - \mathrm{virus\ control})\} * 100\%$$

cell control: the average of Lum of cell control wells
virus control: the average of Lum of virus control wells
min: lower limit of y-axis, max: upper limit of y-axis, X50: x-coordinate of inflection point, Hill: slope of curve at midpoint between min and max

The compounds of the present invention were tested essentially as described above. Results are shown in the following table.
Note that, $EC_{50}$ value is set as "A" for less than 1 μM and "B" for 1 μM or more and less than 10 μM.

Compound I-003: 0.177 μM
Compound I-005: 0.328 μM
Compound I-006: 0.747 μM
Compound I-010: 0.306 μM
Compound I-012: 0.131 μM
Compound I-017: 0.0960 μM
Compound I-023: 1.03 μM
Compound I-035: 0.395 μM

[Table 12]

| Compound number | EC50 | Compound number | EC50 | Compound number | EC50 | Compound number | EC50 |
|---|---|---|---|---|---|---|---|
| I-002 | A | I-015 | A | I-024 | A | I-031 | B |
| I-004 | B | I-016 | A | I-025 | A | I-032 | B |
| I-007 | C | I-018 | A | I-026 | A | I-033 | B |
| I-009 | A | I-019 | A | I-027 | A | I-034 | A |

(continued)

| Compound number | EC50 | Compound number | EC50 | Compound number | EC50 | Compound number | EC50 |
|---|---|---|---|---|---|---|---|
| I-011 | A | I-020 | B | I-028 | B | | |
| I-013 | B | I-021 | A | I-029 | A | | |
| I-014 | A | I-022 | B | I-030 | B | | |

Test Example 1-2: Inhibitory activity of cytopathic effect (CPE) in Vero E6 cells expressing human TMPRSS2 (Vero E6/TMPRSS2 cells)

<Operation procedure>

· Diluting and dispensing of test sample

The test sample is preliminarily diluted with DMSO to an appropriate concentration, and a 3-fold serial dilution series is prepared and then dispensed into a 96-well plate.

· Diluting and dispensing of cells and SARS-CoV-2

VeroE6/TMPRSS2 cells (JCRB1819, $1.5 \times 10^4$ cells/well) and SARS-CoV-2 hCoV-19/Japan/TY/WK-521/2020, hCoV-19/Japan/QK002/2020, hCoV-19/Japan/QHN001/2020, hCoV-19/Japan/QHN002/2020, hCoV-19/Japan/TY7-501/2021, hCoV-19/Japan/TY7-503/2021, hCoV-19/Japan/TY8-612/2021, hCoV-19/Japan/TY11-927-P1/2021, hCoV-19/Japan/TY33-456/2021, hCoV-19/Japan/TY28-444/2021, hCoV-19/Japan/TY26-717/2021 (30-3000 $TCID_{50}$/well) are mixed in a culture medium (MEM, 2% FBS, penicillin-streptomycin), dispensed into a well containing the test sample, and then cultured in a $CO_2$ incubator for 3 days or 4 days.

· Dispensing and measuring of luminescence signal of CellTiter-Glo (registered trademark) 2.0

After returning the plate cultured for 3 days to room temperature, CellTiter-Glo (registered trademark) 2.0 is dispensed into each well and mixed with a plate mixer. After a certain time interval, the luminescence signal (Lum) is measured with a plate reader.

<Calculation of each measurement item>

· Calculation of 50% SARS-CoV-2 infected cell death inhibitory concentration ($EC_{50}$)

When x is taken as the logarithmic value of the compound concentration and y as %Efficacy, the inhibition curve is approximated by the following Logistic regression equation, and the value of x when substituting 50 (%) for y is calculated as $EC_{50}$.

$$y = \min + (\max - \min)/\{1 + (X50/x)\ \hat{}\,\mathrm{Hill}\}$$

$$\%\mathrm{Efficacy} = \{(\mathrm{Sample} - \mathrm{virus\ control})/(\mathrm{cell\ control} - \mathrm{virus\ control})\} * 100\%$$

cell control: the average of Lum of cell control wells
virus control: the average of Lum of virus control wells
min: lower limit of y-axis, max: upper limit of y-axis, X50: x-coordinate of inflection point, Hill: slope of curve at midpoint between min and max

The compounds of the present invention were tested essentially as described above. Results are shown below.

[Table 13-1]

| Virus strain | p-Toluenesulfonate crystal Form I of compound represented by Formula (I-A) $EC_{50}$ ($\mu$M) | Fumaric acid cocrystal Form I crystal of compound represented by Formula (I-B) $EC_{50}$ ($\mu$M) |
|---|---|---|
| WT strain (WK-521) | 0.12 | 0.37 |
| Alpha strain (QHN001/QHN002/QK002) | 0.14/0.13/0.16 | 0.31/0.46/0.33 |
| Beta strain (TY8-612) | 0.16 | 0.40 |
| Gamma strain (TY7-501/TY7-503) | 0.22/0.15 | 0.50/0.43 |
| Delta strain (TY11-927-P1) | Not tested | 0.41 |
| Lambda strain (TY33-456) | Not tested | 0.27 |
| Theta strain (TY28-444) | Not tested | 0.29 |
| Mu strain (TY26-717) | Not tested | 0.43 |

Test Example 1-3: Cytopathic effect (CPE) suppression effect confirmation test using Vero E6 cells expressing human TMPRSS2 (Vero E6/TMPRSS2 cells)

<Operation procedure>

· Diluting and dispensing of test sample

The test sample is preliminarily diluted with DMSO to an appropriate concentration, and a 3-fold serial dilution series is prepared and then dispensed into a 96-well plate.

· Diluting and dispensing of cells and SARS-CoV-2

VeroE6/TMPRSS2 cells (JCRB1819, $1.5 \times 10^4$ cells/well) and SARS-CoV-2 hCoV-19/Japan/TY38-873/2021, hCoV-19/Japan/TY38-871/2021, hCoV-19/Japan/TY40-385/2022, hCoV-19/Japan/TY41-716/2022, hCoV-19/Japan/TY41-703/2022, hCoV-19/Japan/TY41-702/2022, hCoV-19/Japan/TY41-686/2022 (300-3000 $TCID_{50}$/well) are mixed in a culture medium (MEM, 2% FBS, penicillin-streptomycin), dispensed into a well containing the test sample, and then cultured in a $CO_2$ incubator for 4 days.

· Dispensing and measuring of luminescence signal of CellTiter-Glo (registered trademark) 2.0

After returning the plate cultured for 3 days to room temperature, CellTiter-Glo (registered trademark) 2.0 is dispensed into each well and mixed with a plate mixer. After a certain time interval, the luminescence signal (Lum) is measured with a plate reader.

<Calculation of each measurement item>

· Calculation of 50% SARS-CoV-2 infected cell death inhibitory concentration ($EC_{50}$)

When x is taken as the logarithmic value of the compound concentration and y as %Efficacy, the inhibition curve is approximated by the following Logistic regression equation, and the value of x when substituting 50 (%) for y is calculated as $EC_{50}$.

$$y = \min + (\max - \min)/\{1 + (X50/x)\ \hat{}\ Hill\}$$

$$\%Efficacy = \{(Sample - virus\ control)/(cell\ control - virus\ control)\} * 100\%$$

cell control: the average of Lum of cell control wells
virus control: the average of Lum of virus control wells
min: lower limit of y-axis, max: upper limit of y-axis, X50: x-coordinate of inflection point, Hill: slope of curve at midpoint between min and max

The compounds of the present invention were tested essentially as described above. Results are shown below.

[Table 13-2]

| Virus strain Omicron Strain | Fumaric acid cocrystal Form I crystal of compound represented by Formula (I-B) $EC_{50}$ (μM) |
|---|---|
| TY38-873 (BA.1) | 0.29 |
| TY38-871 (BA.1.1) | 0.36 |
| TY40-385 (BA.2) | 0.52 |
| TY41-716 (BA.2.75) | 0.30 |
| TY41-703 (BA.4) | 0.22 |
| TY41-702 (BA.5) | 0.40 |

(continued)

| Virus strain Omicron Strain | Fumaric acid cocrystal Form I crystal of compound represented by Formula (I-B) $EC_{50}$ (μM) |
|---|---|
| TY41-686 (XE) | 0.44 |

Test Example 2: Inhibitory activity test against SARS-CoV-2 3CL protease

<Material>

- · Commercially available Recombinant SARS-CoV-2 3CL Protease
- · Commercially available substrate peptide
  Dabcyl-Lys-Thr-Ser-Ala-Val-Leu-Gln-Ser-Gly-Phe-Arg-Lys-Met-Glu(Edans)-NH2 (SEQ ID NO: 1)
- · Internal Standard peptide
  Dabcyl-Lys-Thr-Ser-Ala-Val-Leu(13C6,15N)-Gln (SEQ ID NO: 2)

Dabcyl-Lys-Thr-Ser-Ala-Val-Leu(13C6,15N)-Gln can be synthesized with reference to the literature (Atherton, E. ; Sheppard, R. C., "In Solid Phase Peptide Synthesis, A Practical Approach", IRL Press at Oxford University Pres, 1989. and Bioorg. Med. Chem., Volume 5, Issue 9, 1997, pp. 1883-1891, etc.). An example will be described below.

H-Lys-Thr-Ser-Ala-Val-Leu(13C6,15N)-Glu(resin)-OαOtBu (the Lys side chain is Boc-protected, the Thr side chain is protected with a tert-butyl group, the Ser side chain is protected with a tert-butyl group, the C-terminal OH of Glu is protected with a tert-butyl group, and the carboxylic acid of the Glu side chain is condensed into the resin) is synthesized using Rink amide resin by Fmoc solid-phase synthesis. Modification of the N-terminus Dabcyl group condenses 4-dimethylaminoazobenzene-4'-carboxylic acid (Dabcyl-OH) on resins using EDC/HOBT. Final deprotection and excision from the resins are performed by treatment with TFA/EDT = 95 : 5. Thereafter, purification is performed by reverse phase HPLC.

· RapidFire Cartridge C4 type A

<Operation procedure>

· Preparation of assay buffer

In this test, an assay buffer consisting of 20 mM Tris-HCl, 100 mM sodium chloride, 1 mM EDTA, 10 mM DTT, and 0.01% BSA is used. For compounds with an $IC_{50}$ value of 10 nM or less, an assay buffer consisting of 20 mM Tris-HCl, 1 mM EDTA, 10 mM DTT, and 0.01% BSA is used.

· Diluting and dispensing of test sample

The test sample is preliminarily diluted with DMSO to an appropriate concentration, and a 2- to 5-fold serial dilution series is prepared and then dispensed into a 384-well plate.

· Addition of enzyme and substrate and enzymatic reaction

To the prepared compound plate, 8 μM of substrate and 6 or 0.6 nM of enzyme solution are added and incubation is performed for 3 to 5 hours at room temperature. Thereafter, a reaction stop solution (0.067 μM Internal Standard, 0.1% formic acid, 10 or 25% acetonitrile) is added to stop the enzymatic reaction.

· Measurement of reaction product

The plate in which the reaction has been completed is measured using RapidFire System 360 and mass spectrometer (Agilent Technologies, Inc., 6550 iFunnel Q-TOF), or Rapid Fire System 365 and mass spectrometer (Agilent Technologies, Inc., 6495C Triple Quadrupole). As the mobile phase at the time of measurement, A solution (75% isopropanol, 15% acetonitrile, 5 mM ammonium formate) and B solution (0.01% trifluoroacetic acid, 0.09% formic acid) are used.

Reaction products detected by the mass spectrometer are calculated using RapidFire Integrator or a program capable of performing equivalent analysis and are taken as Product area value. Furthermore, Internal Standard detected at the same time is also calculated and taken as Internal Standard area value.

<Calculation of each measurement item>

· Calculation of P/IS

The area values obtained in the previous section is calculated by the following equation to calculate P/IS.
P/IS = Product area value/Internal Standard area value

· Calculation of 50% SARS-CoV-2 3CL protease inhibitory concentration ($IC_{50}$)

When x is taken as the logarithmic value of the compound concentration and y as %Inhibition, the inhibition curve is approximated by the following Logistic regression equation, and the value of x when substituting 50 (%) for y is calculated as $IC_{50}$.

$$y = min + (max - min)/\{1 + (X50/x)\ \hat{}Hill\}$$

$$\%Inhibition = \{1-(Sample - Control(-))/Control(+)-Control(-))\} * 100$$

Control(-): the average of P/IS of enzyme inhibited condition wells
Control(+): the average of P/IS of DMSO control wells
min: lower limit of y-axis, max: upper limit of y-axis, X50: x-coordinate of inflection point, Hill: slope of curve at midpoint between min and max

The compounds of the present invention were tested essentially as described above. Results are shown in the following table.
Note that, $IC_{50}$ value is set as "A" for less than 0.1 μM, "B" for 0.1 μM or more and less than 1 μM, and "C" for 1 μM or more and less than 10 μM.

Compound I-003: 0.014 μM
Compound I-005: 0.010 μM
Compound I-006: 0.0058 μM
Compound I-010: 0.0054 μM
Compound I-012: 0.0091 μM
Compound I-017: 0.0034 μM
Compound I-023: 0.0063 μM
Compound I-035: 0.0098 μM

[Table 14-1]

| Compound number | IC50 | Compound number | IC50 | Compound number | IC50 | Compound number | IC50 |
|---|---|---|---|---|---|---|---|
| I-001 | C | I-013 | A | I-021 | A | I-029 | A |
| I-002 | A | I-014 | A | I-022 | A | I-030 | A |
| I-004 | A | I-015 | A | I-024 | A | I-031 | A |
| I-007 | A | I-016 | A | I-025 | A | I-032 | A |
| I-008 | B | I-018 | A | I-026 | A | I-033 | A |
| I-009 | A | I-019 | A | I-027 | A | I-034 | A |
| I-011 | A | I-020 | A | I-028 | A | | |

Test Example 2-2: Inhibitory activity test against SARS-CoV-2 3CL protease

<Material>

- · Commercially available Recombinant SARS-CoV-2 3CL Protease
- · SARS-CoV-2 3CL Protease mutations (G15S, T21I, T24I, K88R, L89F, K90R, P108S, P132H, A193V, H246Y, A255V)
- · Commercially available substrate peptide
  Dabcyl-Lys-Thr-Ser-Ala-Val-Leu-Gln-Ser-Gly-Phe-Arg-Lys-Met-Glu(Edans)-NH2 (SEQ ID NO: 1)
- · Internal Standard peptide
  Dabcyl-Lys-Thr-Ser-Ala-Val-Leu(13C6,15N)-Gln (SEQ ID NO: 2)

Dabcyl-Lys-Thr-Ser-Ala-Val-Leu(13C6,15N)-Gln can be synthesized with reference to the literature (Atherton, E. ; Sheppard, R. C., "In Solid Phase Peptide Synthesis, A Practical Approach", IRL Press at Oxford University Pres, 1989. and Bioorg. Med. Chem., Volume 5, Issue 9, 1997, pp. 1883-1891, etc.). An example will be described below.

H-Lys-Thr-Ser-Ala-Val-Leu(13C6,15N)-Glu(resin)-OaOtBu (the Lys side chain is Boc-protected, the Thr side chain is protected with a tert-butyl group, the Ser side chain is protected with a tert-butyl group, the C-terminal OH of Glu is protected with a tert-butyl group, and the carboxylic acid of the Glu side chain is condensed into the resin) is synthesized using Rink amide resin by Fmoc solid-phase synthesis. Modification of the N-terminus Dabcyl group condenses 4-dimethylaminoazobenzene-4'-carboxylic acid (Dabcyl-OH) on resins using EDC/HOBT. Final deprotection and excision from the resins are performed by treatment with TFA/EDT = 95 : 5. Thereafter, purification is performed by reverse phase HPLC.

· RapidFire Cartridge C4 type A

<Operation procedure>

· Preparation of assay buffer

In this test, an assay buffer consisting of 20 mM Tris-HCl, 1 mM EDTA, 10 mM DTT, and 0.01% BSA is used.

· Diluting and dispensing of test sample

The test sample is preliminarily diluted with DMSO to an appropriate concentration, and a 3-fold serial dilution series is prepared and then dispensed into a 384-well plate.

· Addition of enzyme and substrate and enzymatic reaction

To the prepared compound plate, a substrate with a final concentration of 2 to 4 μM and an enzyme solution with a final concentration of 2 to 6 nM are added, and incubation is performed at room temperature for 2 to 3 hours. Thereafter, a reaction stop solution (0.072 μM Internal Standard, 0.1% formic acid, 10% acetonitrile) is added to stop the enzymatic reaction.

· Measurement of reaction product

The plate in which the reaction has been completed is measured using RapidFire System 360 and mass spectrometer (Agilent Technologies, Inc., 6550 iFunnel Q-TOF). As the mobile phase at the time of measurement, A solution (75% isopropanol, 15% acetonitrile, 5 mM ammonium formate) and B solution (0.01% trifluoroacetic acid, 0.09% formic acid) are used.

Reaction products detected by the mass spectrometer are calculated using RapidFire Integrator and are taken as Product area value. Furthermore, Internal Standard detected at the same time is also calculated and taken as Internal Standard area value.

<Calculation of each measurement item>

· Calculation of P/IS

The area values obtained in the previous section is calculated by the following equation to calculate P/IS. P/IS = Product area value/Internal Standard area value

· Calculation of 50% SARS-CoV-2 3CL protease inhibitory concentration ($IC_{50}$)

When x is taken as the logarithmic value of the compound concentration and y as %Inhibition, the inhibition curve is approximated by the following Logistic regression equation, and the value of x when substituting 50 (%) for y is calculated as $IC_{50}$.

$$y = \min + (\max - \min)/\{1 + (X50/x)\ \hat{}\,Hill\}$$

$$\%Inhibition = \{1-(Sample - Control(-))/Control(+)-Control(-))\} * 100$$

Control(-): the average of P/IS ratio in the wells without SARS-CoV-2 3CL protease and test substance
Control(+): the average of P/IS ratio in the wells with SARS-CoV-2 3CL protease and without test substance
min: lower limit of y-axis, max: upper limit of y-axis, X50: x-coordinate of inflection point, Hill: slope of curve at midpoint between min and max

The compounds of the present invention were tested essentially as described above. Results are shown below.

[Table 14-2]

| 3CL Protease mutation | p-Toluenesulfonate crystal Form I of compound represented by Formula (I-A) $EC_{50}$ (μM) | Fumaric acid cocrystal Form I crystal of compound represented by Formula (I-B) $EC_{50}$ (μM) |
|---|---|---|
| WT | 0.0167 | 0.0132 |
| G15S | Not tested | 0.0080 |
| T21I | Not tested | 0.0143 |
| T24I | Not tested | 0.0140 |
| K88R | Not tested | 0.0121 |
| L89F | Not tested | 0.0150 |
| K90R | Not tested | 0.0097 |
| P108S | Not tested | 0.0132 |
| P132H | Not tested | 0.0144 |
| A193V | Not tested | 0.0102 |
| H246Y | Not tested | 0.0125 |
| A255V | Not tested | 0.0101 |

Test Example 3: Lung virus titer increase suppression test of SARS-CoV-2-infected mice by administration of fumaric acid cocrystal Form I crystal of compound represented by Formula (I-B)

<Material and method>

· Compound

The fumaric acid cocrystal Form I crystal of the compound represented by Formula (I-B) was prepared using a 0.5% methylcellulose (0.5% MC) solution. The administration volume was set to 10 mL/kg. The dose indicates an amount calculated as a free form.

· Virus

The SARS-CoV-2 hCoV19/Japan/TY7-501/2021 strain separated in National Institute of Infectious Diseases was used.

· Murine lung infection, drug administration, lung collection

Specific pathogen-free 5-week-old female BALB/c mice (CLEA Japan, Inc.) were used in this research. Upon virus inoculation, the mice were anesthetized by intramuscular administration of 100 $\mu$L of an anesthetic solution containing 0.03 mg/mL of medetomidine hydrochloride, 0.4 mg/mL of midazolam, and 0.5 mg/mL of butorphanol tartrate in PBS. Under anesthesia, the mice were intranasally inoculated with 50 $\mu$L of hCoV19/Japan/TY7-501/2021 ($1.00 \times 10^4$ $TCID_{50}$). While the start point was set as being the point immediately after virus infection, the fumaric acid cocrystal Form I crystal of the compound represented by Formula (I-B) was orally administered to mice (n = 5-10/group) at a dose of 2 mg/kg, 4 mg/kg, 8 mg/kg, 16 mg/kg, 32 mg/kg, and 64 mg/kg in a single dose or at a dose of 1 mg/kg, 2 mg/kg, 4 mg/kg, 8 mg/kg, 16 mg/kg, and 32 mg/kg twice a day. To mice for comparison, 0.5% MC was orally administered twice a day. The murine lung was collected 1 day after virus infection, 2 mL of PBS was added thereto and homogenized, and the supernatant after centrifugation was collected.

· Measurement of lung virus titer

The lung homogenate solution was diluted in a culture medium (MEM, 2% FBS, penicillin-streptomycin) to prepare a 10-fold dilution series, and then mixed with VeroE6/TMPRSS2 cells (JCRB1819, $1.5 \times 10^4$ cells/well), and the cells were seeded in a 96-well plate. After cultured in a $CO_2$ incubator for 4 days, Cytopathic effect (CPE) was observed, and the virus titer contained in the lung homogenate solution was calculated.

· Statistical analysis

A difference in virus titer in the lung homogenate solution between groups was analyzed by Dunnett's test. The statistical analysis was carried out using statistical analysis software SAS ver. 9.4 for Windows (SAS Institute (Cary, NC)) Adjusted two-tailed P values of less than 0.05 were regarded to have statistical significance.

The compounds of the present invention were tested essentially as described above. Results are shown below.

The group administered with the fumaric acid cocrystal Form I crystal of the compound represented by Formula (I-B) shows a decrease in lung virus titer 1 day after infection in a dose-dependent manner even in a single-dose administration or in administration of twice a day and shows a significantly lower virus titer than in the group administered with 0.5% MC at the entire dose of 2 mg/kg or more (Fig. 5A and Fig. 5B). The lung virus titer reached approximately lower limit of quantitation (1.80-$log_{10}TCID_{50}$/mL) in the group administered at 32 and 64 mg/kg in a single-dose administration and the group administered at 16 and 32 mg/kg in administration of twice a day.

Test Example 4: Lung virus titer increase suppression test of SARS-CoV-2-infected mice by delayed administration of fumaric acid cocrystal Form I crystal of compound represented by Formula (I-B)

<Material and method>

· Compound

The compound of the present invention (fumaric acid cocrystal Form I crystal of the compound represented by Formula (I-B)) was prepared using a 0.5% methylcellulose (0.5% MC) solution. The administration volume was set to

10 mL/kg. The dose indicates an amount calculated as a free form.

· Virus

The SARS-CoV-2 hCoV19/Japan/TY7-501/2021 strain separated in National Institute of Infectious Diseases was used.

· Murine lung infection, drug administration, lung collection

Specific pathogen-free 5-week-old female BALB/c mice (CLEA Japan, Inc.) were used in this research. Upon virus inoculation, the mice were anesthetized by intramuscular administration of 100 μL of an anesthetic solution containing 0.03 mg/mL of medetomidine hydrochloride, 0.4 mg/mL of midazolam, and 0.5 mg/mL of butorphanol tartrate in PBS. Under anesthesia, the mice were intranasally inoculated with 50 μL of hCoV19/Japan/TY7-501/2021 ($1.00 \times 10^4$ $TCID_{50}$). While the start point was set as being the point 1 or 3 days after virus infection, the fumaric acid cocrystal Form I of the compound represented by Formula (I-B) was orally administered to mice (n = 5/group) at a dose of 30 mg/kg and 150 mg/kg twice a day. To mice for comparison, 0.5% MC was orally administered once a day. The administration of the compound was set to two days from the start of administration. The murine lung was collected 2 days after the start of administration, 2 mL of PBS was added thereto and homogenized, and the supernatant after centrifugation was collected.

· Measurement of lung virus titer

The lung homogenate solution was diluted in a culture medium (MEM, 2% FBS, penicillin-streptomycin) to prepare a 10-fold dilution series, and then mixed with VeroE6/TMPRSS2 cells (JCRB1819, $1.5 \times 10^4$ cells/well), and the cells were seeded in a 96-well plate. After cultured in a $CO_2$ incubator for 4 days, Cytopathic effect (CPE) was observed, and the virus titer contained in the lung homogenate solution was calculated.

The compounds of the present invention were tested essentially as described above. Results are shown below.

In the groups which were started to be administrated from Day 1 of infection, the lung virus titer in the group administered with 0.5% MC 2 days after the start of administration was 6.47-$\log_{10}TCID_{50}$/mL, and the lung virus titers in the groups administered with the fumaric acid cocrystal Form I of the compound represented by Formula (I-B) at a dose of 30 and 150 mg/kg were 4.47-$\log_{10}TCID_{50}$/mL and 2.90-$\log_{10}TCID_{50}$/mL, respectively (Fig. 6A). In the groups which were started to be administrated from Day 3 of infection, the lung virus titer in the group administered with 0.5% MC 2 days after the start of administration was 4.83-$\log_{10}TCID_{50}$/mL, and the lung virus titers in the groups administered with the fumaric acid cocrystal Form I of the compound represented by Formula (I-B) at a dose of 30 and 150 mg/kg were 3.63-$\log_{10}TCID_{50}$/mL and 3.35-$\log_{10}TCID_{50}$/mL, respectively (Fig. 6B).

From the fact that the lung virus titer was lower in the group administered with the fumaric acid cocrystal Form I of the compound represented by Formula (I-B) than in the group administered with 0.5% MC even in both cases 1 or 3 days after infection from the start of administration, it was suggested that the effect of decreasing the lung virus titer is shown even when there is some time between infection and administration.

Test Example 4-2: Lung virus titer increase suppression test of SARS-CoV-2-infected mice by delayed administration of fumaric acid cocrystal Form I crystal of compound represented by Formula (I-B)

<Material and method>

· Compound

The fumaric acid cocrystal Form I crystal of the compound represented by Formula (I-B) was prepared using a 0.5% methylcellulose (0.5% MC) solution. The administration volume was set to 10 mL/kg. The dose indicates an amount calculated as a free form.

· Virus

The SARS-CoV-2 hCoV19/Japan/TY7-501/2021 strain separated in National Institute of Infectious Diseases was used.

· Murine lung infection, drug administration, lung collection

Specific pathogen-free 5-week-old female BALB/c mice (CLEA Japan, Inc.) were used in this research. Upon virus inoculation, the mice were anesthetized by intramuscular administration of 100 μL of an anesthetic solution containing 0.03 mg/mL of medetomidine hydrochloride, 0.4 mg/mL of midazolam, and 0.5 mg/mL of butorphanol tartrate in PBS. Under anesthesia, the mice were intranasally inoculated with 50 μL of hCoV19/Japan/TY7-501/2021 ($1.00 \times 10^4$ $TCID_{50}$). While the start point was set as being the point 1 day after virus infection, the fumaric acid cocrystal Form I of the compound represented by Formula (I-B) was orally administered to mice (n = 5/group) at a dose of 8, 16, 32, and 64 mg/kg three times a day. To mice for comparison, 0.5% MC was orally administered once a day. The administration of the compound was set to two days from the start of administration. The murine lung was collected 2 days after the start of administration, 2 mL of PBS was added thereto and homogenized, and the supernatant after centrifugation was collected.

· Measurement of lung virus titer

The lung homogenate solution was diluted in a culture medium (MEM, 2% FBS, penicillin-streptomycin) to prepare a 10-fold dilution series, and then mixed with VeroE6/TMPRSS2 cells (JCRB1819, $1.5 \times 10^4$ cells/well), and the cells were seeded in a 96-well plate. After cultured in a $CO_2$ incubator for 4 days, Cytopathic effect (CPE) was observed, and the virus titer contained in the lung homogenate solution was calculated.

The compounds of the present invention were tested essentially as described above. Results are shown below.

In the groups which were started to be administrated from Day 1 of infection, the lung virus titer in the group administered with 0.5% MC 2 days after the start of administration was 6.57-$\log_{10}TCID_{50}$/mL, and the lung virus titers in the groups administered with the fumaric acid cocrystal Form I of the compound represented by Formula (I-B) at a dose of 8, 16, 32, and 64 mg/kg were 5.55, 4.66, 2.85, 2.40-$\log_{10}TCID_{50}$/mL, respectively. From the fact that the lung virus titer was lower in the group administered with the fumaric acid cocrystal Form I of the compound represented by Formula (I-B) than in the group administered with 0.5% MC, it was suggested that the effect of decreasing the lung virus titer is shown even when there is some time between infection and administration (Fig. 7).

Test Example 5: Death and body weight decrease suppression test of SARS-CoV-2-infected mice by administration of fumaric acid cocrystal Form I of compound represented by Formula (I-B)

<Material and method>

· Compound

The compound of the present invention (fumaric acid cocrystal Form I crystal of the compound represented by Formula (I-B)) was prepared using a 0.5% methylcellulose (0.5% MC) solution. The administration volume was set to 10 mL/kg. The dose indicates an amount calculated as a free form.

· Virus

The SARS-CoV-2 hCoV19/Japan/TY7-501/2021 strain (TY7-501) separated in National Institute of Infectious Diseases and the SARS-CoV-2 hCoV19/Japan/TY/WK-521/2020 strain mouse-adapted strain MA-P10 separated in Hokkaido University were used.

· Murine lung infection, drug administration, body weight measurement

Specific pathogen-free 15-week-old or 35 to 45-week-old retired female BALB/c mice (CLEA Japan, Inc.) were used in this research. Upon virus inoculation, the mice were anesthetized by intramuscular administration of 100 μL of an anesthetic solution containing 0.03 mg/mL of medetomidine hydrochloride, 0.4 mg/mL of midazolam, and 0.5 mg/mL of butorphanol tartrate in PBS. Under anesthesia, the mice were intranasally inoculated with 50 μL of TY7-501 ($1.00 \times 10^5$ $TCID_{50}$) and MAP10 ($1.00 \times 10^3$ $TCID_{50}$ or $1.00 \times 10^4$ $TCID_{50}$). TY7-501 was used only for 35 to 45-week-old retired mice. The fumaric acid cocrystal Form I of the compound represented by Formula (I-B) was orally administered to mice (n = 4/group) at a dose of 50 mg/kg twice a day starting 1 day after virus infection. To mice for comparison, 0.5% MC was orally administered twice a day. The body weight was monitored once a day, and in the survival rate evaluation, a case where the body weight was less than 80% on the basis of the body weight immediately before infection was regarded as death.

The compounds of the present invention were tested essentially as described above. Results are shown below.

When aged 35 to 45-week-old retired mice were infected with TY7-501 at $1.00 \times 10^5$ $TCID_{50}$, the body weight was decreased 3 days after infection and all cases were dead 5 days after infection. At this time, in the group administered with the fumaric acid cocrystal Form I of compound represented by Formula (I-B) at a dose of 50 mg/kg, all cases observed were alive until Day 7 of infection, and a decrease in body weight was suppressed (Fig. 8A and Fig. 8B). When 15-week-old mice were infected with MA-P10 at $1.00 \times 10^3$ $TCID_{50}$, a decrease in body weight 3 days after infection was recognized, but dead cases were not recognized. On the other hand, when 15-week-old mice were infected with MA-P10 at $1.00 \times 10^4$ $TCID_{50}$, the body weight was decreased 3 days after infection, dead cases were recognized in some individual 4 days after infection, and all cases were dead 6 days after infection. At this time, in the group administered with the fumaric acid cocrystal Form I of compound represented by Formula (I-B) at a dose of 50 mg/kg, all cases observed were alive until 7 days post-infection, and a decrease in body weight was not recognized (Fig. 8C and Fig. 8D).

Next, when aged 35 to 45-week-old retired mice were infected with MA-P10 at $1.00 \times 10^3$ $TCID_{50}$, the body weight was decreased 3 days after infection, dead cases were recognized in some individual 5 days after infection, and all cases were dead 6 days after infection. From these, it is considered that the symptom of the aged 35 to 45-week-old retired mice is likely to be severe even a low infection dose. At this time, in the group administered with the fumaric acid cocrystal Form I of compound represented by Formula (I-B) at a dose of 50 mg/kg, all cases observed were alive until Day 7 of infection, and a decrease in body weight was suppressed (Fig. 8E and Fig. 8F).

From the above results, it was suggested that, in both of the 15-week-old mice and the aged 35 to 45-week-old retired mice, the effects of suppressing death and a decrease in body weight are attained by administration of the fumaric acid cocrystal Form I of compound represented by Formula (I-B).

Comparing young mice, it is known that, in aged mice, similarly to humans, the expression level of ACE2 receptors known as SARS-CoV-2 receptors is increased (reference literatures: Scientific Reports (2020) 10:22401 and Molecular Therapy Methods & Clinical Development, Vol. 18, P1-6, 2020), and since the normal immune response protecting the body from infectious sources and eliminating these infectious sources is degraded, it is assumed that the aged mice are dead due to the virus infection. However, as described above, in the SARS-CoV-2-infected mouse model using aged mice, the survival rate of the mice 7 days after infection is 100% by administration of the fumaric acid cocrystal Form I of the compound represented by Formula (I-B). Since the non-clinical test using aged mice is positioned as one of non-clinical evaluation system in which a process of the symptom of high-risk patients having an underlying disease becoming severe is simulated, as the treatment option for patients having a high exacerbation risk, usefulness of the fumaric acid cocrystal Form I of the compound represented by Formula (I-B) is supported.

Furthermore, in the above-described test results, it is suggested that the exacerbation due to the virus infection is suppressed in the group administered with the fumaric acid cocrystal Form I of the compound represented by Formula (I-B), and not only the anti-virus effect of the fumaric acid cocrystal Form I of the compound represented by Formula (I-B) against SARS-CoV-2 but also usefulness as a pharmaceutical preparation for suppressing exacerbation of symptoms of infective disease due to SARS-CoV-2 are supported.

Test Example 6: antiviral effect on SARS-CoV

<Material>

· Maintenance medium (2% FBS in MEM)

The maintenance medium was prepared by adding 10 mL of 8.5% NaHCOs, 20 mL of FBS, and 10 mL of L-Glutamine in 1000 mL of Minimum Essential Medium.

· MTT solution

3-(4,5-dimethyl-2-thiazol)-2,5-diphenyl-2H-tetrazolium bromide was dissolved in PBS to become 5 μg/mL and filtered by a 0.45 μm or 0.22 μm filter.

· Cell lysate (virus inactivation solution)

To 500 mL of isopropanol, 50 mL of Triton X and 4 mL of hydrochloric acid (12 mol/L) were added to prepare a cell lysate.

· Dimethyl sulfoxide (DMSO)
· Plate reader (Thermo Fisher Scientific, Perkin-Elmer Corporation, etc.)

<Operation procedure>

· Diluting and dispensing of test sample

The test sample was preliminarily diluted with DMSO to an appropriate concentration, and a 3-fold serial dilution series was prepared. Further, the test sample was diluted in a maintenance medium and dispensed into a 96-well plate so as to become 50 μL/well.

· Diluting and dispensing of cells and virus

Each of viruses (SARS-CoV Hanoi strain (1000 $TCID_{50}$/well) was diluted in a maintenance medium and dispensed for each 50 μL/well into a 96-well plate containing the test sample.
VeroE6/TMPRSS2 cells ($1.5 \times 10^4$ cells/well) prepared in the maintenance medium were dispensed for each 100 μL/well into a 96-well plate containing the test sample and viruses. The resultant product was admixed with a plate mixer and cultured in a $CO_2$ incubator for 3 days.

· Dispensing of MTT solution and cell lysate

The 96-well plate cultured for 3 days was observed by the naked eye with a microscope and the form of cells, the existence of the crystal, etc. were checked. The MTT solution was dispensed for each 30 μL into each well, and culturing was carried out in a $CO_2$ incubator for 4 to 6 hours. The supernatant was removed for each 150 μL such that cells were not suctioned from the plate. The cell lysate (virus inactivation solution) was dispensed for each 150 μL into each well. The plate was covered with plastic wrap so as not to be dried, and left to stand at room temperature overnight.

· Measurement of absorbance (96-well plate)

The plate was admixed the next day with a plate mixer. In the 96-well plate, the absorbance at two wavelengths of 570 nm and 630 nm were measured using a plate reader.

<Calculation of each measurement item>

· Calculation of 50% virus infected cell death inhibitory concentration ($EC_{50}$)

When x was taken as the logarithmic value of the compound concentration and y as %Efficacy, the inhibition curve was approximated by the following Logistic regression equation, and the value of x when substituting 50 (%) for y was calculated as $EC_{50}$.

$$y = \min + (\max - \min)/\{1 + (X50/x)\,\hat{}\,Hill\}$$

$$\%Efficacy = \{(Sample - virus\ control)/(cell\ control - virus\ control)\} * 100\%$$

Sample: the average of ODs of Sample wells
cell control: the average of ODs of cell control wells
virus control: the average of ODs of virus control wells
OD:OD(570 nm)-OD(630 nm)
min; lower limit of y-axis, max; upper limit of y-axis, X50; x-coordinate of inflection point, Hill; slope of curve at midpoint between min and max

The compounds of the present invention were tested essentially as described above. $EC_{50}$ is shown below.
Fumaric acid cocrystal Form I of the compound represented by Formula (I-B): SARS-CoV 0.209 μM

Test Example 7: antiviral effect on HCoV-OC43

<Operation procedure>

· Diluting and dispensing of test sample

The test sample is preliminarily diluted with DMSO to an appropriate concentration, and a 3-fold serial dilution series is prepared and then dispensed into a 96-well plate and diluted in a maintenance medium (MEM, 2% FBS, penicillin-streptomycin).

· Diluting and dispensing of cells and HCoV-OC43

MRC-5 cells ($2 \times 10^4$ cells/well) suspended in a subculture medium (DMEM, 10% FBS, penicillin-streptomycin) are seeded in a 96-well plate a day before infection, and the next day, HCoV-OC43 (100 $TCID_{50}$/well) suspended in a maintenance medium (MEM, 2% FBS, penicillin-streptomycin) is infected for 1 hour. Thereafter, washing is carried out once in the maintenance medium, a maintenance medium including a test reagent is added, and culturing is carried out in a $CO_2$ incubator for 42 hours. Furthermore, in order to examine cytotoxicity of the test reagent, the same operation is performed in the absence of virus.

· Quantitative determination of virus amount

The supernatant on the plate cultured for 42 hours is collected and RNA is extracted using Quick-RNA Viral Kit (ZYMO RESEARCH, #R1041). The extracted RNA solution is quantitatively determined by real-time PCR (Applied BioSystems QuantStudio 3). Regarding the primer, literature (Journal of Clinical Microbiology., 2005, Jun 43(11), 5452-5456) is referred to.

<Calculation of each measurement item>

· Calculation of 90% HCoV-OC43 viral replication inhibitory concentration ($EC_{90}$)

In a concentration-dependent curve in which x is taken as the logarithmic value of the compound concentration and y as the number of copies of virus (Log copies/mL), a value corresponding to 1 log reduction from virus control is calculated by the two-point method from the number of copies in two concentrations therearound. That is,

X = log10 (high conc.)
x = log10 (low conc.)

$$Y = \log10\ (\text{low copies/mL}) - \log10\ (\text{control copies /mL})$$

$$y = \log10\ (\text{high copies /mL}) - \log10\ (\text{control copies /mL})$$

$EC_{90}$ value was calculated by the following formula.

$$EC_{90} = 10\ [x + (-1 - y) \times (X - x)/(Y - y)]$$

The compounds of the present invention were tested essentially as described above. Results are shown below.

Fumaric acid cocrystal Form I of the compound represented by Formula (I-B): $EC_{90}$ 0.074 μM

Test Example 8: antiviral effect on HCoV-229E

<Operation procedure>

· Diluting and dispensing of test sample

The test sample is preliminarily diluted with DMSO to an appropriate concentration, and a 3-fold serial dilution

series is prepared and then dispensed into a 96-well plate and diluted in a maintenance medium (MEM, 2% FBS, penicillin-streptomycin).

· Diluting and dispensing of cells and HCoV-229E

MRC-5 cells ($2 \times 10^4$ cells/well) suspended in a subculture medium (DMEM, 10% FBS, penicillin-streptomycin) are seeded in a 96-well plate a day before infection, and the next day, HCoV-229E (1000 $TCID_{50}$/well) suspended in a maintenance medium (MEM, 2% FBS, penicillin-streptomycin) is infected for 1 hour. Thereafter, virus fluid is removed, a maintenance medium including a test reagent is added, and culturing is carried out in a $CO_2$ incubator for 72 hours. Furthermore, in order to examine cytotoxicity of the test reagent, the same operation is performed in the absence of virus.

· Dispensing and measuring of luminescence signal of CellTiter-Glo (registered trademark) 2.0

After returning the plate cultured for 72 hours to room temperature, CellTiter-Glo (registered trademark) 2.0 is dispensed into each well and mixed with a plate mixer. After a certain time interval, the luminescence signal (Lum) is measured with a plate reader.

<Calculation of each measurement item>

· Calculation of 50% HCoV-229E infected cell death inhibitory concentration ($EC_{50}$)

When x is taken as the logarithmic value of the compound concentration and y as %Efficacy, the inhibition curve is approximated by the following Logistic regression equation, and the value of x when substituting 50 (%) for y is calculated as $EC_{50}$.

$$y = \min + (\max - \min)/\{1 + (X50/x)\ \hat{}\ Hill\}$$

$$\%Efficacy = \{(Sample - virus\ control)/(cell\ control - virus\ control)\} * 100\%$$

cell control: the average of Lum of cell control wells
virus control: the average of Lum of virus control wells
min: lower limit of y-axis, max: upper limit of y-axis, X50: x-coordinate of inflection point, Hill: slope of curve at midpoint between min and max

· Calculation of 50% cytotoxicity concentration

When x is taken as the logarithmic value of the compound concentration and y as %Cytotoxicity, the inhibition curve is approximated by the following Logistic regression equation, and the value of x when substituting 50 (%) for y is calculated as $CC_{50}$.

$$y = \min + (\max - \min)/\{1 + (X50/x)\ \hat{}\ Hill\}$$

% Cytotoxicity = {(Sample - medium control)/(cell control - medium control)} * 100%

cell control: the average of Lum of cell control wells
medium control: the average of Lum of medium control wells
min: lower limit of y-axis, max: upper limit of y-axis, X50: x-coordinate of inflection point, Hill: slope of curve at midpoint between min and max

The compounds of the present invention were tested essentially as described above. Results are shown below.
Fumaric acid cocrystal Form I of the compound represented by Formula (I-B): $EC_{50}$ 5.53 μM, $CC_{50}$ > 200 μM

Test Example 9: Influence of human serum and mouse serum on anti-SARS-CoV-2 activity

<Operation procedure>

· Diluting and dispensing of test sample

The test sample is preliminarily diluted with DMSO to an appropriate concentration, and a 3-fold serial dilution series is prepared and then dispensed into a 96-well plate.

· Addition of serum-supplemented medium

A culture medium (MEM, 2% FBS, penicillin-streptomycin) is adjusted so as to have 0, 6.25%, 12.5, 25, 50% human serum or 0, 3.125, 6.25, 12.5, 25% mouse serum, dispensed into a well containing test sample, and incubated at room temperature for 1 hour.

· Diluting and dispensing of cells and SARS-CoV-2

VeroE6/TMPRSS2 cells (JCRB1819, 1.5 $\times$ $10^3$ cells/well) and SARS-CoV-2 hCoV-19/Japan/TY7-501/2021 (1000 $TCID_{50}$/well in the human serum, 10000 $TCID_{50}$/well in the mouse serum) are mixed in a culture medium (MEM, 2% FBS, penicillin-streptomycin), dispensed in equal amount into a well containing the test sample, the human serum, and the mouse serum, and then cultured in a $CO_2$ incubator for 3 days.

· Dispensing and measuring of luminescence signal of CellTiter-Glo (registered trademark) 2.0

After returning the plate cultured for 3 days to room temperature, CellTiter-Glo (registered trademark) 2.0 is dispensed into each well and mixed with a plate mixer. After a certain time interval, the luminescence signal (Lum) is measured with a plate reader.

<Calculation of each measurement item>

· Calculation of 50% SARS-CoV-2 infected cell death inhibitory concentration ($EC_{50}$)

When x is taken as the logarithmic value of the compound concentration and y as %Efficacy, the inhibition curve is approximated by the following Logistic regression equation, and the value of x when substituting 50 (%) for y is calculated as $EC_{50}$.

$$y = \min + (\max - \min)/\{1 + (X50/x)\ \text{^}\mathrm{Hill}\}$$

$$\%\mathrm{Efficacy} = \{(\mathrm{Sample} - \mathrm{virus\ control})/(\mathrm{cell\ control} - \mathrm{virus\ control})\} * 100\%$$

cell control: the average of Lum of cell control wells

virus control: the average of Lum of virus control wells

min: lower limit of y-axis, max: upper limit of y-axis, X50: x-coordinate of inflection point, Hill: slope of curve at midpoint between min and max

· Calculation of Protein-Adjusted $EC_{50}$ (PA-$EC_{50}$)

After $EC_{50}$ at each serum concentration was calculated, the PA-$EC_{50}$ value was calculated under the serum 100% condition by linear regression.

The compounds of the present invention were tested essentially as described above. Results are shown below.

Fumaric acid cocrystal Form I of the compound represented by Formula (I-B): human serum PA-$EC_{50}$ 3.02 $\mu$M, mouse serum PA-$EC_{50}$ 3.93 $\mu$M

Test Example 10-1:Cytopathic effect (CPE) suppression effect confirmation test using HEK293T cells expressing human ACE2, TMPRSS2 (HEK293T/ACE2-TMPRSS2 cells)

<Operation procedure>

· Diluting and dispensing of test sample

The test sample is preliminarily diluted with DMSO to an appropriate concentration, and a 3-fold serial dilution series is prepared and then dispensed into a 96-well plate.

· Diluting and dispensing of cells and SARS-CoV-2

HEK293T/ACE2-TMPRSS2 cells (SL222, 1.5 $\times$ $10^4$ cells/well) and SARS-CoV-2 hCoV-19/Japan/TY/WK-521/2020, hCoV-19/Japan/QK002/2020, hCoV-19/Japan/TY7-501/2021, hCoV-19/Japan/TY8-612/2021, hCoV-19/Japan/TY11-927-P1/2021 (3000-9000 $TCID_{50}$/well) are mixed in a culture medium (MEM, 2% FBS, penicillin-streptomycin), dispensed into a well containing the test sample, and then cultured in a $CO_2$ incubator for 3 days.

· Dispensing and measuring of luminescence signal of CellTiter-Glo (registered trademark) 2.0

After returning the plate cultured for 3 days to room temperature, CellTiter-Glo (registered trademark) 2.0 is dispensed into each well and mixed with a plate mixer. After a certain time interval, the luminescence signal (Lum) is measured with a plate reader.

<Calculation of each measurement item>

· Calculation of 50% SARS-CoV-2 infected cell death inhibitory concentration ($EC_{50}$)

When x is taken as the logarithmic value of the compound concentration and y as %Efficacy, the inhibition curve is approximated by the following Logistic regression equation, and the value of x when substituting 50 (%) for y is calculated as $EC_{50}$.

$$y = \min + (\max - \min)/\{1 + (X50/x)\ \hat{}\,\mathrm{Hill}\}$$

$$\%\mathrm{Efficacy} = \{(\mathrm{Sample} - \mathrm{virus\ control})/(\mathrm{cell\ control} - \mathrm{virus\ control})\} * 100\%$$

cell control: the average of Lum of cell control wells
virus control: the average of Lum of virus control wells
min: lower limit of y-axis, max: upper limit of y-axis, X50: x-coordinate of inflection point, Hill: slope of curve at midpoint between min and max

The compounds of the present invention were tested essentially as described above. Results are shown below.

Fumaric acid cocrystal Form I crystal of the compound represented by Formula (I-B): 0.026 to 0.058 pM

Test Example 10-2:Cytopathic effect (CPE) suppression effect confirmation test using HEK293T cells expressing human ACE2, TMPRSS2 (HEK293T/ACE2-TMPRSS2 cells)

<Operation procedure>

· Diluting and dispensing of test sample

The test sample is preliminarily diluted with DMSO to an appropriate concentration, and a 3-fold serial dilution series is prepared and then dispensed into a 96-well plate.

· Diluting and dispensing of cells and SARS-CoV-2

HEK293T/ACE2-TMPRSS2 cells (SL222, 1.5 × $10^4$ cells/well) and SARS-CoV-2 hCoV-19/Japan/TY38-873/2021 (9000 $TCID_{50}$/well) are mixed in a medium (MEM, 2% FBS, penicillin-streptomycin), dispensed into a well containing the test sample, and then cultured in a $CO_2$ incubator for 3 days.

· Dispensing and measuring of luminescence signal of CellTiter-Glo (registered trademark) 2.0

After returning the plate cultured for 3 days to room temperature, CellTiter-Glo (registered trademark) 2.0 is dispensed into each well and mixed with a plate mixer. After a certain time interval, the luminescence signal (Lum) is measured with a plate reader.

<Calculation of each measurement item>

· Calculation of 50% SARS-CoV-2 infected cell death inhibitory concentration ($EC_{50}$)

When x is taken as the logarithmic value of the compound concentration and y as %Efficacy, the inhibition curve is approximated by the following Logistic regression equation, and the value of x when substituting 50 (%) for y is calculated as $EC_{50}$.

$$y = min + (max - min)/\{1 + (X50/x)\ \text{^}Hill\}$$

$$\%Efficacy = \{(Sample - virus\ control)/(cell\ control - virus\ control)\} * 100\%$$

cell control: the average of Lum of cell control wells
virus control: the average of Lum of virus control wells
min: lower limit of y-axis, max: upper limit of y-axis, X50: x-coordinate of inflection point, Hill: slope of curve at midpoint between min and max

The compounds of the present invention were tested essentially as described above. Results are shown below.
Fumaric acid cocrystal Form I crystal of the compound represented by Formula (I-B): 0.083 μM

Test Example 11: Test for suppression of viral spread from SARS-CoV-2-infected animal to uninfected animal by preventive administration of fumaric acid cocrystal Form I crystal of compound represented by Formula (I-B)

<Material and method>

· Compound

The fumaric acid cocrystal Form I crystal of the compound represented by Formula (I-B) was prepared using a 0.5% methylcellulose (0.5% MC) solution. The administration volume was set to 10 mL/kg. The dose indicates an amount calculated as a free form.

· Virus

The SARS-CoV-2 hCoVl9/Japan/TY11-927/2021 strain separated in National Institute of Infectious Diseases was used.

· Hamster lung infection, drug administration, lung collection

Specific pathogen-free 5-week-old male Syrian hamsters (Japan SLC, Inc.) were used in this research. One infected hamster inoculated with virus and three administered hamsters not inoculated with virus were reared in the same cage, and viral spread to the administered hamsters living together with the infected hamster was tested. Upon virus inoculation, the hamsters were anesthetized by inhalation administration of isoflurane. Under anesthesia, the hamsters were intranasally inoculated with 200 μL of hCoV19/Japan/TY11-927/2021 (5.00 × $10^3$ PFU). While the start point was set at virus inoculation to the infected hamster, the fumaric acid cocrystal Form I crystal of the compound

represented by Formula (I-B) was orally administered to the administered hamsters not inoculated with virus at a dose of 30 and 200 mg/kg twice a day. To hamsters for comparison, 0.5% MC was orally administered twice a day. The administration of the compound was set to six days from the start of administration. The hamster lung was collected 6 days after the start of administration, 5 mL of PBS was added thereto and homogenized, and the supernatant after centrifugation was collected.

· Measurement of lung virus titer

The lung homogenate solution was diluted in a culture medium (MEM, 2% FBS, penicillin-streptomycin) to prepare a 10-fold dilution series, and then the cells were seeded in VeroE6/TMPRSS2 cells (JCRB1819, 2.0 $\times$ $10^5$ cells/well) previously cultured in a 24-well plate. After cultured in a $CO_2$ incubator for 2 days, a viral plaque was observed, and the virus titer contained in the lung homogenate solution was calculated.

The compounds of the present invention were tested essentially as described above. Results are shown below.

Six days after the start of living together with the infected hamster, the lung virus titers of the administered hamsters in the group administered with 0.5% MC were 6.12-$\log_{10}$PFU/mL, and the lung virus titers in the groups administered with the fumaric acid cocrystal Form I crystal of the compound represented by Formula (I-B) at a dose of 30 and 200 mg/kg were 3.48-$\log_{10}$$PFU_{50}$/mL, and a detection limit value or less, respectively (Fig. 9). From the fact that the lung virus titer was lower in the group administered with the fumaric acid cocrystal Form I crystal of the compound represented by Formula (I-B) than in the group administered with 0.5% MC, it was suggested that the effect of decreasing viral spread from the infected hamster is shown.

Test Example 12: Test for suppression of viral spread from SARS-CoV-2-infected animal to uninfected animal by administration of fumaric acid cocrystal Form I crystal of compound represented by Formula (I-B) immediately after infection

<Material and method>

· Compound

The fumaric acid cocrystal Form I crystal of the compound represented by Formula (I-B) was prepared using a 0.5% methylcellulose (0.5% MC) solution. The administration volume was set to 10 mL/kg. The dose indicates an amount calculated as a free form.

· Virus

The SARS-CoV-2 hCoV19/Japan/TY11-927/2021 strain separated in National Institute of Infectious Diseases was used.

· Hamster lung infection, drug administration, lung collection

Specific pathogen-free 5-week-old male Syrian hamsters (Japan SLC, Inc.) were used in this research. One infected hamster inoculated with virus and two uninfected hamsters not inoculated with virus were reared in the same cage, and viral spread to the uninfected hamsters living together with the infected hamster was tested. Upon virus inoculation, the hamsters were anesthetized by inhalation administration of isoflurane and intranasally inoculated with 200 $\mu$L of hCoV-19/Japan/TY11-927/2021 (5.00 $\times$ $10^3$ PFU). While the start point was set at virus inoculation, the fumaric acid cocrystal Form I crystal of the compound represented by Formula (I-B) was orally administered to the infected hamster at a dose of 200 mg/kg twice a day. To the infected hamster for comparison, 0.5% MC was orally administered twice a day. The administration of the compound was set to four days from the start of administration. The uninfected hamster lungs were collected 6 days after infection, 5 mL of PBS was added thereto and homogenized, and the supernatant after centrifugation was collected.

· Measurement of lung virus titer

The lung homogenate solution was diluted in a culture medium (MEM, 2% FBS, penicillin-streptomycin) to prepare a 10-fold dilution series, and then the cells were seeded in VeroE6/TMPRSS2 cells (JCRB1819, 2.0 $\times$ $10^5$ cells/well) previously cultured in a 24-well plate. After cultured in a $CO_2$ incubator for 2 days, a viral plaque was observed, and the virus titer contained in the lung homogenate solution was calculated.

The compounds of the present invention were tested essentially as described above. Results are shown below.

Six days after the start of living together with the infected hamster, the lung virus titers of the uninfected hamsters

in the group administered with 0.5% MC were 6.36-$log_{10}$PFU/mL, and the lung virus titer in the group administered with the fumaric acid cocrystal Form I crystal of the compound represented by Formula (I-B) at a dose of 200 mg/kg was the detection limit value or less (Fig. 10). From the fact that the lung virus titer was lower in the group administered with the fumaric acid cocrystal Form I crystal of the compound represented by Formula (I-B) than in the group administered with 0.5% MC, it was suggested that the effect of decreasing viral spread from the infected hamster is shown.

Test Example 13: Test for suppression of viral spread from SARS-CoV-2-infected animal to uninfected animal by delayed administration of fumaric acid cocrystal Form I crystal of compound represented by Formula (I-B) after infection

<Material and method>

· Compound

The fumaric acid cocrystal Form I crystal of the compound represented by Formula (I-B) was prepared using a 0.5% methylcellulose (0.5% MC) solution. The administration volume was set to 5 mL/kg. The dose indicates an amount calculated as a free form.

· Virus

The SARS-CoV-2 hCoV19/Japan/TY11-927/2021 strain separated in National Institute of Infectious Diseases was used.

· Hamster lung infection, drug administration, lung and nasal concha collection

Specific pathogen-free 6-week-old male Syrian hamsters (Japan SLC, Inc.) were used in this research. One infected hamster inoculated with virus and one uninfected hamster not inoculated with virus were reared in the same cage while the start point was set as being the point 2 days after infection, and viral spread to the uninfected hamster living together with the infected hamster was tested. The test was executed with n = 3 for each group. Upon virus inoculation, the hamster was anesthetized by subcutaneous administration of 200 $\mu$L of an anesthetic solution containing 0.07 mg/mL of medetomidine hydrochloride, 6.98 mg/mL of alfaxalone, and 1.16 mg/mL of butorphanol tartrate, and intranasally inoculated with 100 $\mu$L of hCoV-19/Japan/TY11-927/2021 ($1.00 \times 10^2$ $TCID_{50}$). While the start point was set as being the point 1 day after virus infection, the fumaric acid cocrystal Form I crystal of the compound represented by Formula (I-B) was orally administered to the infected hamster at a dose of 50 mg/kg twice a day. To the infected hamster for comparison, 0.5% MC was orally administered twice a day. The administration of the compound was set to four days from the start of administration. 5 days after infection, infected and uninfected hamster lungs and nasal conchas were collected, 5 mL or 1 mL of PBS was added thereto and homogenized, and the supernatant after centrifugation was collected.

· Measurement of lung and intranasal concha virus titers

The lung or nasal concha homogenate solution was diluted in a culture medium (MEM, 2% FBS, penicillin-streptomycin) to prepare a 10-fold dilution series, and then the cells were seeded in VeroE6/TMPRSS2 cells (JCRB1819, $1.5 \times 10^4$ cells/well) previously cultured in a 96-well plate. After cultured in a $CO_2$ incubator for 5 days, Cytopathic effect (CPE) was observed, and the virus titer contained in the lung or nasal concha homogenate solution was calculated.

The compounds of the present invention were tested essentially as described above. Results are shown below.

Three days after the start of living together with the infected hamster, the lung virus titers of the uninfected hamster in the group administered with 0.5% MC were 5.30-$log_{10}TCID_{50}$/mL, and the lung virus titer in the group administered with the fumaric acid cocrystal Form I crystal of the compound represented by Formula (I-B) at a dose of 50 mg/kg was 2.97-$log_{10}TCID_{50}$/mL (Fig. 11A). Furthermore, 3 days after the start of living together with the infected hamster, the intranasal concha virus titers of the uninfected hamsters in the group administered with 0.5% MC were 6.06-$log_{10}TCID_{50}$/mL, and the intranasal concha virus titer in the group administered with the fumaric acid cocrystal Form I crystal of the compound represented by Formula (I-B) at a dose of 50 mg/kg was 2.89-$log_{10}TCID_{50}$/mL (Fig. 11B). From the fact that the lung and intranasal concha virus titers were lower in the group administered with the fumaric acid cocrystal Form I crystal of the compound represented by Formula (I-B) than in the group administered with 0.5% MC, it was suggested that the effect of decreasing viral spread from the infected hamster is shown.

Test Example 14: Death and body weight decrease suppression test of SARS-CoV-2-infected mice by preventive administration of compound represented by Formula (I-B)

<Material and method>

· Compound

The compound of the present invention (the compound represented by Formula (I-B)) was prepared using a 0.5% methylcellulose (0.5% MC) solution. The administration volume was set to 10 mL/kg $\times$ 2 sites.

· Virus

The SARS-CoV-2 hCoV19/Japan/TY/WK-521/2020 strain mouse-adapted strain MA-P10 separated in Hokkaido University was used.

· Murine lung infection, drug administration, body weight measurement

Specific pathogen-free 37 to 57-week-old retired female BALB/c mice (CLEA Japan, Inc.) were used in this research. Upon virus inoculation, the mice were anesthetized by intramuscular administration of 100 $\mu$L of an anesthetic solution containing 0.03 mg/mL of medetomidine hydrochloride, 0.4 mg/mL of midazolam, and 0.5 mg/mL of butorphanol tartrate in PBS. Under anesthesia, the mice were intranasally inoculated with 50 $\mu$L of MA-P10 ($3.00 \times 10^2$ $TCID_{50}$). One day before virus infection, the compound represented by Formula (I-B) was subcutaneously administered to mice (n = 15/group) at a dose of 32, 64, 96, 128 mg/kg once. To mice for comparison, 0.5% MC was subcutaneously administered once. The body weight was monitored once a day, and in the survival rate evaluation, a case where the body weight was less than 80% on the basis of the body weight immediately before infection was regarded as death.

The compounds of the present invention were tested essentially as described above. Results are shown below.

On the other hand, when the aged 37 to 57-week-old retired mice were infected with MA-P10 at $3.00 \times 10^2$ $TCID_{50}$, the body weight was decreased from the day after infection, dead cases were recognized in some individual4 days after infection, and all cases were dead 8 days after infection. At this time, in the groups administered with the compound represented by Formula (I-B) at a does of 32, 64, 96, and 128 mg/kg, the survival rates until Day 14 of infection were 6.7%, 60%, 80%, and 100%, respectively, and in the groups administered at 32 mg/kg or more, a decrease in body weight was suppressed (Figs. 12A, 12B). Plasma concentrations immediately before infection, that is, 1 day after administration were measured to be 1,740, 2,990, 3,110, and 3,370 $\mu$g/mL, respectively.

From the above results, it was suggested that, in the aged 37 to 57-week-old retired mice, the effects of suppressing death and a decrease in body weight are attained when 64 mg/kg or more of the compound represented by Formula (I-B) was subcutaneously administered one day before infection, and the plasma concentration at the time of viral infection was 2,990 $\mu$g/mL or more.

The above test results show that the exacerbation due to the virus infection was suppressed in the preventive subcutaneous administration of the compound represented by Formula (I-B), and usefulness of the compound represented by Formula (I-B) as a preventive agent for infective disease due to SARS-CoV-2 is supported.

Test Example 15: Test for confirming virus proliferation suppression effect using human airway epithelial cells cells

<Operation procedure>

· Diluting and dispensing of test sample

The test sample was preliminarily diluted with DMSO to an appropriate concentration, and a 3-fold serial dilution series was prepared, then diluted 200 fold with a MucilAir™ culture solution, and dispensed into a 24-well plate.

· SARS-CoV-2 infection

MucilAir™ (Nasal cavity, about $5.0 \times 10^5$ cells/well) seeded on transwell was infected with SARS-CoV-2 hCoV-19/Japan/TY11-927-P1/2021 and hCoV-19/Japan/TY38-873/2021 (5000 $TCID_{50}$/well), and cultured in a $CO_2$ incubator for 2 hours. Thereafter, the transwell was washed with a MucilAir™ culture solution and placed on the well containing the test sample, and they are cultured in a $CO_2$ incubator. Two and 3 days afterinfection with hCoV-19/Japan/TY11-927-P1/2021, and 1 and 2 days after infection with hCoV-19/Japan/TY38-873/2021, a MucilAir™ culture solution was added to transwell, and the supernatant was collected.

· Measurement of virus titer in supernatant

The collected supernatant was diluted in a culture medium (MEM, 2% FBS, penicillin-streptomycin) to prepare a 10-fold dilution series, and then mixed with VeroE6/TMPRSS2 cells (JCRB1819, 1.5 × $10^4$ cells/well), and the cells were seeded in a 96-well plate. After cultured in a COz incubator for 4 days, Cytopathic effect (CPE) was observed, and the virus titer contained in the supernatant was calculated.

<Calculation of each measurement item>

· Calculation of 90% SARS-CoV-2 virus production inhibitory concentration ($EC_{90}$)

In a concentration-dependent curve in which x is taken as the logarithmic value of the compound concentration and y as the virus titer ($Log_{10}$ $TCID_{50}$/mL), a value corresponding to 1 $log_{10}$ reduction from virus control is calculated by the two-point method from the virus titer in two concentrations therearound.

X = the lowest conc. at the average of reduction from control viral titer of < -1 x = the highest conc. at the average of reduction from control viral titer of ≥ -1 Y = the average of reduction from control viral titer at x y = the average of reduction from control viral titer at X

$EC_{90}$ value were calculated by the following formula.

$$EC_{90} = 10^{[\log(x) + (-1 - Y) \times (\log(x) - \log(X)) / (Y - y)]}$$

The compounds of the present invention were tested essentially as described above. Results are shown below.

[Table 15]

| Virus strain | Days of culture (days) | Fumaric acid cocrystal Form I crystal of compound represented by Formula (I-B) $EC_{90}$ (μM) |
|---|---|---|
| Delta TY11-927-P1 | 2 | 0. 0514 |
| | 3 | 0.117 |
| Omicorn BA.1 TY38-873 | 1 | 0.0660 |
| | 2 | 0.160 |

The following formulation examples are merely examples and not intended to limit the scope of the invention.

The compound according to the present invention can be administered as a pharmaceutical composition in any conventional route, particularly, in an enteral route, for example, orally, for example in the form of a tablet or a capsule, or parenterally, for example, in the form of an injection or a suspension, locally, for example, in the form of a lotion, a gelling agent, an ointment, or a cream, or in the intranasal form or suppository form. The pharmaceutical composition comprising the compound of the present invention in the free form or in the form of a pharmaceutically acceptable salt can be produced together with at least one kind of pharmaceutically acceptable carrier or diluent by a conventional method such as a mixing, granulating, or coating method. For example, as a composition for oral, tablets, granules, and capsules containing excipients, disintegrants, binders, lubricants, etc. and an active ingredient, etc. can be used. Furthermore, as a composition for injection, solutions or suspensions can be used, and sterilization can be carried out, or preservatives, stabilizing agents, buffer agents, and the like may be contained.

[INDUSTRIAL APPLICABILITY]

The compound according to present invention has inhibitory activity against the coronavirus 3CL protease, and the pharmaceutical composition comprising the compound according to the present invention is useful as a therapeutic agent and/or prophylactic agent for coronavirus disease.

[SEQUENCE LISTING]

## Claims

**1.** A pharmaceutical composition comprising a compound represented by Formula (I):

[Chemical Formula 1]

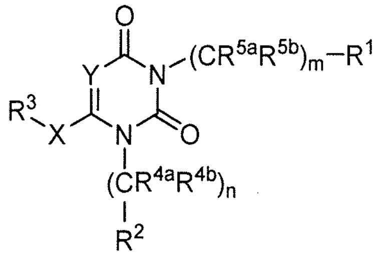

(I)

wherein Y is N;
$R^1$ is substituted or unsubstituted aromatic heterocyclyl;
$R^2$ is substituted or unsubstituted 6-membered aromatic carbocyclyl;
$R^3$ is substituted or unsubstituted aromatic heterocyclyl;
-X- is -NH-;
m is 0 or 1;
$R^{5a}$ is a hydrogen atom;
$R^{5b}$ is a hydrogen atom;
n is 1;
$R^{4a}$ is a hydrogen atom; and
$R^{4b}$ is a hydrogen atom, or a pharmaceutically acceptable salt thereof.

**2.** The pharmaceutical composition according to claim 1, wherein $R^1$ is substituted or unsubstituted 5- to 6-membered aromatic heterocyclyl.

**3.** The pharmaceutical composition according to claim 1 or 2, wherein $R^2$ is 6-membered aromatic carbocyclyl substituted with one, two, or three substituents selected from a substituent group G;
the substituent group G described here is a group consisting of halogen, cyano, and unsubstituted alkyl.

**4.** The pharmaceutical composition according to any one of claims 1 to 3, wherein $R^3$ is substituted or unsubstituted 9- to 10-membered aromatic heterocyclyl.

**5.** A pharmaceutical composition comprising the compound according to claim 1 or a pharmaceutically acceptable salt thereof, wherein the compound represented by Formula (I) is selected from the group consisting of Compound I-003, Compound I-005, Compound I-017, and Compound I-023.

**6.** The pharmaceutical composition according to any one of claims 1 to 5 which is a 3CL protease inhibitor.

**7.** The pharmaceutical composition according to any one of claims 1 to 6 which is used for inhibiting virus proliferation of SARS-CoV-2.

**8.** The pharmaceutical composition according to any one of claims 1 to 7 which is a therapeutic agent and/or prophylactic agent for coronavirus disease 2019 (COVID-19).

**9.** The pharmaceutical composition according to any one of claims 1 to 8 which is used for suppression of exacerbation of infective disease due to SARS-CoV-2.

**10.** The pharmaceutical composition according to any one of claims 1 to 9 which is used such that administration is started within 72 hours from onset of a symptom of infective disease due to SARS-CoV-2 or from SARS-CoV-2 positive diagnosis.

**11.** The pharmaceutical composition according to any one of claims 1 to 9 which is used such that administration is started within 24 hours from onset of a symptom of infective disease due to SARS-CoV-2 or from SARS-CoV-2

positive diagnosis.

**12.** The pharmaceutical composition according to any one of claims 1 to 9 which is used for suppressing viral spread of SARS-CoV-2.

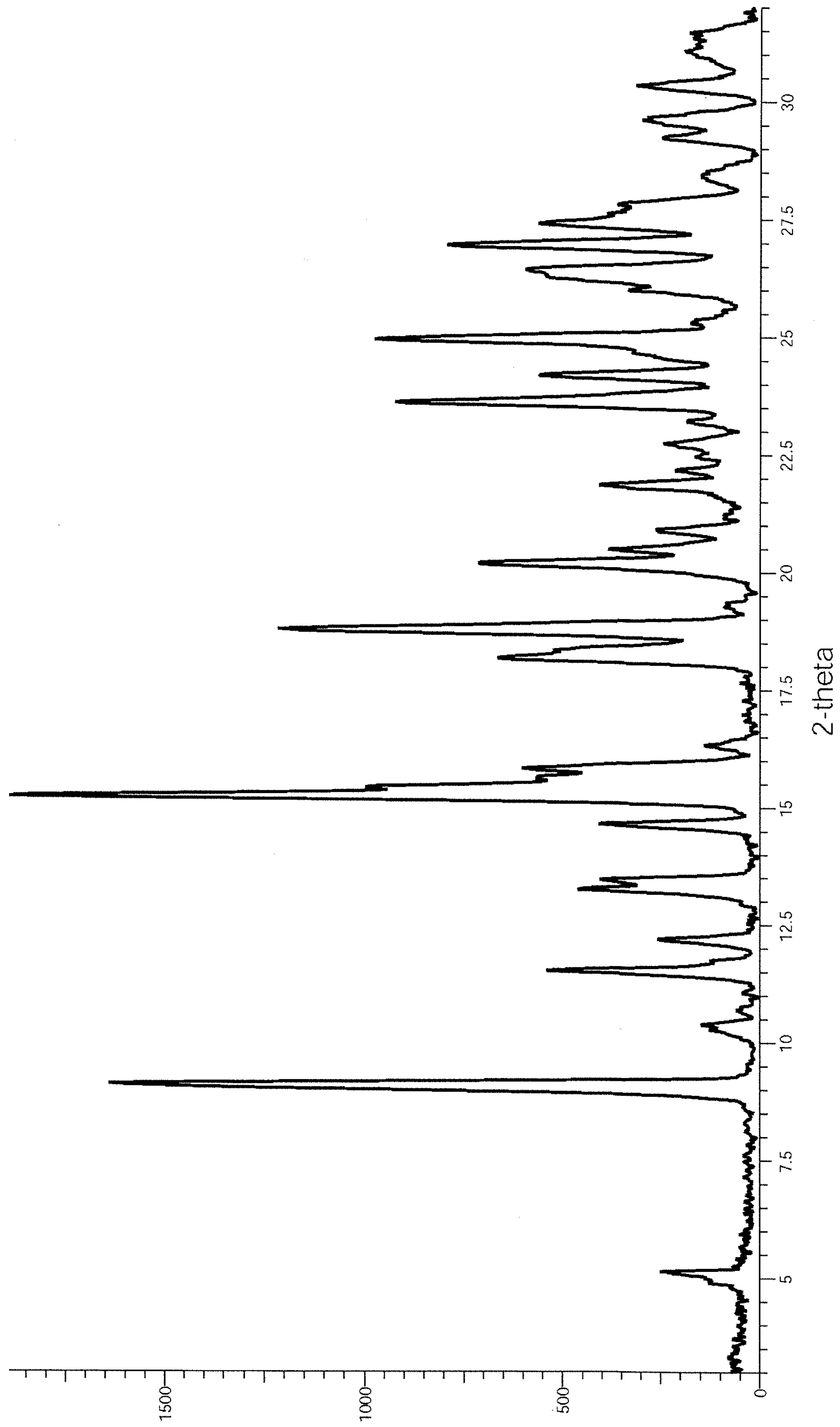


Fig. 1

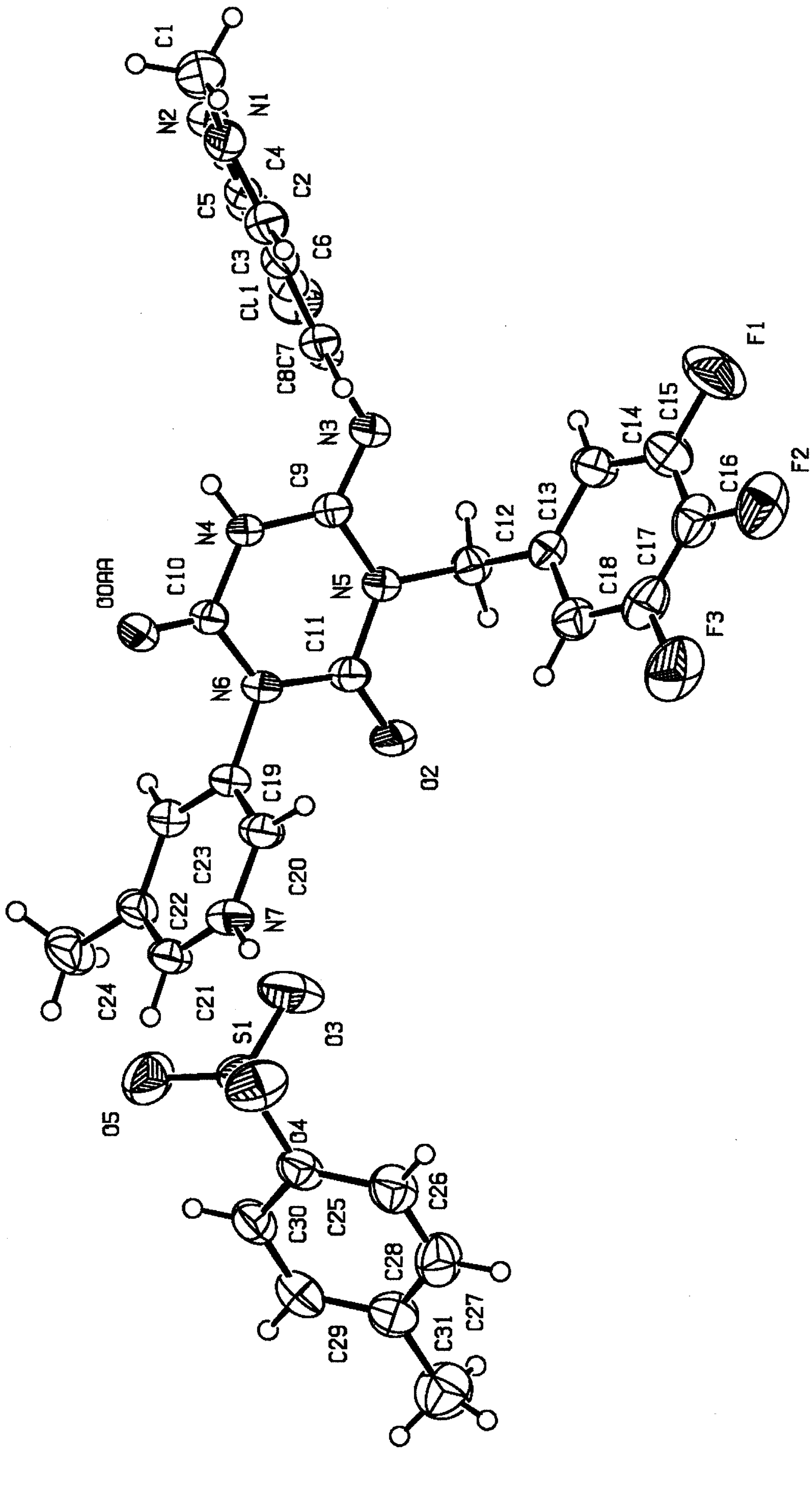

Fig. 2

Fig. 3

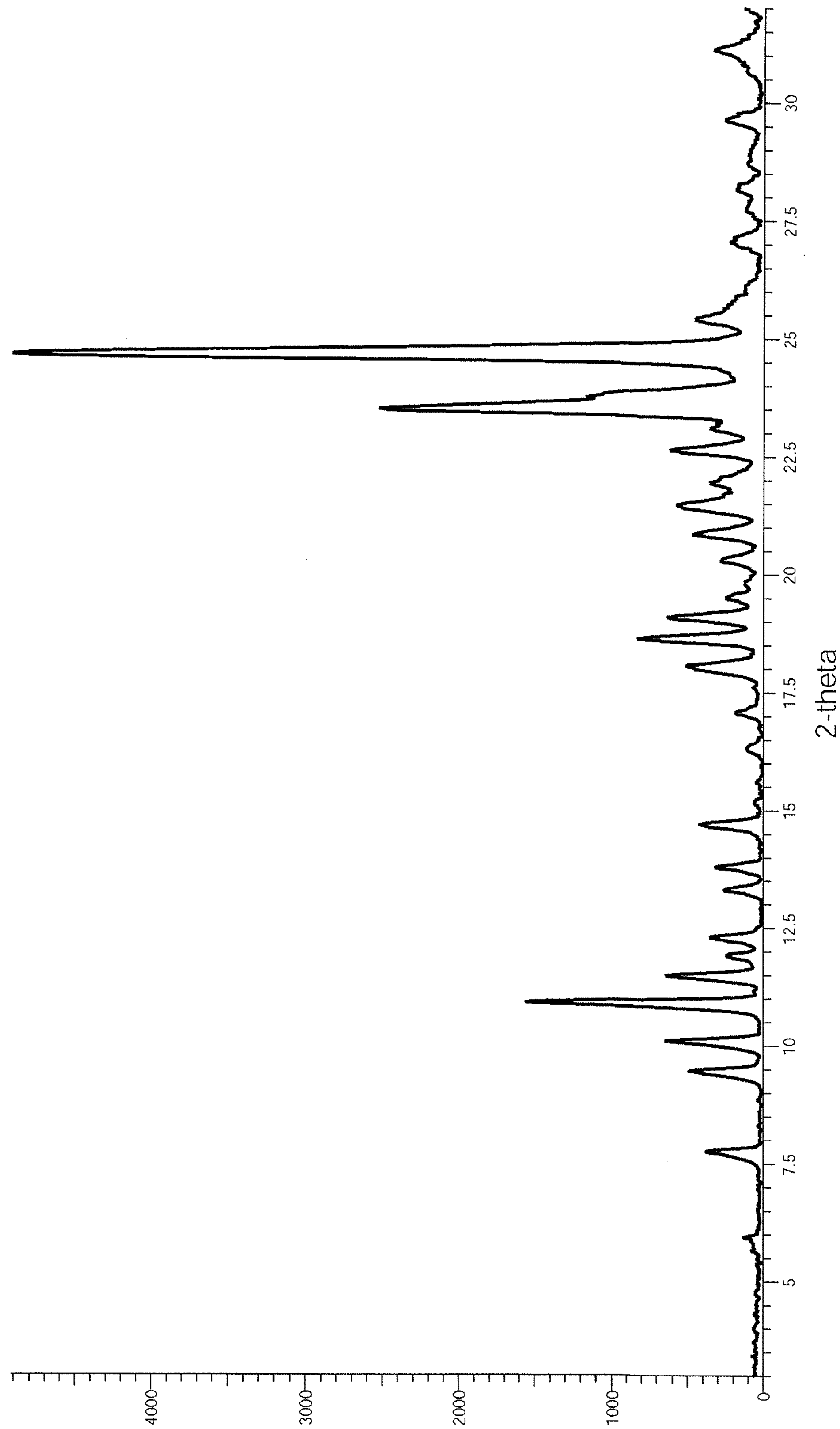

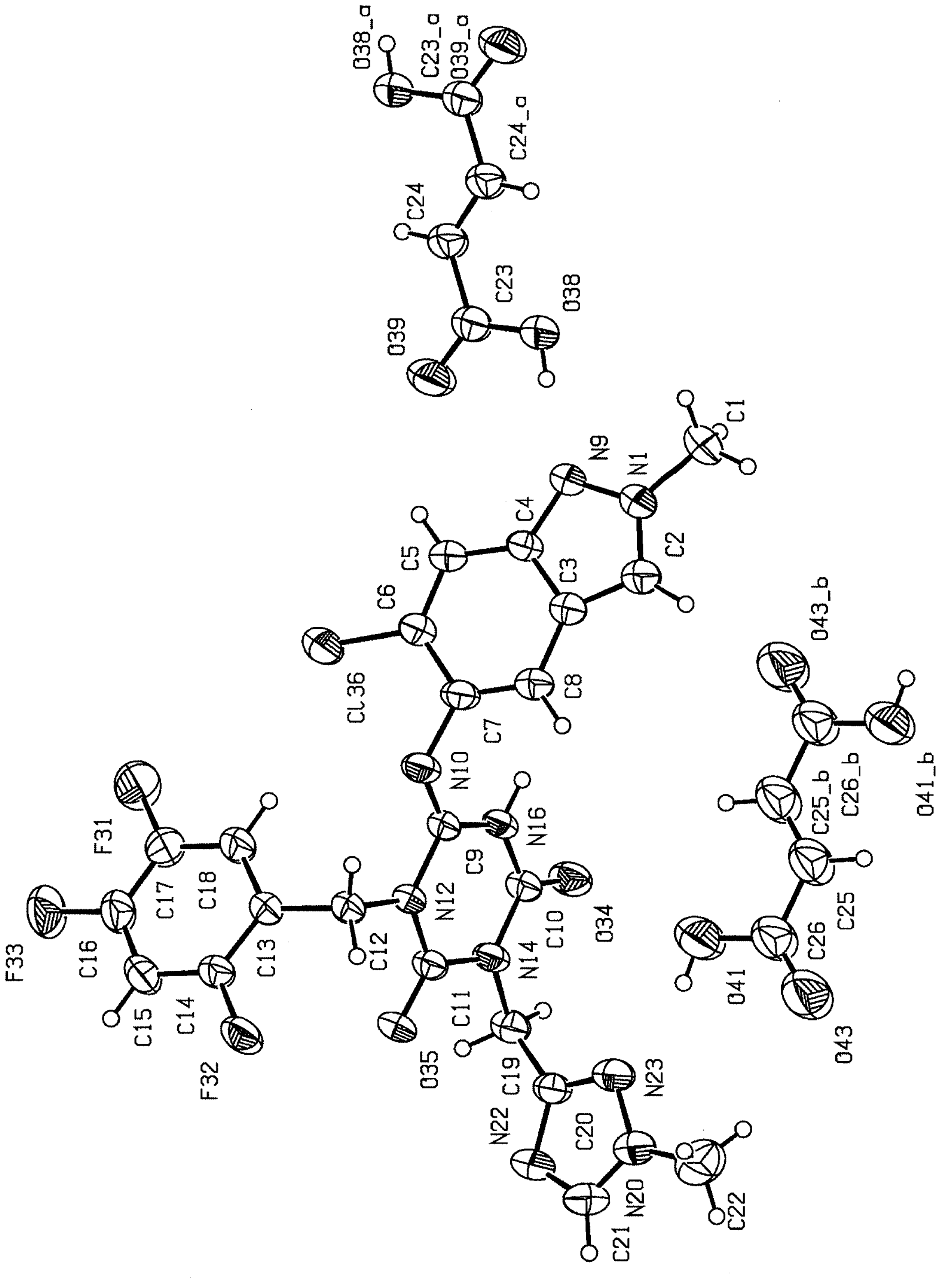

Fig. 4

Fig. 5A

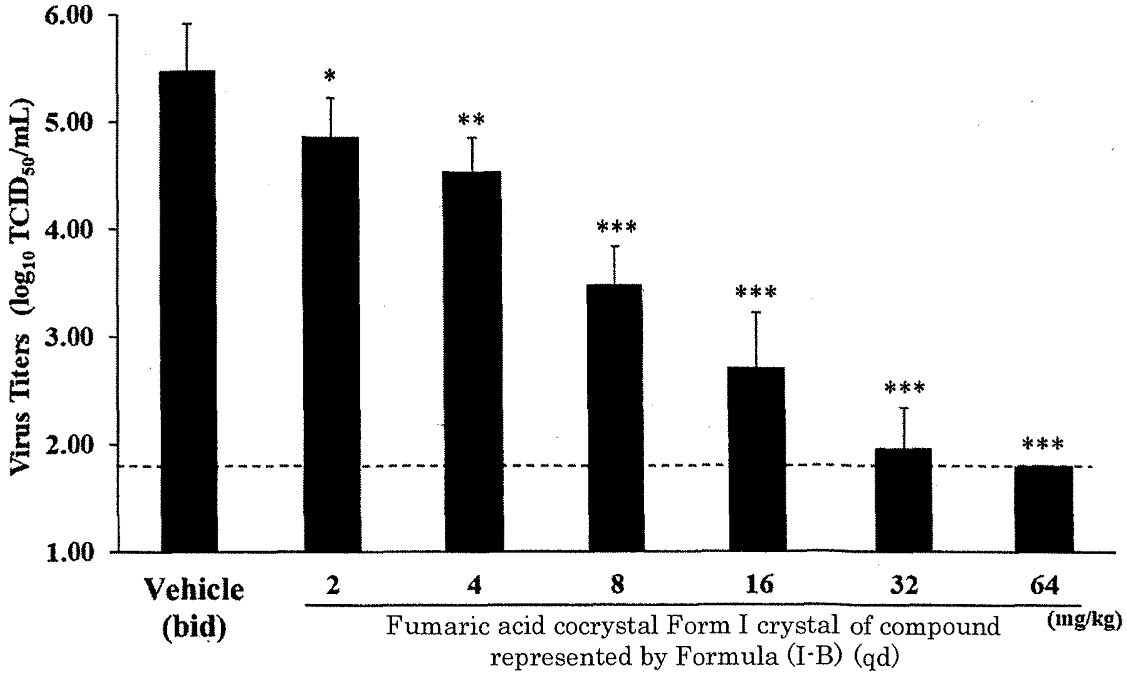


* P<0.05, ** P<0.001, *** P<0.0001 vs vehicle (dunnett's method)

Fig. 5B

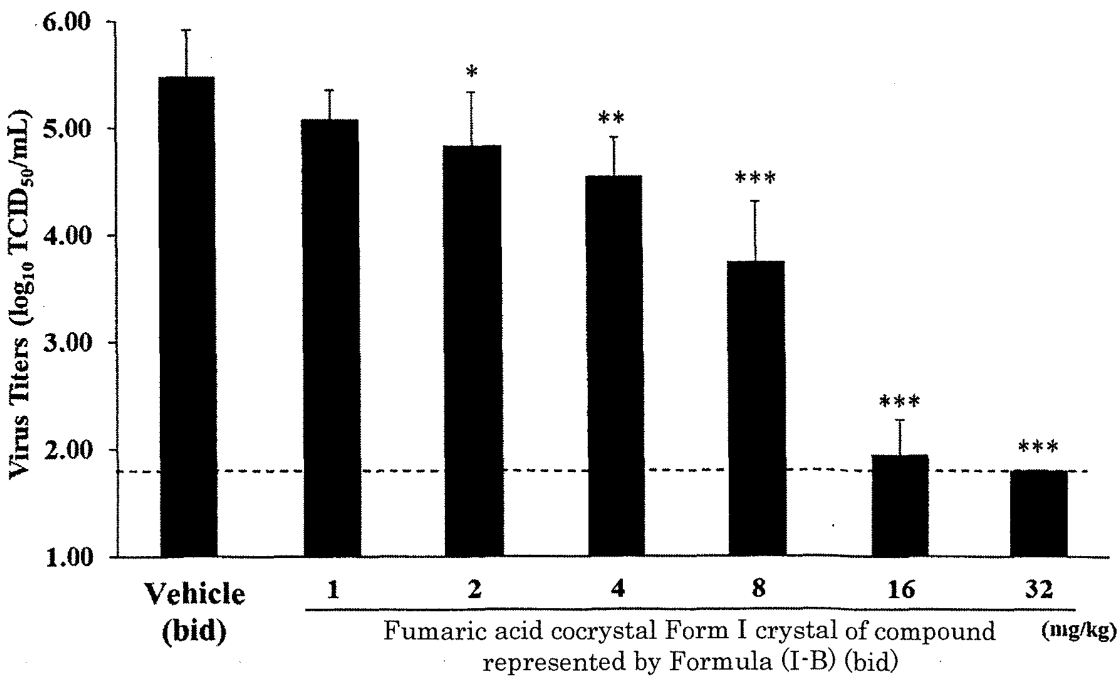


* P<0.05, ** P<0.001, *** P<0.0001 vs vehicle (dunnett's method)

Fig. 6A

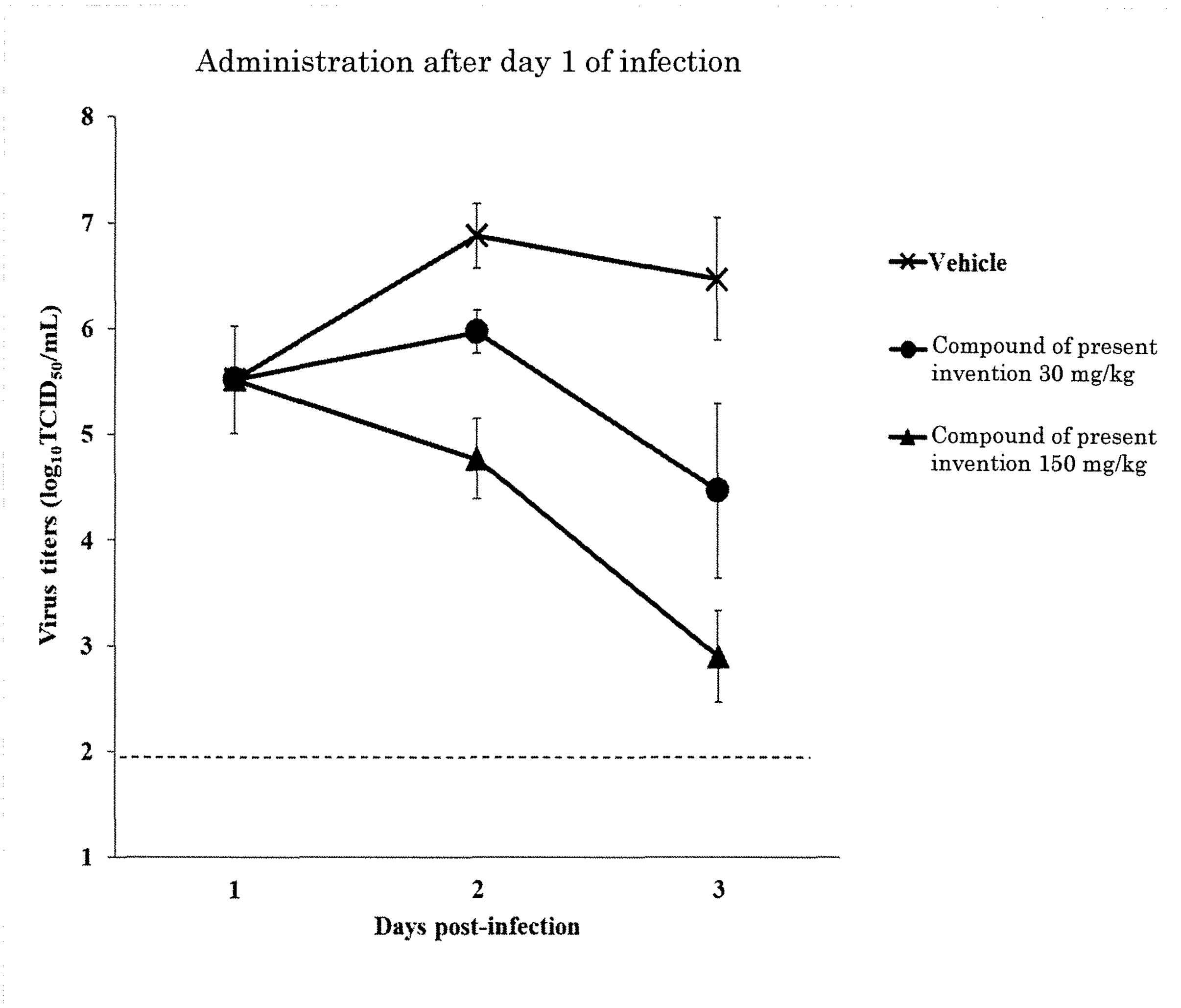
Administration after day 1 of infection
Virus titers ($log_{10}TCID_{50}$/mL)
8
7
6
5
4
3
2
1
1
2
3
Days post-infection
Vehicle
Compound of present invention 30 mg/kg
Compound of present invention 150 mg/kg

Fig. 6B

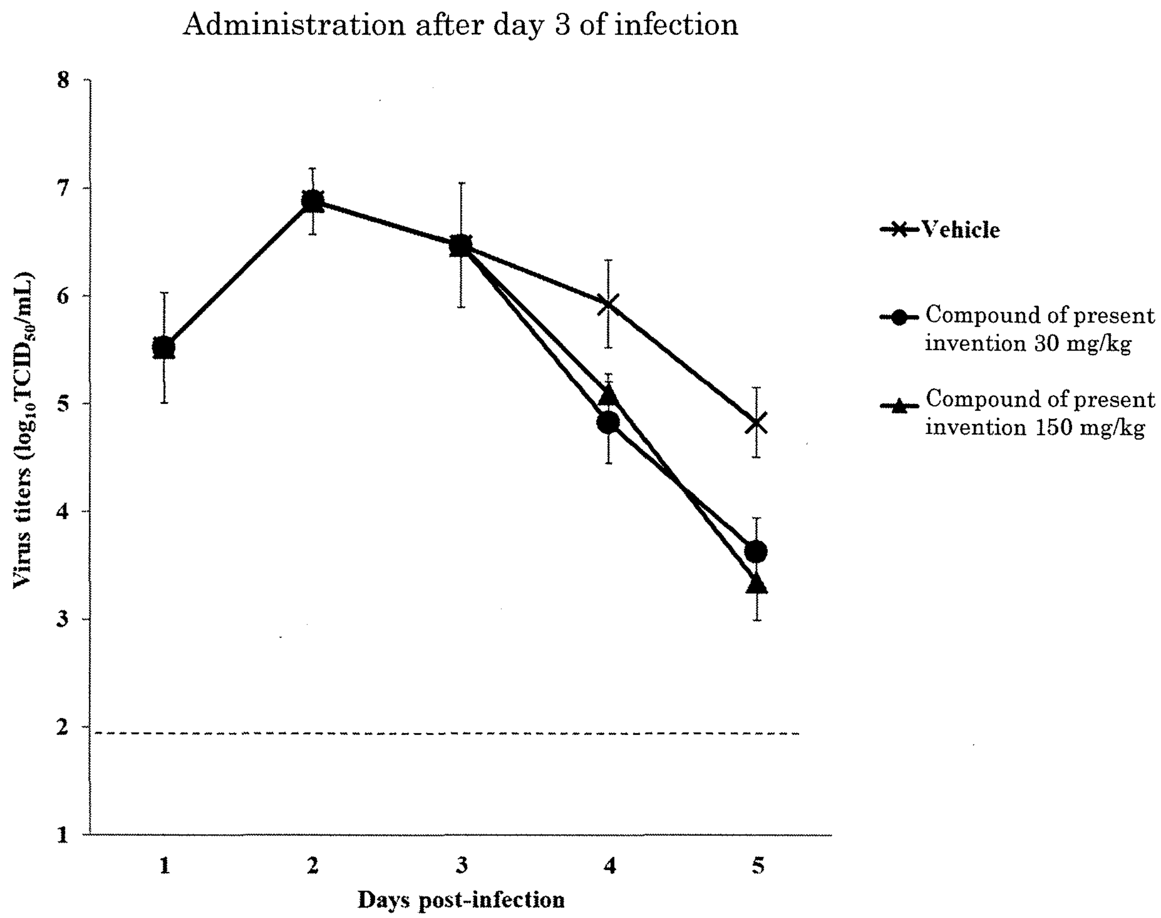
Administration after day 3 of infection
8
7
6
5
4
3
2
1
Virus titers ($log_{10}TCID_{50}/mL$)
1
2
3
4
5
Days post-infection
Vehicle
Compound of present invention 30 mg/kg
Compound of present invention 150 mg/kg

Fig. 7

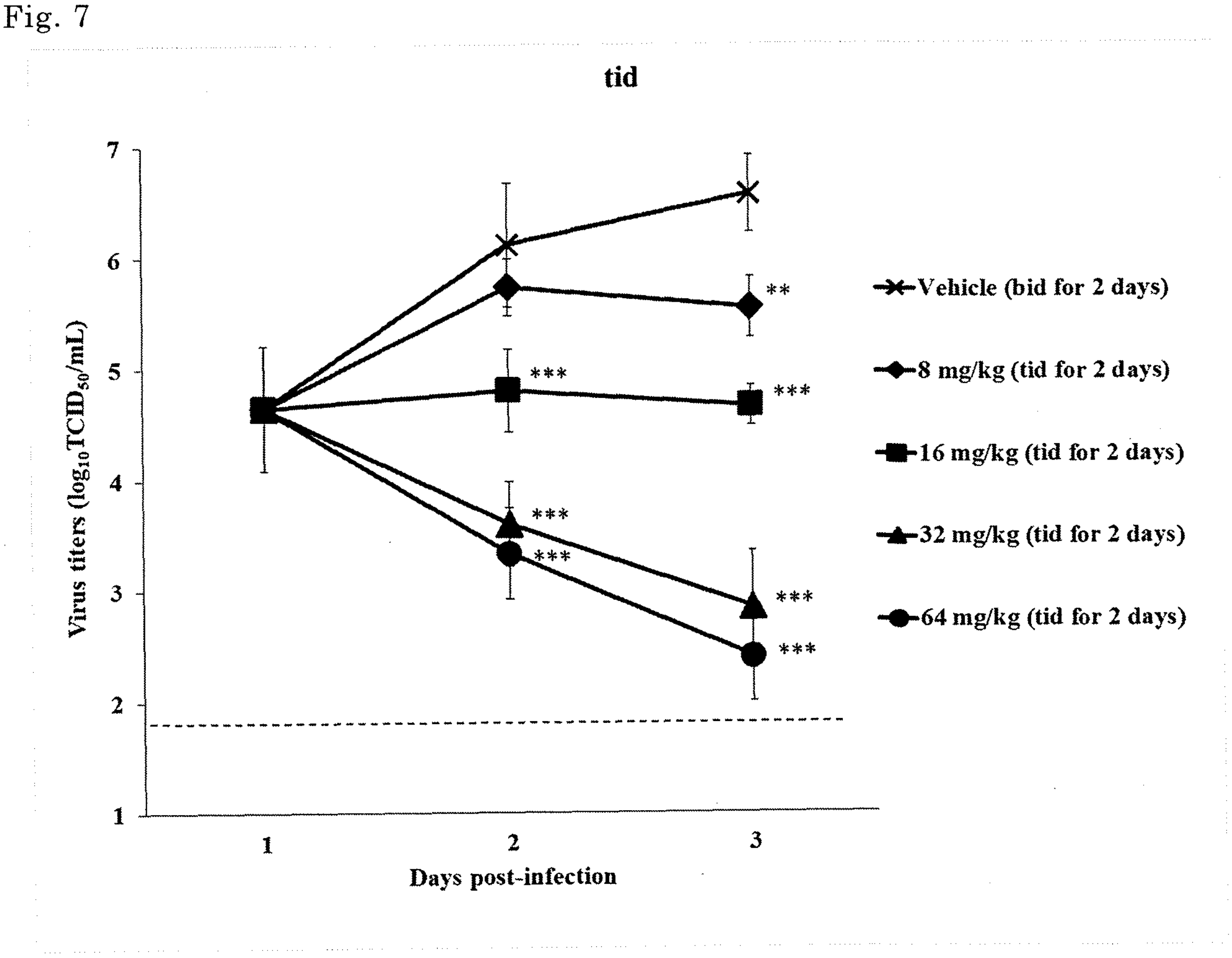
tid
7
6
5
4
3
2
1
Virus titers ($log_{10}TCID_{50}$/mL)
1
2
3
Days post-infection
**
***
***
***
***
***
***
Vehicle (bid for 2 days)
8 mg/kg (tid for 2 days)
16 mg/kg (tid for 2 days)
32 mg/kg (tid for 2 days)
64 mg/kg (tid for 2 days)

Fig. 8A

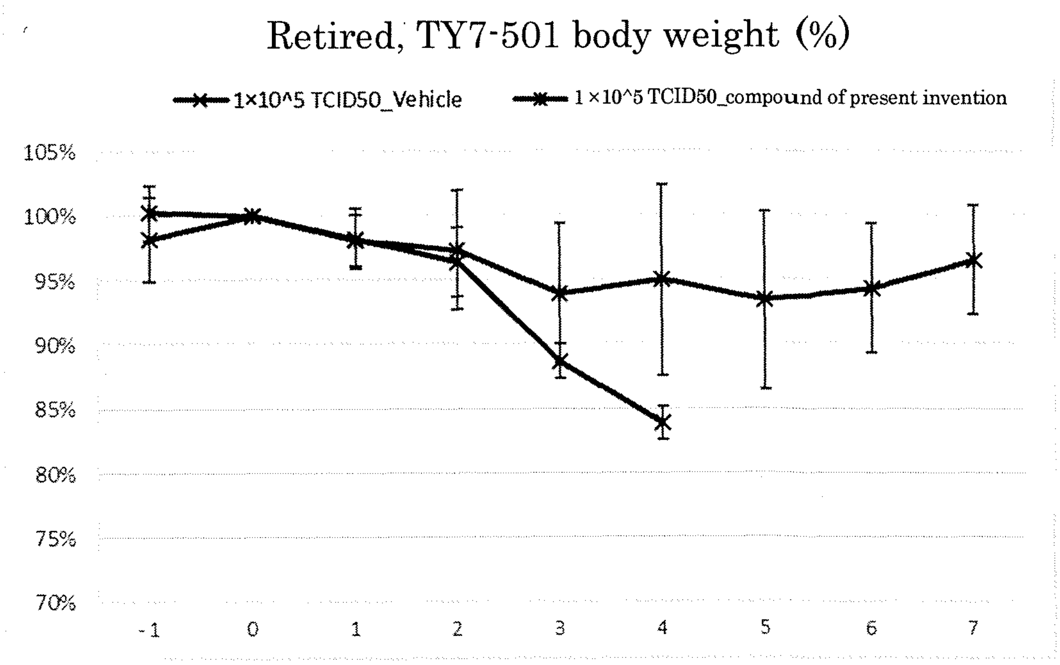
Retired, TY7-501 body weight (%)
1×10^5 TCID50_Vehicle
1×10^5 TCID50_compound of present invention
105%
100%
95%
90%
85%
80%
75%
70%
-1
0
1
2
3
4
5
6
7

Fig. 8B

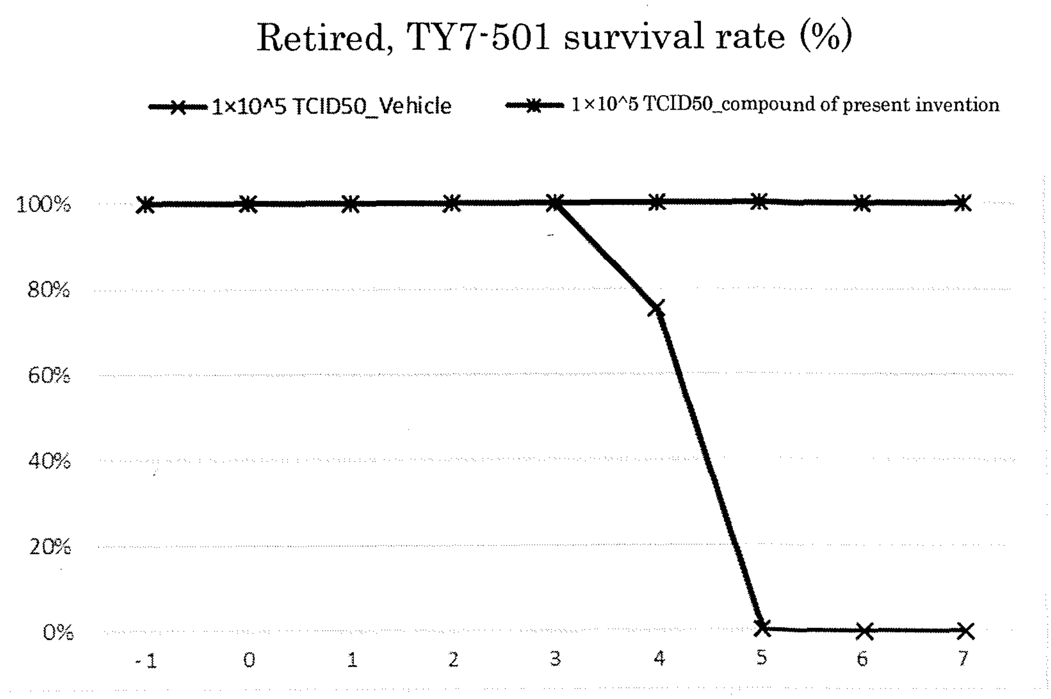
Retired, TY7-501 survival rate (%)
1×10^5 TCID50_Vehicle
1×10^5 TCID50_compound of present invention
100%
80%
60%
40%
20%
0%
-1
0
1
2
3
4
5
6
7

Fig. 8C

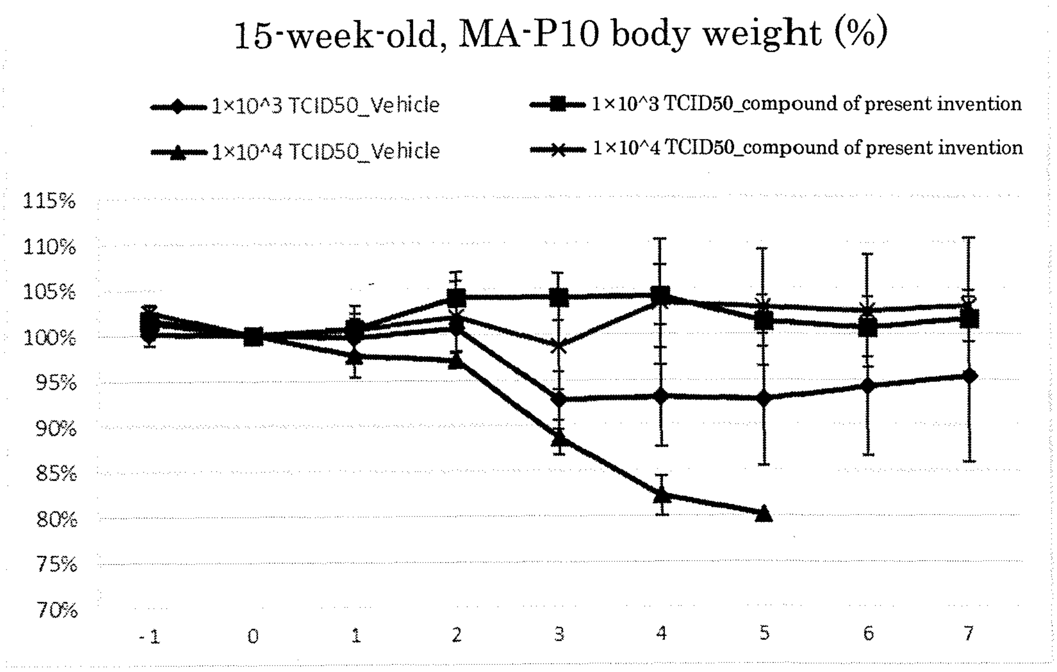
15-week-old, MA-P10 body weight (%)
1×10^3 TCID50_Vehicle
1×10^3 TCID50_compound of present invention
1×10^4 TCID50_Vehicle
1×10^4 TCID50_compound of present invention
115%
110%
105%
100%
95%
90%
85%
80%
75%
70%
-1
0
1
2
3
4
5
6
7

Fig. 8D

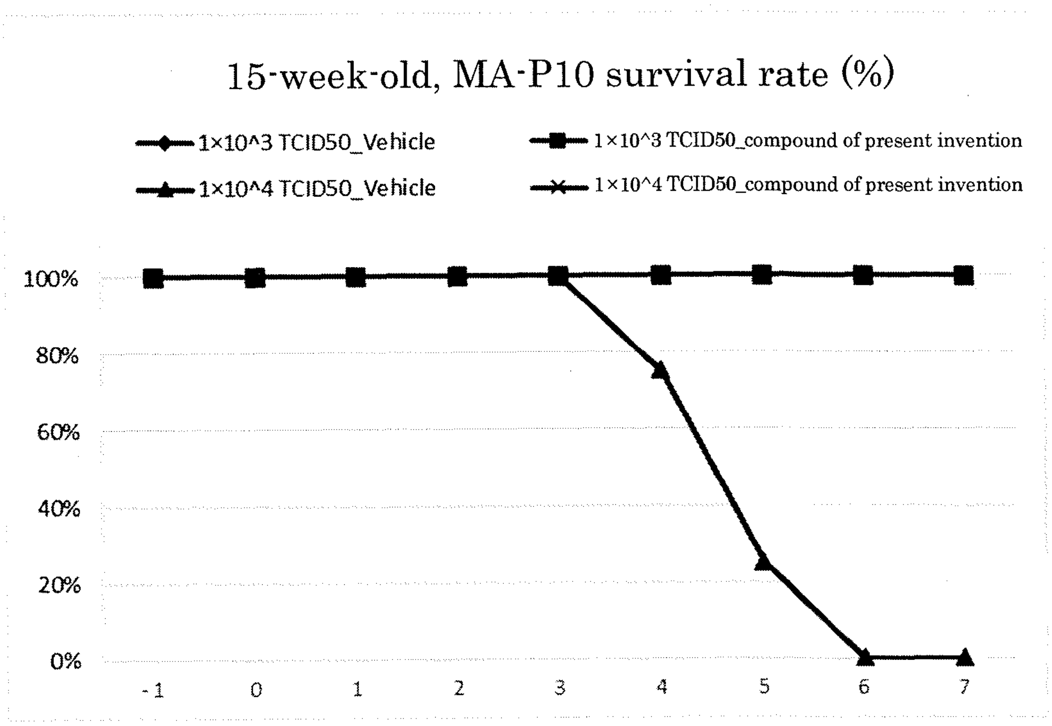
15-week-old, MA-P10 survival rate (%)
1×10^3 TCID50_Vehicle
1×10^3 TCID50_compound of present invention
1×10^4 TCID50_Vehicle
1×10^4 TCID50_compound of present invention
100%
80%
60%
40%
20%
0%
-1
0
1
2
3
4
5
6
7

Fig. 8E

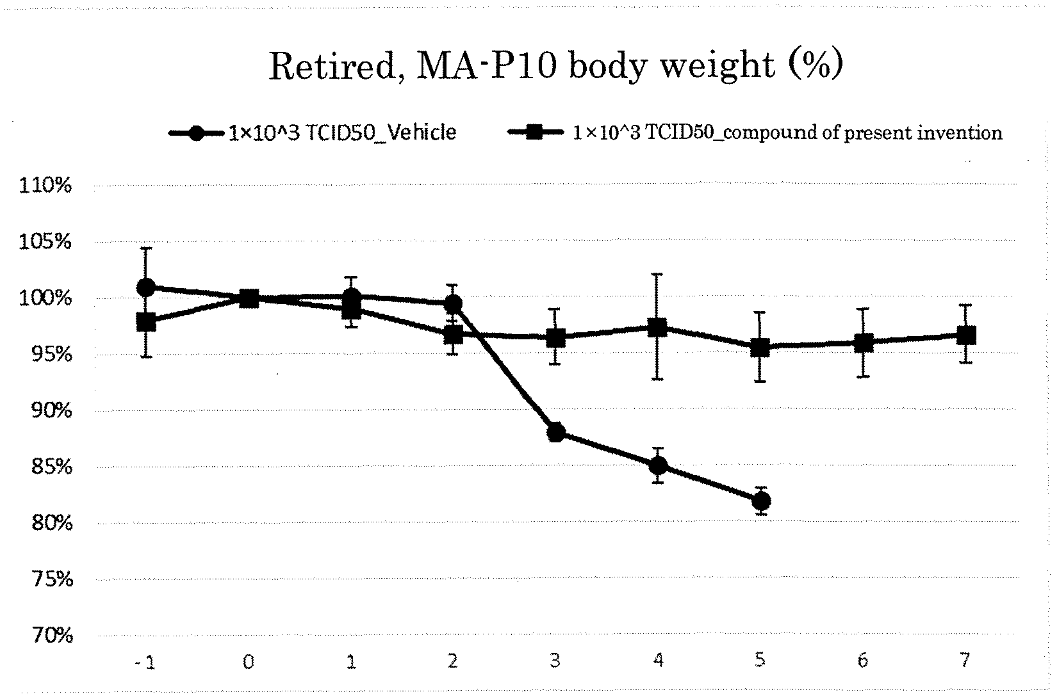
Retired, MA-P10 body weight (%)
1×10^3 TCID50_Vehicle
1×10^3 TCID50_compound of present invention
110%
105%
100%
95%
90%
85%
80%
75%
70%
-1
0
1
2
3
4
5
6
7

Fig. 8F

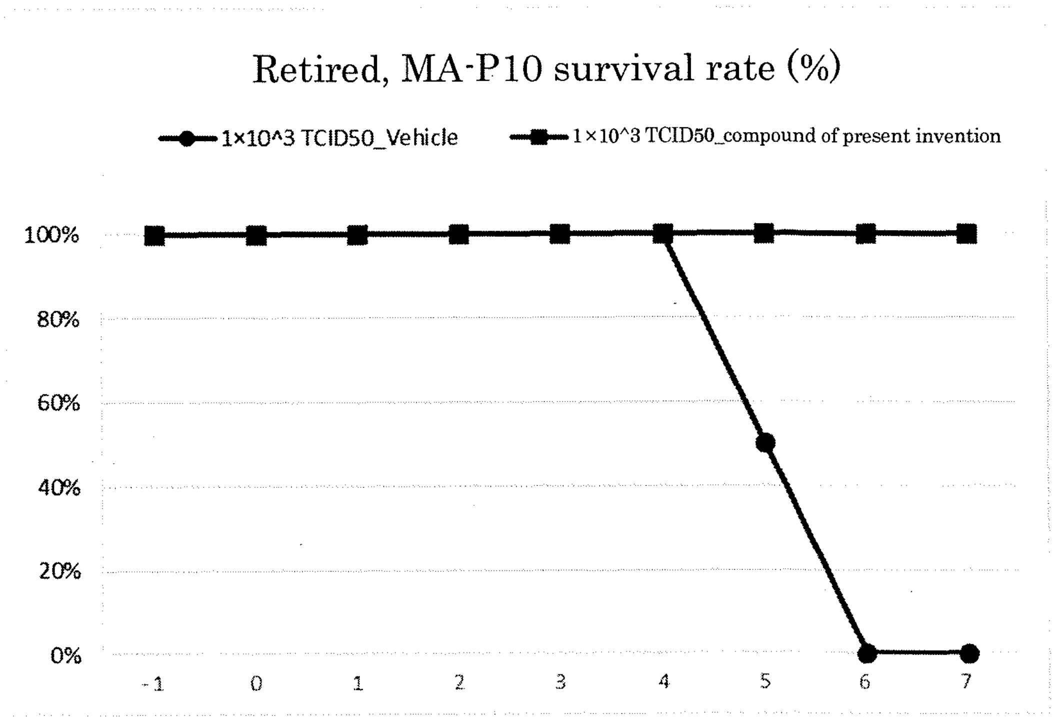
Retired, MA-P10 survival rate (%)
1×10^3 TCID50_Vehicle
1×10^3 TCID50_compound of present invention
100%
80%
60%
40%
20%
0%
-1
0
1
2
3
4
5
6
7

Fig. 9

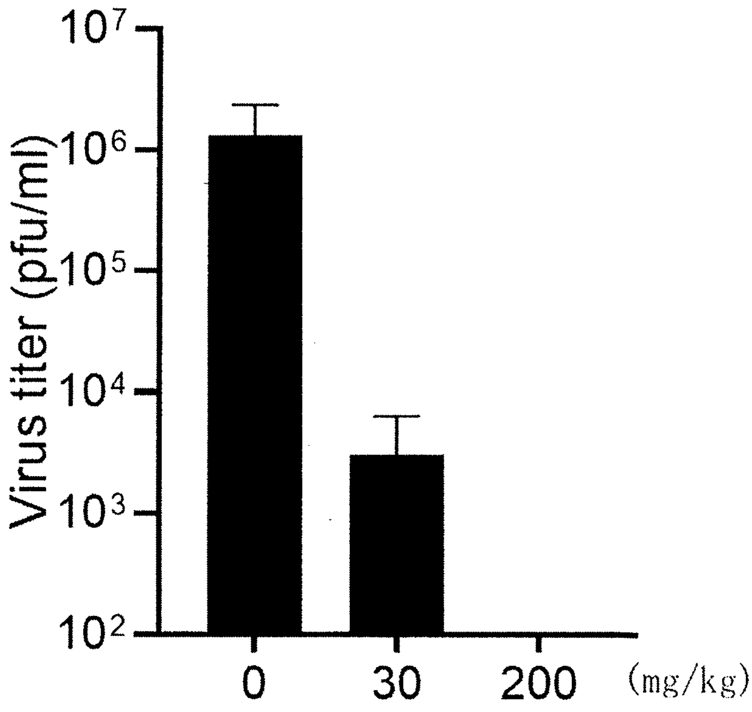


Fumaric acid cocrystal Form I crystal of compound represented by Formula (I-B) (bid)

Fig. 10

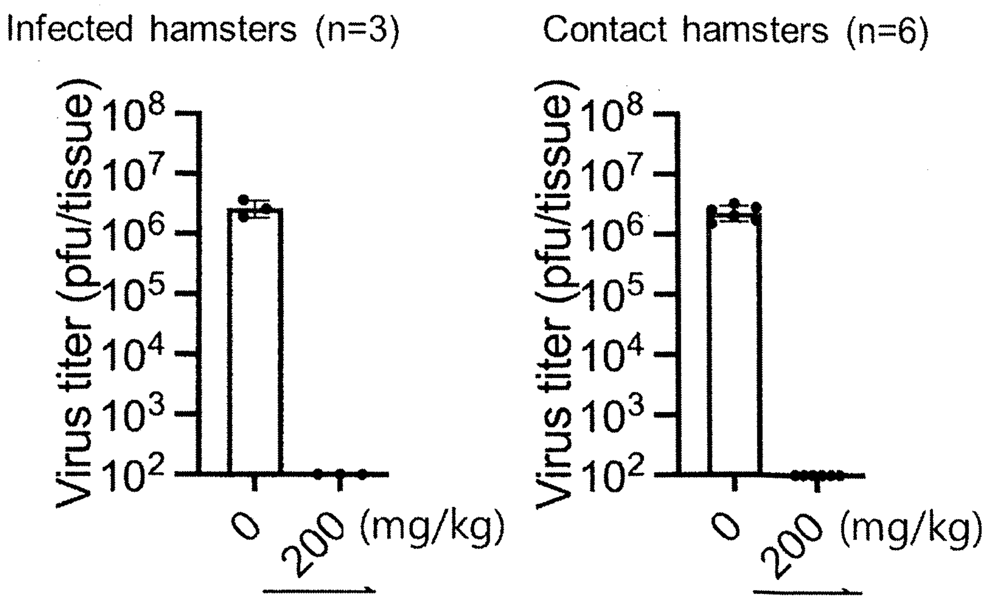


Fumaric acid cocrystal Form I crystal of compound represented by Formula (I-B) (BID)

Fumaric acid cocrystal Form I crystal of compound represented by Formula (I-B) (BID)

Fig. 11A

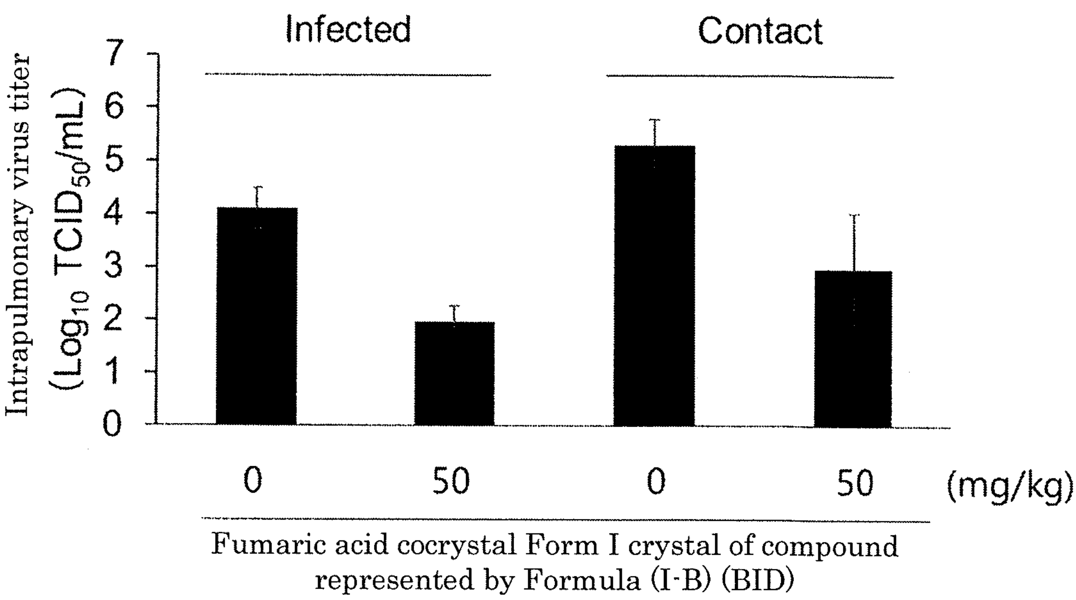


Fig. 11B

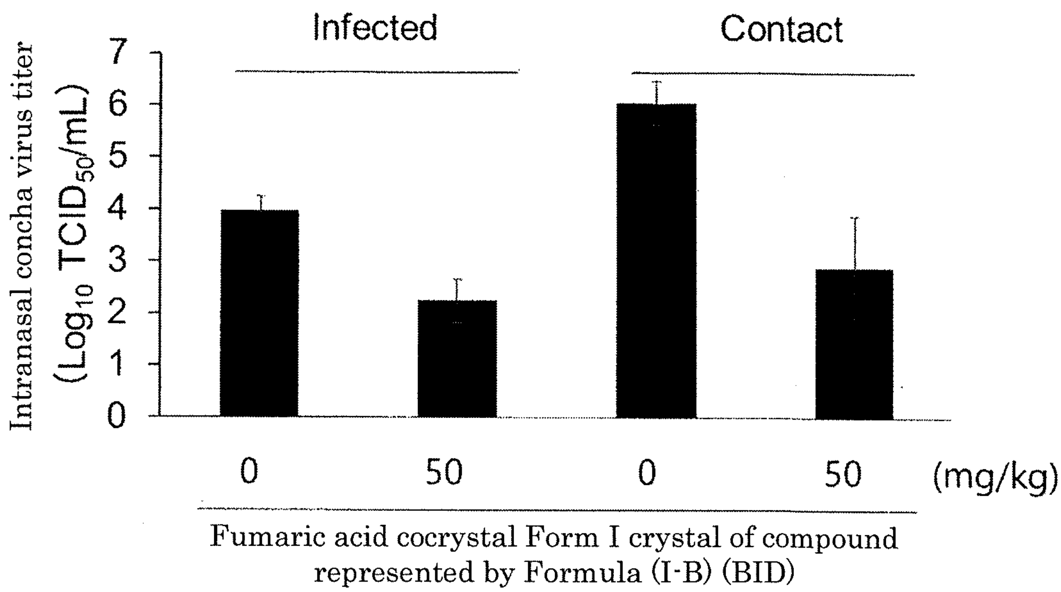

Fig. 12A

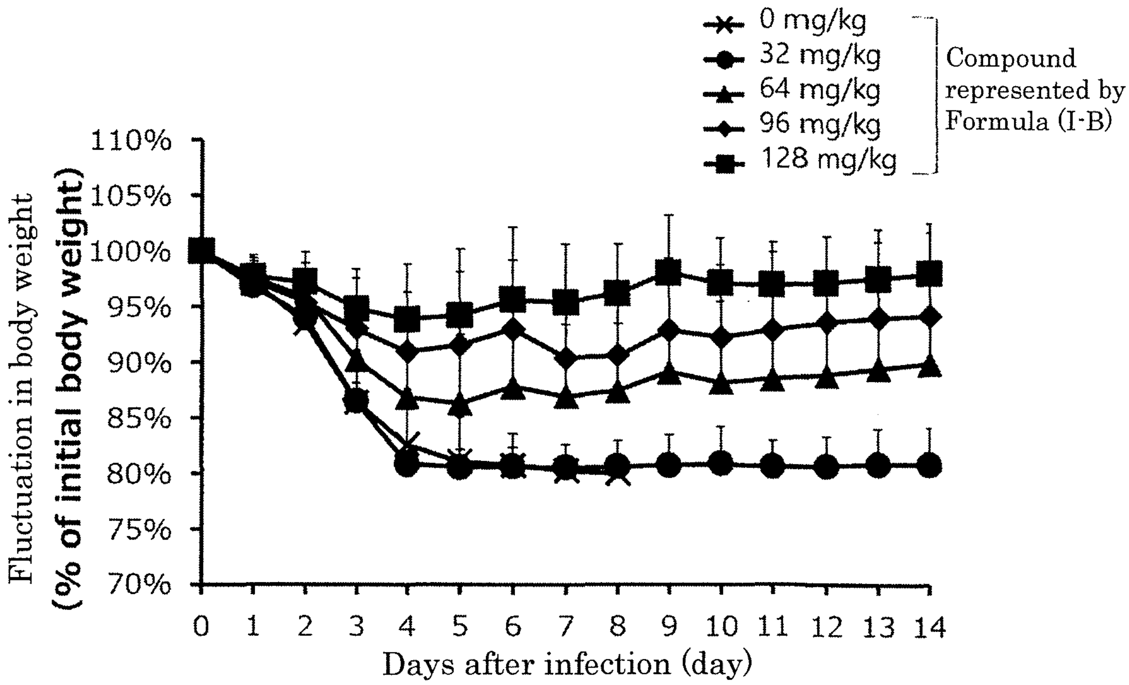


Fig. 12B

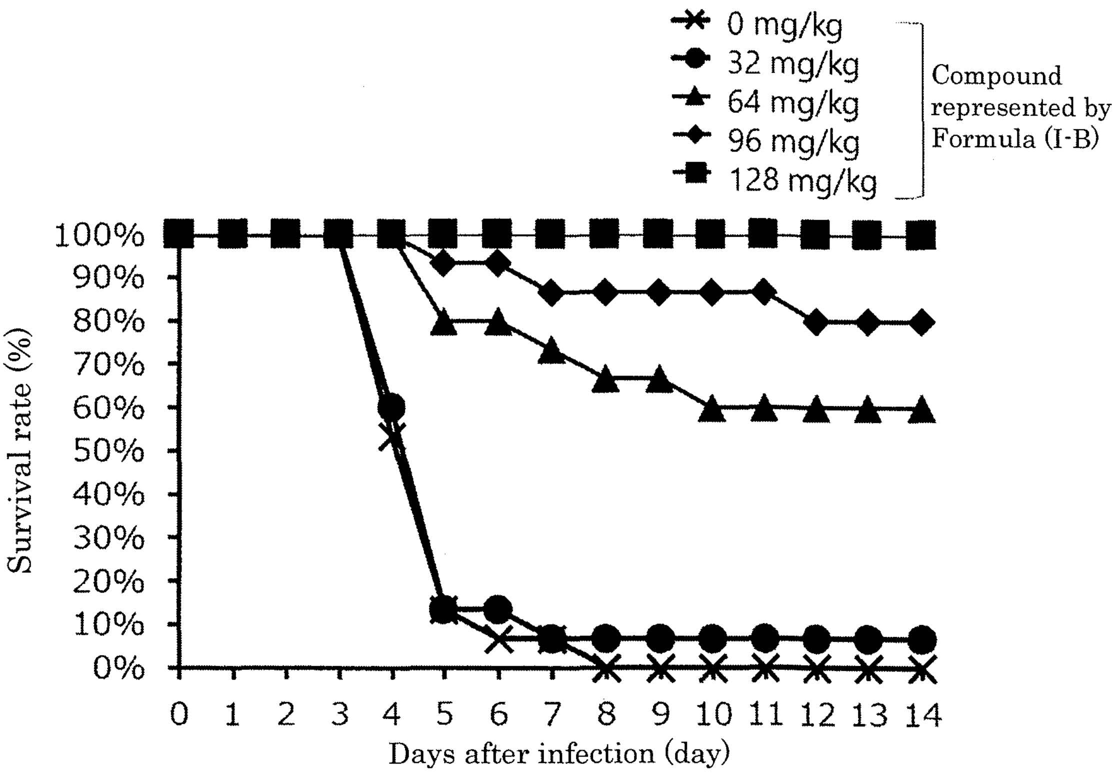

**INTERNATIONAL SEARCH REPORT**

International application No. **PCT/JP2022/035803**

**A. CLASSIFICATION OF SUBJECT MATTER**

***A61K 31/5355***(2006.01)i; ***A61K 31/53***(2006.01)i; ***A61K 31/5377***(2006.01)i; ***A61P 31/12***(2006.01)i; ***A61P 31/14***(2006.01)i; ***A61P 43/00***(2006.01)i
FI: A61K31/5355 ZNA; A61P31/14; A61P43/00 111; A61P31/12; A61K31/5377; A61K31/53

According to International Patent Classification (IPC) or to both national classification and IPC

**B. FIELDS SEARCHED**

Minimum documentation searched (classification system followed by classification symbols)

A61K31/5355; A61K31/53; A61K31/5377; A61P31/12; A61P31/14; A61P43/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| P, X | WO 2022/138987 A1 (SHIONOGI & CO) 30 June 2022 (2022-06-30) entire text | 1-12 |
| P, X | WO 2022/158528 A1 (UNIV HOKKAIDO NAT UNIV CORP) 28 July 2022 (2022-07-28) entire text | 1-12 |
| E, X | WO 2022/212314 A1 (SHIREEN NATURE COMPANY FOR GENERAL TRADING, LTD.) 06 October 2022 (2022-10-06) claim 121 | 1-12 |
| A | LIU, Y. et al. The development of Coronavirus 3C-Like protease (3CLpro) inhibitors from 2010 to 2020. European Journal of Medicinal Chemistry. 2020, 206, 112711(pp. 1-18) entire text | 1-12 |
| A | WO 2019/087884 A1 (FUJIFILM CORP) 09 May 2019 (2019-05-09) entire text | 1-12 |
| A | JP 2002-509140 A (SCRIPTGEN PHARMACEUTICALS, INC) 26 March 2002 (2002-03-26) entire text | 1-12 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **16 November 2022** | **13 December 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2022/035803**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).
2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.
3. Additional comments:

   The “form of an Annex C/ST.25 text file” is replaced with the “form of ST.26.”

Form PCT/ISA/210 (continuation of first sheet) (January 2015)

**INTERNATIONAL SEARCH REPORT**
**Information on patent family members**

International application No.
**PCT/JP2022/035803**

| Patent document cited in search report | Publication date (day/month/year) | Patent family member(s) | Publication date (day/month/year) |
|---|---|---|---|
| WO 2022/138987 A1 | 30 June 2022 | JP 7105430 B1<br>WO 2022/138988 A1 | |
| WO 2022/158528 A1 | 28 July 2022 | (Family: none) | |
| WO 2022/212314 A1 | 06 October 2022 | (Family: none) | |
| WO 2019/087884 A1 | 09 May 2019 | (Family: none) | |
| JP 2002-509140 A | 26 March 2002 | WO 1999/036410 A1<br>EP 1053230 A1<br>CA 2318362 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2012020749 A
- WO 2013089212 A
- WO 2010092966 A
- WO 2014200078 A
- WO 2021205298 A
- WO 2021250648 A
- WO 2012020742 A
- WO 2013118855 A


### Non-patent literature cited in the description


- COVID-19 Dashboard by the Center for Systems Science and Engineering at Johns Hopkins University. Johns Hopkins University, 21 September 2022
- The NEW ENGLAND JOURNAL of MEDICINE. 2020, vol. 382, 1564-1567
- Report of the WHO-China Joint Mission on Coronavirus Disease 2019 (COVID-19). WHO, 28 February 2020
- *Science,* 2003, vol. 300, 1763-1767
- A comparative analysis of SARS-CoV-2 antivirals characterizes 3CLpro inhibitor PF-00835231 as a potential new treatment for COVID-19. *Journal of Virology,* 23 February 2021, https://jvi.asm.org/content/early/2021/02/19/JVI.01819-20><doi: 10.1128/JVI.01819-20>
- *Cell Research,* 2020, vol. 30, 678-692
- *Science,* 2020, vol. 368, 409-412
- *ACS Central Science,* 2021, vol. 7 (3), 467-475
- *Cancer Treatment Reviews,* 1984, vol. 11 (1), 99-110
- *Contributions to Oncology,* 1984, vol. 18, 221-234
- *Arzneimittel-Forschung,* 1984, vol. 6 (11), 663-668
- *261st Am Chem Soc (ACS) Natl Meet,* 20210416
- *Science,* 2021, vol. 374, 1586-1593
- Pfizer's Novel COVID-19 Oral Antiviral Treatment Candidate Reduced Risk Of Hospitalization Or Death By 89% In Interim Analysis Of Phase 2/3 EPIC-HR Study. Pfizer Press Release, 05 November 2021
- Isotopes in the Physical and Biomedical Sciences. Labeled Compounds. 1987, vol. 1
- **SAKURAI TOSHIO.** Manual of X-ray structural analysis. Shokabo Co., Ltd, 1983
- **STOUT ; JENSEN.** X-Ray Structure Determination: A Practical Guide. Macmillan Co, 1968
- **ATHERTON, E. ; SHEPPARD, R. C.** In Solid Phase Peptide Synthesis, A Practical Approach. IRL Press at Oxford University Pres, 1989
- *Bioorg. Med. Chem.,* 1997, vol. 5 (9), 1883-1891
- **ATHERTON, E. ; SHEPPARD, R. C.** In Solid Phase Peptide Synthesis, A Practical Approach. IRL Press at Oxford University Pres, 1989
- *Journal of Clinical Microbiology,* 04 June 2005, (11), 5452-5456